(19) Europäisches Patentamt
European Patent Office
Office européen des brevets

(11) EP 1 728 500 B1

(12) FASCICULE DE BREVET EUROPEEN

(45) Date de publication et mention de la délivrance du brevet:
**27.01.2010 Bulletin 2010/04**

(51) Int Cl.:
***A61K 8/49*** *(2006.01)* ***A61K 8/41*** *(2006.01)*
***A61Q 5/10*** *(2006.01)*

(21) Numéro de dépôt: **06114651.0**

(22) Date de dépôt: **29.05.2006**

# (54) Composition pour la teinture des fibres kératiniques comprenant un dérivé de diamino-N,N-dihydro-pyrazolone et un colorant d'oxydation cationique

Mittel zum Färben von Keratinfasern enthaltend ein Diamino-N,N-dihydro-pyrazolon-Derivat und einen kationischen Oxidationsfarbstoff

Composition for dyeing keratinic fibres comprising a diamino-N,N-dihydro-pyrazolon derivative and a cationic oxidation dye

(84) Etats contractants désignés:
**AT BE BG CH CY CZ DE DK EE ES FI FR GB GR HU IE IS IT LI LT LU LV MC NL PL PT RO SE SI SK TR**

(30) Priorité: **31.05.2005 FR 0551428**

(43) Date de publication de la demande:
**06.12.2006 Bulletin 2006/49**

(73) Titulaire: **L'Oréal**
**75008 Paris (FR)**

(72) Inventeur: **Hercouet, Leïla**
**93360 Neuilly Plaisance (FR)**

(74) Mandataire: **Prevel, Estelle Nicole**
**L'Oréal**
**D.I.P.I.**
**25-29, Quai Aulagnier**
**92665 Asnières-sur-Seine Cedex (FR)**

(56) Documents cités:
**EP-A- 1 550 656**
**DE-A1- 1 617 893**
**DE-A1- 10 148 847**
**FR-A- 2 766 177**
**FR-A- 2 766 178**
**FR-A- 2 776 289**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



Printed by Jouve, 75001 PARIS (FR)

## Description

L'invention a pour objet une composition pour la teinture des fibres kératiniques, et en particulier des fibres kératiniques humaines telles que les cheveux, comprenant au moins une base d'oxydation du type diamino-N,N-dihydropyrazolone ou un de ses sels d'addition et un colorant d'oxydation cationique ainsi que le procédé de coloration mettant en oeuvre une telle composition.

Il est connu de teindre les fibres kératiniques, et en particulier les fibres kératiniques humaines telles que les cheveux, avec des compositions tinctoriales comprenant des colorants d'oxydation, en particulier des ortho ou para-phénylènediamines, des ortho ou para-aminophénols, des composés hétérocycliques tels que des dérivés de diaminopyrazole, des dérivés de pyrazolo[1,5-a]pyrimidine, des dérivés de pyrimidine, des dérivés de pyridine, des dérivés d'indole, des dérivés d'indoline appelés généralement bases d'oxydation. Les colorants d'oxydation, ou bases d'oxydation, sont des composés incolores ou faiblement colorés qui, associés à des produits oxydants, peuvent donner naissance par un processus de condensation oxydative à des composés colorés ou colorants. On obtient ainsi des colorations permanentes.

On sait également que l'on peut faire varier les nuances obtenues avec ces bases d'oxydation en les associant à des coupleurs ou modificateurs de coloration, ces derniers étant choisis notamment parmi les méta-phénylènediamines, les méta-aminophénols, les méta-hydroxyphénols et certains composés hétérocycliques.

La variété des molécules mises en jeu au niveau des bases d'oxydation et des coupleurs permet l'obtention d'une riche palette de couleurs.

L'utilisation de bases d'oxydation telle que les dérivés de para-phénylènediamine et de para-aminophénol permettent d'obtenir une gamme de couleurs assez large à pH basique sans toutefois atteindre des nuances de bonne chromaticité tout en conférant aux cheveux d'excellentes propriétés d'intensité de couleur, de variété de nuances, d'uniformité de la couleur et de la ténacité aux agents extérieurs.

L'utilisation de ces bases à pH neutre ne permet pas d'atteindre une gamme de nuances variées, en particuliers pour les nuances chaudes telles que les rouges et les orangés.

Le but de la présente invention est de fournir de nouvelles compositions de coloration des fibres kératiniques qui permettent d'obtenir une coloration aux nuances variées, en particulier naturelles, puissante, chromatique, esthétique, peu sélective et résistant bien aux diverses agressions que peuvent subir les cheveux tels que les shampooings, la lumière, la sueur et les déformations permanentes.

La présente invention a donc pour objet une composition de coloration des fibres kératiniques comprenant, dans un milieu approprié,

- au moins une base d'oxydation choisie parmi un dérivé de la diamino-N,N-dihydro-pyrazolone de formule (I) ou l'un de ses sels d'addition :

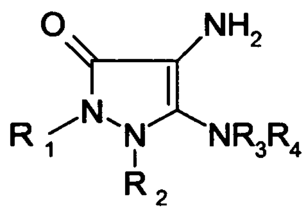

(I)

dans laquelle :

$R_1$, $R_2$, $R_3$ et $R_4$, identiques ou différents, représentent :

- un radical alkyle en $C_1$-$C_{10}$ linéaire ou ramifié éventuellement substitué par un ou plusieurs radicaux choisi dans le groupe constitué par un radical $OR_5$, un radical $NR_6R_7$, un radical carboxy, un radical sulfonique, un radical carboxamido $CONR_6R_7$, un radical sulfonamido $SO_2NR_6R_7$, un hétéroaryle, un aryle éventuellement substitué par un ou plusieurs groupes ($C_1$-$C_4$)alkyle, hydroxy, alcoxy en $C_1$-$C_2$, amino, (di)alkyl($C_1$-$C_2$)amino,
- un radical aryle éventuellement substitué par un ou plusieurs ($C_1$-$C_4$)alkyle, hydroxy, alcoxy en $C_1$-$C_2$, amino, (di)alkyl($C_1$-$C_2$)amino ;
- un radical hétéroaryle à 5 ou 6 chaînons, éventuellement substitué par un ou plusieurs radicaux choisis parmi ($C_1$-$C_4$)alkyle, ($C_1$-$C_2$)alcoxy;

$R_3$ et $R_4$ peuvent représenter également un atome d'hydrogène ;

$R_5$, $R_6$ et $R_7$, identiques ou différents, représentent

- un atome d'hydrogène ;
- un radical alkyle linéaire ou ramifié en $C_1$-$C_4$ éventuellement substitué par un ou plusieurs radicaux choisis parmi hydroxy, alcoxy en $C_1$-$C_2$, carboxamido $CONR_8R_9$, sulfonyle $SO_2R_8$, aryle éventuellement substitué par un ($C_1$-$C_4$)alkyle, hydroxy, alcoxy en $C_1$-$C_2$, amino, (di)alkyl($C_1$-$C_2$)amino ; aryle éventuellement substitué par un ($C_1$-$C_4$)alkyle , hydroxy, alcoxy en $C_1$-$C_2$, amino, (di)alkyl($C_1$-$C_2$)amino ;

$R_6$ et $R_7$, identiques ou différents, peuvent représenter également un radical carboxamido $CONR_8R_9$; un radical sulfonyle $SO_2R_8$;

$R_8$ et $R_9$, identiques ou différents, représentent un atome d'hydrogène ; un radical alkyle en $C_1$-$C_4$ linéaire ou ramifié éventuellement substitués par un ou plusieurs hydroxy, alcoxy en $C_1$-$C_2$ ;

$R_1$ et $R_2$ d'une part, et $R_3$ et $R_4$ d'autre part peuvent former avec le ou les atomes d'azote auxquels ils sont rattachés, un hétérocycle saturé ou insaturé, comportant 5 à 7 chaînons, éventuellement substitué par un ou plusieurs radicaux choisis dans le groupe constitué par les atomes d'halogène, les radicaux amino, (di)alkyl($C_1$-$C_4$)amino, hydroxy, carboxy, carboxamido, ($C_1$-$C_2$)alcoxy, les radicaux alkyles en $C_1$-$C_4$ éventuellement substitués par un ou plusieurs radicaux hydroxy, amino, (di)-alkylamino, alcoxy, carboxy, sulfonyle ;

$R_3$ et $R_4$ peuvent également former ensemble avec l'atome d'azote auquel ils sont rattachés, un hétérocycle à 5 ou 7 chaînons dont les atomes de carbone peuvent être remplacés par un atome d'oxygène ou d'azote éventuellement substitué,

• au moins un colorant d'oxydation substitué par au moins un radical comportant un atome d'azote quaternisé.

La présente invention permet en particulier d'obtenir une coloration des fibres kératiniques aux nuances variées, puissante, esthétique, peu sélective et résistant bien aux diverses agressions que peuvent subir les cheveux tels que les shampooings, la lumière, la sueur et les déformations permanentes. Elle permet notamment d'obtenir des colorations homogènes et puissantes sur des cheveux sensibilisés soit par des agents extérieurs soit par des traitements tels que la permanente ou la décoloration des cheveux.
Elle permet de plus d'obtenir des colorations intenses et variées à pH neutre.
Un autre objet de l'invention est un procédé de teinture des fibres kératiniques mettant en oeuvre la composition de la présente invention, ainsi que l'utilisation de cette composition pour la teinture des fibres kératiniques.
L'invention a enfin pour objet un kit de coloration comprenant d'une part une composition de coloration contenant une base d'oxydation de formule (I) et un colorant d'oxydation substitué par au moins un radical comportant un atome d'azote quaternisé et d'autre part une composition contenant un agent oxydant.
Dans le cadre de l'invention, sauf autre indication, on entend par radical alkyle des radicaux alkyles linéaires ou ramifiés, de préférence en $C_1$-$C_{10}$ sauf indication contraire, préférentiellement en $C_1$-$C_6$ , de préférence $C_1$-$C_4$, tels que le radical méthyle, éthyle, propyle, isopropyle, isobutyle, tertiobutyle, pentyle, hexyle.
Plus particulièrement, dans la formule (I) les radicaux $R_1$ et $R_2$, identiques ou différents, sont choisis parmi

- un radical alkyle en $C_1$-$C_6$ , de préférence $C_1$-$C_4$, éventuellement substitué par un hydroxy, un ($C_1$-$C_2$)alcoxy, un amino, un (di)alkyl($C_1$-$C_2$)amino ;
- un radical phényle, méthoxyphényle, éthoxyphényle, benzyle De préférence, les radicaux $R_1$ et $R_2$, identiques ou non, sont choisis parmi un radical méthyle, éthyle, 2-hydroxyéthyle, 3-hydroxypropyle, 2-hydroxypropyle, phényle.

Selon un autre mode de réalisation, les radicaux $R_1$ et $R_2$ forment ensemble avec les atomes d'azotes auxquels ils sont rattachés, un cycle à 5 ou 6 chaînons, saturé ou insaturé, éventuellement substitué.
De préférence, les radicaux $R_1$ et $R_2$ forment ensemble avec les atomes d'azotes auxquels ils sont rattachés, un cycle pyrazolidine, pyridazolidine, éventuellement substitué par un ou plusieurs radicaux alkyle en $C_1$-$C_4$, hydroxy, ($C_1$-$C_2$)alcoxy, carboxy, carboxamido, amino, (di)alkyl($C_1$-$C_2$)amino.
De manière encore plus avantageuse, les radicaux $R_1$ et $R_2$ forment ensemble avec les atomes d'azotes auxquels ils sont rattachés, un cycle pyrazolidine, pyridazolidine.
En ce qui concerne les radicaux $R_3$ et $R_4$, ces derniers, identiques ou différents, sont plus particulièrement choisis parmi un atome d'hydrogène ; un radical alkyle en $C_1$-$C_6$ , de préférence $C_1$-$C_4$ , linéaire ou ramifié, éventuel-

lement substitué par un ou plusieurs hydroxy, ($C_1$-$C_2$)alcoxy, amino, un (di)alkyl($C_1$-$C_2$)amino ; un radical phényle éventuellement substitué par un ou plusieurs radicaux hydroxy, amino, ($C_1$-$C_2$)alcoxy.

De préférence, les radicaux $R_3$ et $R_4$, identiques ou non, sont choisis parmi un atome d'hydrogène, un radical méthyle, éthyle, isopropyle, 2-hydroxyéthyle, 3-hydroxypropyle, 2-hydroxypropyle, 2-carboxyéthyle. Selon un mode de réalisation particulier, les radicaux $R_3$ et $R_4$, représentent un atome d'hydrogène.

Selon un autre mode de réalisation, les radicaux $R_3$ et $R_4$ forment ensemble avec l'atome d'azote auquel ils sont rattachés, un cycle à 5 ou 7 chaînons choisi parmi les hétérocycles pyrrolidine, pipéridine, homopipéridine, pipérazine, homopipérazine ; lesdits cycles pouvant être substitués par un ou plusieurs radicaux hydroxy, amino, (di)alkyl ($C_1$-$C_2$)amino, carboxy, carboxamido, alkyle en $C_1$-$C_4$ éventuellement substitué par un ou plusieurs radicaux hydroxy, amino, (di)alkylamino en $C_1$-$C_2$.

Plus particulièrement, les radicaux $R_3$ et $R_4$ forment ensemble avec l'atome d'azote auquel ils sont rattachés un cycle à 5 ou 7 chaînons choisi parmi la pyrrolidine, le 2,5-diméthylpyrrolidine, l'acide pyrrolidine-2-carboxylique, l'acide 3-hydroxypyrrolidine-2-carboxylique, l'acide 4-hydroxypyrrolidine-2-carboxylique, la 2,4-dicarboxypyrrolidine, la 3-hydroxy-2-hydroxyméthylpyrrolidine, la 2-carboxamidopyrrolidine, la 3-hydroxy-2-carboxamidopyrrolidine, la 2-(diéthylcarboxamido)pyrrolidine, la 2-hydroxyméthyl pyrrolidine, la 3,4-dihydroxy-2-hydroxyméthyl pyrrolidine, la 3-hydroxypyrrolidine, la 3,4-dihydroxy pyrrolidine, la 3-amino pyrrolidine, la 3-méthylamino pyrrolidine, la 3-diméthylamino-pyrrolidine, la 4-amino-3-hydroxy pyrrolidine, la 3-hydroxy-4-(2-hydroxyéthyl)amino- pyrrolidine, la pipéridine, la 2,6-diméthylpipéridine, la 2-carboxypipéridine, la 2-carboxamidopipéridine, la 2-hydroxyméthylpipéridine, la 3-hydroxy-2-hydroxyméthylpipéridine, 3-hydroxypipéridine, la 4-hydroxypipéridine, la 3-hydroxyméthylpipéridine, la homopipéridine, la 2-carboxyhomopipéridine, la 2-carboxamidohomopipéridine, l'homopipérazine, le N-méthyl-homopipérazine, le N-(2-hydroxyéthyl)-homopipérazine.

De préférence, les radicaux $R_3$ et $R_4$ forment ensemble avec l'atome d'azote auquel ils sont rattachés un cycle à 5 ou 7 chaînons choisi parmi la pyrrolidine, la 3-hydroxypyrrolidine, la 3-aminopyrrolidine, la 3-diméthylamino-pyrrolidine, l'acide pyrrolidine-2-carboxylique, l'acide 3-hydroxypyrrolidine-2-carboxylique, la pipéridine, l'hydroxypipéridine, l'homopipéridine, le diazépane, la N-méthyl homopipérazine, la N β-hydroxyéthyihomopipérazine.

Conformément à un mode de réalisation encore plus préféré de l'invention, les radicaux $R_3$ et $R_4$ forment ensemble avec l'atome d'azote auquel ils sont rattachés un cycle à 5 chaînons tels que la pyrrolidine, la 3-hydroxypyrrolidine, la 3-aminopyrrolidine, la 3-diméthylamino-pyrrolidine.

Les composés de formule (I) peuvent être éventuellement salifiés par des acides minéraux forts tels que par exemple HCl, HBr, HI, $H_2SO_4$, $H_3PO_4$, ou des acides organiques tels que, par exemple, l'acide acétique, lactique, tartrique, citrique ou succinique, benzènesulfonique, para-toluènesulfonique, formique, méthanesulfonique.

Ils peuvent aussi être sous forme de solvates par exemple un hydrate ou un solvate d'alcool linéaire ou ramifié tel que l'éthanol ou l'isopropanol.

A titre d'exemples de dérivés de formule (I), on peut citer les composés présentés ci-dessous ou leurs sels d'addition.

4,5-diamino-1,2-diméthyl-1,2-dihydro-pyrazol-3-one.
4-amino-5-méthylamino-1,2-di méthyl-1,2-dihydro-pyrazol-3-one.
4-amino-5-di méthylamino-1,2-diméthyl-1,2-dihydro-pyrazol-3-one.
4-amino-5-(2-hydroxyéthyl)amino-1,2-di méthyl-1,2-di hydro-pyrazol-3-one.
4-amino-5-(pyrrol idi n-1-yl)-1,2-diméthyl-1,2-di hydro-pyrazol-3-one.
4-amino-5-(pipéridin-1-yl)-1,2-diméthyl-1,2-dihydro-pyrazol-3-one.

4,5-diamino-1,2-di-(2-hydroxyéthyl)-1,2-dihydro-pyrazol-3-one.
4-amino-5-méthylamino-1,2-di-(2-hydroxyéthyl)-1,2-dihydro-pyrazol-3-one.
4-amino-5-diméthylamino-1,2-di-(2-hydroxyéthyl)-1,2-dihydro-pyrazol-3-one.
4-amino-5-(2-hydroxyéthyl)amino-1,2-di-(2-hydroxyéthyl)-1,2-dihydro-pyrazol-3-one.
4-amino-5-(pyrrolidin-1-yl)-1,2-di-(2-hydroxyéthyl)-1,2-dihydro-pyrazol-3-one.
4-amino-5-(pipéridin-1-yl)-1,2-di-(2-hydroxyéthyl)-1,2-dihydro-pyrazol-3-one.

4,5-d iamino-1,2-d iéthyl-1,2-di hydro-pyrazol-3-one.
4,5-diamino-1,2-phényl-1,2-dihydro-pyrazol-3-one.
4,5-d iamino-1-éthyl-2-méthyl-1,2-di hydro-pyrazol-3-one.
4,5-d iamino-2-éthyl-1-méthyl-1,2-di hydro-pyrazol-3-one.
4,5-diamino-1-phényl-2-méthyl-1,2-dihydro-pyrazol-3-one.
4,5-diamino-2-phényl-1-méthyl-1,2-dihydro-pyrazol-3-one.
4,5-d iamino-1-(2-hydroxyéthyl)-2-méthyl-1,2-di hydro-pyrazol-3-one.
4,5-d iamino-2-(2-hydroxyéthyl)-1-méthyl-1,2-di hydro-pyrazol-3-one.

2,3-diamino-6,7-dihydro-1H,5H-pyrazolo[1,2-a]pyrazol-1-one
2-amino-3-méthylamino-6,7-dihydro-1H,5H-pyrazolo[1,2-a]pyrazol-1-one
2-amino-3-diméthylamino-6,7-dihydro-1H,5H-pyrazolo[1,2-a]pyrazol-1-one
2-amino-3-éthylamino-6,7-dihydro-1 H,5H-pyrazolo[1,2-a]pyrazol-1-one
2-amino-3-isopropylamino-6,7-dihydro-1 H,5H-pyrazolo[1,2-a]pyrazol-1-one
2-amino-3-(2-hydroxyéthyl)amino-6,7-dihydro-1 H,5H-pyrazolo[1,2-a]pyrazol-1-one
2-amino-3-(2-hydroxypropyl)amino-6,7-dihydro-1 H,5H-pyrazolo[1,2-a]pyrazol-1-one
2-amino-3-bis(2-hydroxyéthyl)amino-6,7-dihydro-1 H,5H-pyrazolo[1,2-a]pyrazol-1-one

2-amino-3-(pyrrolidin-1-yl)-6,7-dihydro-1H,5H-pyrazolo[1,2-a]pyrazol-1-one
2-amino-3-(3-hydroxy-pyrrolidin-1-yl)-6,7-dihydro-1H,5H-pyrazolo[1 ,2-a]pyrazol-1-one
2-amino-3-(pipéridin-1-yl)-6,7-dihydro-1 H,5H-pyrazolo[1,2-a]pyrazol-1-one

2,3-diamino-6-hydroxy-6,7-dihydro-1H,5H-pyrazolo[1,2-a]pyrazol-1-one
2,3-diamino-6-méthyl-6,7-dihydro-1H,5H-pyrazolo[1,2-a]pyrazol-1-one
2,3-diamino-6-diméthyl-6,7-dihydro-1H,5H-pyrazolo[1,2-a]pyrazol-1-one
2,3-diamino-5,6,7,8-tétrahydro-1H,6H-pyridazino[1,2-a]pyrazol-1-one
2,3-diamino-5,8-dihydro-1H,6H-pyridazino[1,2-a]pyrazol-1-one

4-Amino-5-dimethylamino-1,2-diethyl-1,2-dihydro-pyrazol-3-one
4-Ami no-1,2-d iethyl-5-ethylamino-1,2-dihydro-pyrazol-3-one
4-Ami no-1,2-diethyl-5-isopropylamino-1,2-di hydro-pyrazol-3-one
4-Amino-1,2-diethyl-5-(2-hydroxy-ethylamino)-1,2-dihydro-pyrazol-3-one
4-Ami no-5-(2-dimethylamino-ethylamino)-1,2-diethyl-1,2-d ihydro-pyrazol-3-one
4-Amino-5-[bis-(2-hydroxy-ethyl)-amino]-1,2-diethyl-1,2-dihydro-pyrazol-3-one
4-Ami no-1,2-diethyl-5-(3-i midazol-1-yl-propylami no)-1,2-d ihydro-pyrazol-3-one
4-Amino-5-dimethylamino-1,2-diethyl-1,2-dihydro-pyrazol-3-one
4-Ami no-1,2-d iethyl-5-ethylamino-1,2-dihydro-pyrazol-3-one
4-Ami no-1,2-diethyl-5-isopropylamino-1,2-di hydro-pyrazol-3-one
4-Amino-1,2-diethyl-5-(2-hydroxy-ethylamino)-1,2-dihydro-pyrazol-3-one
4-Ami no-5-(2-dimethylamino-ethylamino)-1,2-diethyl-1,2-d ihydro-pyrazol-3-one
4-Amino-5-[bis-(2-hydroxy-ethyl)-amino]-1,2-diethyl-1,2-dihydro-pyrazol-3-one
4-Ami no-1,2-diethyl-5-(3-i midazol-1-yl-propylami no)-1,2-d ihydro-pyrazol-3-one
4-Amino-1.2-diethyl-5-(3-hydroxy-pyrrolidin-1 -yl)-1,2-dihydro-pyrazol-3-one
4-Amino-1.2-diethyl-5-pyrrolidin-1-yl-1,2-dihydro-pyrazol-3-one
4-Ami no-5-(3-dimethylamino-pyrrol id in-1-yl)-1,2-d iethyl-1,2-dihydro-pyrazol-3-one
4-Ami no-1,2-d iethyl-5-(4-methyl-piperazi n-1-yl)-pyrazol idin-3-one
2,3-Diamino-6-hydroxy-6,7-dihydro-5H-pyrazolo[1,2-a]pyrazol-1-one

dont certains sont figurés ci-dessous pour illustrer les noms par des structures chimiques :

| | |
|---|---|
| 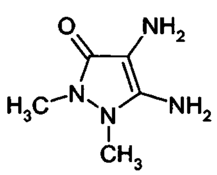 | 4,5-Diamino-1,2-diméthyl-1,2-dihydro-pyrazol-3-one |
| 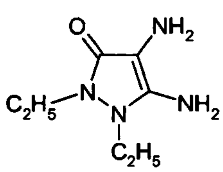 | 4,5-Diamino-1,2-diéthyl-1,2-dihydro-pyrazol-3-one |

(suite)

| | |
|---|---|
| 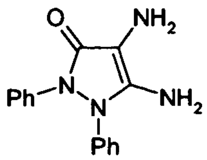 | 4,5-Diamino-1,2-diphényl-1,2-dihydro-pyrazol-3-one |
| 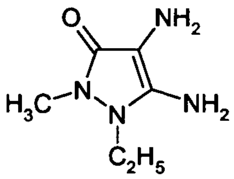 | 4,5-Diamino-1-éthyl-2-méthyl-1,2-dihydro-pyrazol-3-one |
| 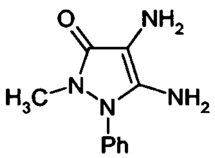 | 4,5-Diamino-1-phényl-2-méthyl-1,2-d ihydro-pyrazol-3-one |
| 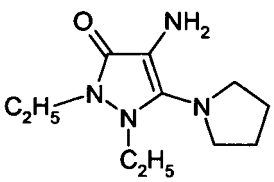 | 4-Ami no-5-(pyrrol idin-1-yl)-1,2-d iethyl-1,2-di hydro-pyrazol-3-one |
| 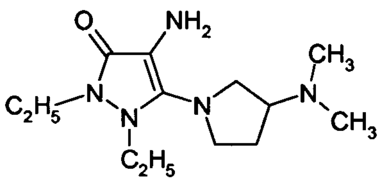 | 4-Amino-5-(3-dimethylamino-pyrrolidin-1-yl)-1,2-diethyl-1,2-dihydro-pyrazol-3-one |
| 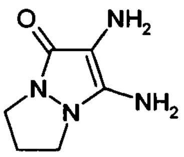 | 2,3-diamino-6,7-dihydro-1H,5H-pyrazolo[1,2-a]pyrazol-1-one |
| 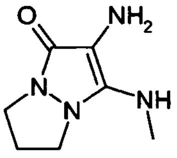 | 2-Amino-3-méthylamino-6,7-dihydro-1H,5H-pyrazolo[1,2-a]pyrazol-1-one |
| 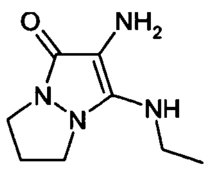 | 2-Amino-3-éthylamino-6,7-dihydro-1H,5H-pyrazolo[1,2-a]pyrazol-1-one |

(suite)

| | |
|---|---|
| 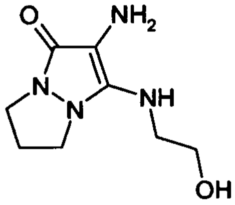 | 2-amino-3-(2-hydroxyéthyl)amino-6,7-dihydro-1H,5H-pyrazolo[1,2-a] pyrazol-1-one |
| 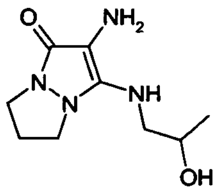 | 2-amino-3-(2-hydroxypropyl)amino-6,7-dihydro-1H,5H-pyrazolo[1,2-a] pyrazol-1-one |
| 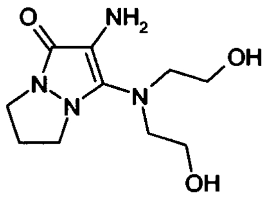 | 2-amino-3-bis(2-hydroxyéthyl)amino-6,7-dihydro-1H,5H-pyrazolo[1,2-a] pyrazol-1-one |
| 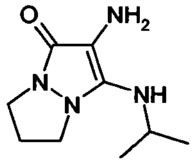 | 2-amino-3-isopropylamino-6,7-dihydro-1H,5H-pyrazolo[1,2-a]pyrazol-1-one |
| 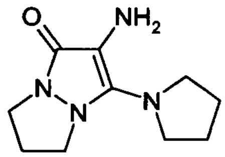 | 2-amino-3-(pyrrolidin-1-yl)-6,7-dihydro-1H,5H-pyrazolo[1,2-a]pyrazol-1-one |
| 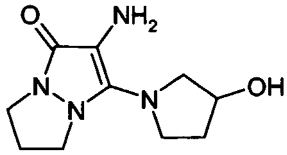 | 2-amino-3-(3-hydroxy-pyrrolidin-1-yl)-6,7-dihydro-1H,5H-pyrazolo[1,2-a] pyrazol-1-one |
| 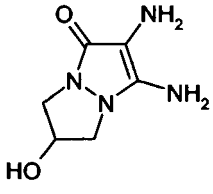 | 2,3-diamino-6-hydroxy-6,7-dihydro-1H,5H-pyrazolo[1,2-a]pyrazol-1-one |

(suite)

| | |
|---|---|
| 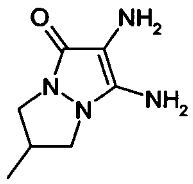 | 2,3-diamino-6-méthyl-6,7-dihydro-1H,5H-pyrazolo[1,2-a] pyrazol-1-one |
| 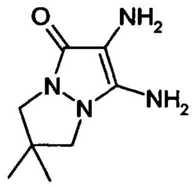 | 2,3-diamino-6,6-diméthyl-6,7-dihydro-1H,5H-pyrazolo[1,2-a] pyrazol-1-one |
| 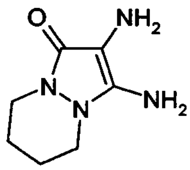 | 2,3-diamino-5,6,7,8-tétrahydro-1H,6H-pyridazino[1,2-a]pyrazol-1-one |
| 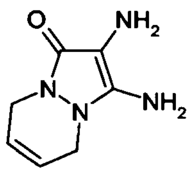 | 2,3-diamino-5,8-dihydro-1H,6H-pyridazino[1,2-a]pyrazol-1-one |
| 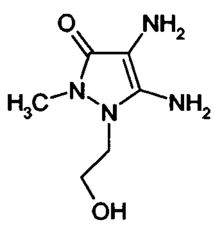 | 4,5-diamino-1-(2-hydroxyéthyl)-2-méthyl-1,2-dihydro-pyrazol-3-one |
| 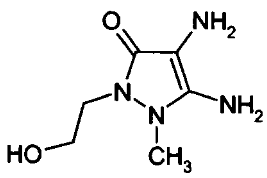 | 4,5-diamino-2-(2-hydroxyéthyl)-1-méthyl-1,2-dihydro-pyrazol-3-one. |

Parmi ces composés, les dérivés de diamino-N,N-dihydropyrazolone de formule (I) ou leurs sels d'addition, particulièrement préférés sont les :

2,3-Diamino-6,7-dihydro-1H,5H-pyrazolo[1,2-a]pyrazol-1-one
2-Amino-3-éthylamino-6,7-dihydro-1H,5H-pyrazolo[1,2-a]pyrazol-1-one
2-Amino-3-isopropylamino-6,7-dihydro-1H,5H-pyrazolo[1,2-a]pyrazol-1-one
2-Amino-3-(pyrrolidin-1-yl)-6,7-dihydro-1H,5H-pyrazolo[1,2-a]pyrazol-1-one
4,5-diamino-1,2-diméthyl-1,2-dihydro-pyrazol-3-one.
4,5-d iamino-1,2-d iéthyl-1,2-di hydro-pyrazol-3-one.
4,5-diamino-1,2-di-(2-hydroxyéthyl)-1,2-dihydro-pyrazol-3-one.
2-amino-3-(2-hydroxyéthyl)amino-6,7-dihydro-1H,5H-pyrazolo[1,2-a]pyrazol-1-one
2-amino-3-diméthylamino-6,7-dihydro-1H,5H-pyrazolo[1,2-a]pyrazol-1-one

2,3-diamino-5,6,7,8-tétrahydro-1H,6H-pyridazino[1,2-a]pyrazol-1-one
4-Amino-1,2-diethyl-5-(pyrrolidin-1-yl)-1,2-dihydro-pyrazol-3-one
4-Amino-5-(3-dimethylamino-pyrrolidin-1-yl)-1,2-diethyl-1,2-dihydro-pyrazol-3-one
2,3-diamino-6-hydroxy-6,7-dihydro-1H,5H-pyrazolo[1,2-a]pyrazol-1-one

La ou les bases d'oxydation de formule (I) sont en général présentes chacune en quantité comprise entre 0,001 à 10 % en poids environ du poids total de la composition tinctoriale, de préférence entre 0,005 et 6 %.

Dans la suite de la description, le radical cationique comportant au moins un atome d'azote quaternisé est appelé Z.

A titre d'exemple de radicaux Z, on peut citer les radicaux trialkylammonium ou les atomes d'azote quaternisés inclus dans un cycle.

A titre d'exemple, on peut citer les radicaux Z tels que les radicaux triméthylammonium, triéthyl ammonium, diéthyl-méthyl ammonium, tri butylammmonium, pyrrolium, imidazolium, pyrazolium, oxazolium, thiazolium, triazolium, pyridinium, pyrimidinium, pyrazinium, oxazinium et triazinium. Les radicaux Z trialkylammonium, imidazolium et pyridinium sont particulièrement préférés.

Les colorants d'oxydation substitués par au moins un radical Z sont connus de la technique. Ces colorants d'oxydation sont par exemple des bases d'oxydation ou des coupleurs. Selon un mode de réalisation particulier, ces colorants d'oxydation sont substitués par un ou deux radicaux Z.

A titre de bases d'oxydation substituées par au moins un radical Z, on peut citer les bases para-phénylènediamines, les bis-phénylalkylènediamines, les para-aminophénols, les bis-para-aminophénols, les ortho-aminophénols, les bases hétérocycliques telles que les dérivés pyridiniques, les dérivés pyrimidiniques et les dérivés pyrazoliques, les dérivés pyrazolo[1,5-a] pyrimidine et leurs sels d'addition.

Les bases d'oxydations du type paraphénylènediamine ou para-aminophénols substituées par un ou plusieurs radicaux Z utiles pour l'invention sont par exemple décrites dans la demande de brevet EP 968 171.

On peut notamment citer :

- le chlorure de [2-(2,5-diamino-phénoxy)-éthyl]-diéthyl-méthyl-ammonium monohydrate ;
- le dichlorure de N,N-bis-(triméthylammonium-propyl)-4-amino-aniline ;
- le chlorure de [4-(4-amino-phénylamino)-pentyl]-diéthyl-méthyl-ammonium ;
- le chlorure de [4-(4-amino-phénylamino)-pentyl]-diéthyl-(2-hydroxyéthyl)-ammonium ;
- le chlorure de [2-(4-amino-phénylamino)-éthyl]-diéthyl-méthyl-ammonium ;
- le chlorure de {2-[(4-aminophényl)-méthyl-amino]-éthyl}-triméthyl-ammonium ;
- le chlorure de [3-(4-amino-phénylamino)-propyl]-triméthyl-ammonium ;
- le chlorure de [2-(4-amino-phénylamino)-propyl]-triméthyl-ammonium ;
- le chlorure de [4-(4-amino-2-méthyl-phénylamino)-pentyl]-diéthyl-méthyl-ammonium ;
- le chlorure de [4-(4-amino-3-méthyl-phénylamino)-pentyl]-diéthyl-méthyl-ammonium ;

et leurs sels d'addition avec un acide.

Parmi ces bases, on préfère plus particulièrement :

- le chlorure de [2-(2,5-diamino-phénoxy)-éthyl]-diéthyl-méthyl-ammonium monohydrate ;
- le chlorure de N,N-bis-(triméthylammonium-propyl)-4-amino-aniline ;
- le chlorure de [4-(4-amino-phénylamino)-pentyl]-diéthyl-méthyl-ammonium ;
- le chlorure de [2-(4-amino-phénylamino)-éthyl]-diéthyl-méthyl-ammonium ;
- le chlorure de {2-[(4-aminophényl)-méthyl-amino]-éthyl}-triméthyl-ammonium ;
- le chlorure de [3-(4-amino-phénylamino)-propyl]-triméthyl-ammonium ;
- le chlorure de [4-(4-amino-phénylamino)-pentyl]-diéthyl-(2-hydroxyéthyl)-ammonium ;

et leurs sels d'addition avec un acide.

On peut aussi citer les bases d'oxydation du type paraphénylènediamine ou para-aminophénols substituée par un ou plusieurs radicaux Z cycliques décrites dans la demande de brevet EP 928289.

A titre d'exemple, on peut citer

- le bromure de 1-[2-(4-amino-phénylamino)-éthyl]-3-méthyl-3H-imidazol-1-ium ;
- le chlorure de 1-[3-(2,5-diamino-phénoxy)-propyl]-3-méthyl-3H-imidazol-1-ium ;
- le chlorure de 3-[3-(4-amino-phénylamino)-propyl]-1-méthyl-3H-imidazol-1-ium ;
- le chlorure de 3-[3-(4-amino-3-méthyl-phénylamino)-propyl]-1-méthyl-3H-imidazol-1-ium ;
- le chlorure de 3-[3-(4-amino-2-méthyl-phénylamino)-propyl]-1-méthyl-3H-imidazol-1-ium ;

- le chlorure de 3-[3-(4-amino-2-fluoro-phénylamino)-propyl]-1-méthyl-3H-imidazol- 1-ium, monohydrate ;
- le chlorure de 3-[3-(4-amino-2-cyano-phénylamino)-propyl]-1-méthyl-3H-imidazol-1-ium ;
- le chlorure de 1-[2-(4-amino-2-méthoxy-phénylamino)-éthyl]-3-méthyl-3H-imidazol-1-ium ;
- le chlorure de 1-(5-amino-2-hydroxy-benzyl)-3-méthyl-3H-imidazol-1-ium ;
- le chlorure de 1-(5-amino-2-hydroxy-benzyl)-2-méthyl-2H-pyrazol-1-ium ;
- le chlorure de 1-[2-(2,5-diamino-phényl)-éthyl]-3-méthyl-3H-imidazol-1-ium ;
- le chlorure de 3-[2-(2,5-diamino-phényl)-éthyl]-1-méthyl-3H-imidazol-1-ium ;
- le chlorure de 1-{2-[(4-amino-phényl)-éthylamino]-éthyl}-3-méthyl-3H-imidazol-1-ium ;
- le dichlorure de N,N-bis-[2-(3-méthyl-3H-imidazol-1-ium)-éthyl]-4-amino-aniline ;
- le chlorure de 3-[2-(4-amino-phénylamino)-butyl]-1-méthyl-3H-imidazol-1-ium ;
- le chlorure de 1-{[5-amino-2-(2-hydroxy-éthylamino)-phénylcarbamoyl]-méthyl}-3-méthyl-3H-imidazol-1-ium ;
- le bromure de 4-[2-(2,5-diamino-phénoxy)-éthyl]-1,3-diméthyl-3H-imidazol-1-ium ;
- le chlorure de 2-(2,5-diamino-phénoxyméthyl)-1,3-diméthyl-3H-imidazol-1-ium ;
- le chlorure de 4-[3-(4-amino-phénylamino)-propyl]-1,3-diméthyl-3H-imidazol-1-ium ;
- le chlorure de 4-[3-(4-amino-3-méthyl-phénylamino)-propyl]-1,3-diméthyl-3H-imidazol-1-ium ;
- le chlorure 4-[(2,5-diamino-phénylcarbamoyl)-méthyl]-1,3-diméthyl-3H-imidazol-1-ium ;
- le chlorure de 4-{2-[2-(2-amino-5-hydroxy-phényl)-acétylamino]-éthyl}-1,3-diméthyl-3H-imidazol-1-ium ;
- le chlorure de 4-[(5-amino-2-hydroxy-benzylcarbamoyl)-méthyl]-1,3-diméthyl-3H-imidazol-1-ium ;

et leurs sels d'addition avec un acide.

On peut aussi citer le composé suivant :

- chlorure de 1-{[5-amino-2-(2-hydroxyéthylamino)-phénylcarbamoyl]-méthyl}-1,4-diméthyl-pipérazin-1-ium.

Des bases d'oxydation du type paraphénylènediamine ou para-aminophénol substituée par un radical Z utiles pour l'invention sont aussi des bases doubles paraphénylènediamine substituées par un ou plusieurs radicaux cationiques décrites dans la demande de brevet EP 932602.

A titre d'exemple, on peut citer

- le dichlorure de 1,3-bis-1{3{3'[(4"-amino-3"-méthyl-aniline)-N-propyl]}-3H-imidazol-1-ium}-propane ;
- le dichlorure de 1,3-bis-1{3{3'[(4"-amino-2"-méthyl-aniline)-N-propyl]}-3H-imidazol-1-ium}-propane, monohydrate, diéthanol ;
- le dichlorure de 1,3-bis-1{3{3'[(4"-amino-aniline)-N-propyl]}-3H-imidazol-1-ium}-propane, monohydrate, éthanol ;
- le tétrachlorure de 1,3-bis-{3{3'[(4'amino-aniline)-N-propyl]}-3H-imidazol-1-ium} propane ;
- le dichlorure de 1,3-bis-1{3{3'[(4"-amino-aniline)-N-propyl]}-3H-imidazol-1-ium}-2-propanol, monohydrate ;
- le dibromure de N1,N3-bis-[3-N(4'-amino-aniline)-propyl]-1,1,3,3-tétraméthyl-diammonium 1-3-propane, monohydrate ;
- le dichlorure de 1,4-bis-1{3[3-(2,5-diamino-phénoxy)-propyl]-3H-imidazol-1-ium}- butane, dihydrate ;
- le monochlorure de 1,3-bis-[3-(2,5-diamino-phénoxy)-propyl]-3H-imidazol-1-ium, monohydrate ;
- le dibromure de N1,N4-bis-[3-N-méthyl-N-(4'-amino-aniline)-éthyl]-1,1,4,4-tétraméthyl-diammonium 1-3-propane, monohydrate ;
- le dichlorure de 1,4-bis-1-[3-(5-amino-2-hydroxy-benzyl)-3H-imidazol-1 ium]-butane, monohydrate ;
- le dibromure de 1,3-bis-([2-(4-amino-aniline)-propyl]-1,1,3,3-tétraméthyl-diammonium-propane ;
- le dichlorure de 1,3-bis-{[4-(4-amino-aniline)-pentyl]-1,1,3,3-tétraméthyl-diammonium-propane ;
- le monochlorure de [4-(4-amino-phénylamino)-pentyl]-(5-amino-2-hydroxy-benzyl)-diéthyl-ammonium ;
- le monochlorure de [2-(4-amino-phénylamino)-propyl]-(5-amino-2-hydroxy-benzyl)-diméthyl-ammonium ;
- le dichlorure de 1,3-bis-1-{3-[3-(2,5-diamino-phénoxy)-propyl]-3H-imidazol-1-ium}-propane, dihydrate ;
- le dichlorure de 1,3-bis-1{3{3'[(4"-amino-aniline)-N-propyl]}-3H-imidazol-1-ium}-propane ;
- le dichlorure de 1,3-bis-1{4{4'(4-[3-(4"-amino-phénylamino)-propyl] }-1,3-diméthyl-3H-imidazol-1-ium}-propane,
- le dichlorure de 1,3-bis-1{4{4'(4-[3-(4"-amino-2"-méthyl-aniline)-propyl] }-1,3-diméthyl-3H-imidazol-1-ium}-propane ;
- le monochlorure de 4-[2-(2,5-diamino-phénoxy)-éthyl]-3-[3-(2,5-diamino-phénoxy)-propyl]-1-méthyl-3-imidazol-1-ium ;
- le monochlorure de 4-[2-(2,5-diamino-phénoxy)-éthyl]-1-[3-(2,5-diamino-phénoxy)-propyl]-3-méthyl-3-imidazol-1-ium ;

et leurs sels d'addition avec un acide.

Parmi ces bases doubles, on préfère plus particulièrement :

- le dichlorure de 1,3-bis-1-(3-{3'-[(4"-amino-aniline)-N-propyl]}-3H-imidazol-1-ium}-propane, monohydrate, éthanol ;
- le monochlorure de 1,3-bis-[3-(2,5-diamino-phénoxy)-propyl]-3H-imidazol-1-ium, monohydrate ;
- le dibromure de N1,N4-bis-[3-N-méthyl-N-(4'-amino-aniline)-éthyl]-1,1,4,4-tétraméthyl-diammonium 1-3-propane, monohydrate ;
- le dichlorure de 1,4-bis-1[3-(5-amino-2-hydroxy-benzyl)-3H-imidazol-1-ium]-butane, monohydrate ;
- le dichlorure de 1,3-bis-1{3{3'[(4"-amino-aniline)-N-propy)]}-3H-imidazol-1-ium}-propane ;

et leurs sels d'addition avec un acide.

D'autres bases d'oxydation du type para-phénylènediamine ou para-aminophénol substituées par un radical Z sont des paraphénylènediamines à groupement pyrrolidinyle substitués par un ou plusieurs radicaux Z. De telles bases sont décrites dans la demande de brevet EP 1 348 695.

A titre d'exemple, on peut citer

| | | | |
|---|---|---|---|
| 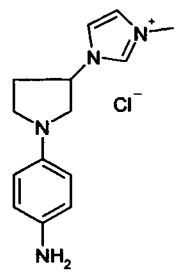 | 3-[1-(4-Amino-phényl)-pyrrolidin-3-yl]-1-méthyl-3H-imidazol-1-ium; chlorure | 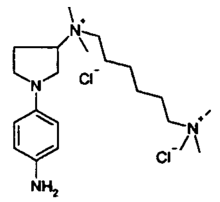 | [1-(4-Amino-phényl)-pyrrolidin-3-y)]-(-triméthylammonium-hexyl)-diméthyl-ammonium; dichlorure |
| 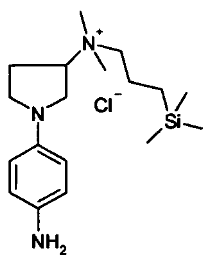 | [1-(4-Amino-phényl)-pyrrolidin-3-yl]-diméthyl-(3-triméthylsilanyl-propyl)-ammonium ; chlorure | 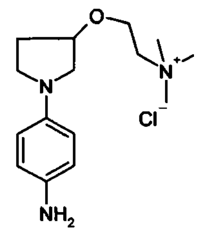 | {2-[1-(4-Amino-phényl)-pyrrolidin-3-yloxy]-ethyl}-triméthyl-ammonium; chlorure |
| 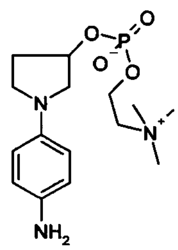 | [1-(4-Amino-phényl)-pyrrolidin-3-yl]-oxophosphorylchol ine | 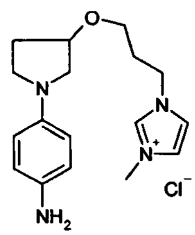 | 3-{3-[1-(4-Amino-phényl)-pyrrolidin-3-yloxy]-propyl}-1-méthyl-3H-imidazol-1-ium ; chlorure |
| 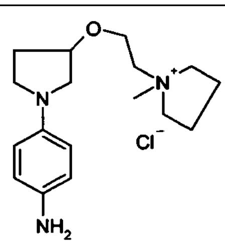 | 1-{2-[1-(4-Amino-phényl)-pyrrolidin-3-yloxy]-ethyl}-1-méthyl-pyrrolidinium; chlorure | 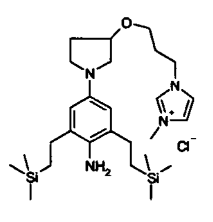 | 3-{3-[1-(5-triméthylsilanylethyl-4-Amino-3-triméthylsilanylethyl-phényl)-pyrrolidin-3-yloxy]-propyl}-1-méthyl-3H-imidazol-1-um ; chlorure |
| 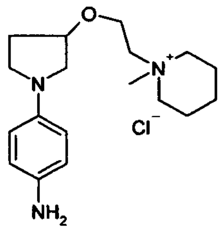 | 1-{2-[1-(4-Amino-phényl)-pyrrolidin-3-yloxy]-ethyl}-1-méthyl-piperidinium; chlorure | 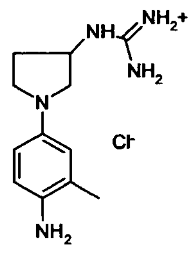 | N-[1-(4-Amino-3-méthyl-phényl)-pyrrolidin-3-yl]-guanidinium ; chlorure |

(suite)

| | | | |
|---|---|---|---|
| 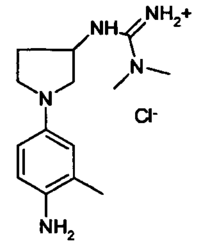 | N'-[1-(4-Amino-3-méthyl-phényl)-pyrrolidin-3-yl]-N,N-diméthyl-guanidinium ; chlorure | 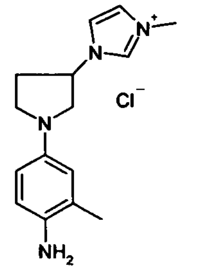 | 3-[1-(4-Amino-3-méthyl-phényl)-pyrrolidin-3-yl]-1-méthyl-3H-imidazol-1-ium; chlorure |
| 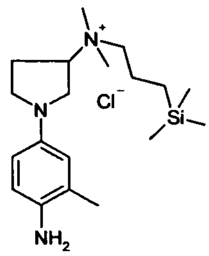 | [1-(4-Amino-3-méthyl-phényl)-pyrrolidin-3-yl]-diméthyl-(3-triméthylsilanyl-propyl ammonium ; chlorure | 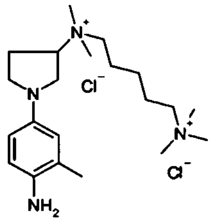 | [1-(4-Amino-3-méthyl-phényl)-pyrrolidin-3-yl]-(-triméthylammonium-hexyl)-diméthyl-ammonium dichlorure |
| 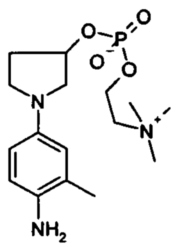 | [1-(4-Amino-3-méthyl-phényl)-pyrrolidin-3-yl]-oxophosphorylcholine | 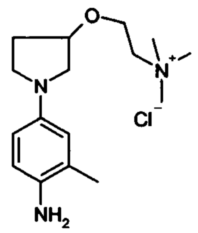 | {2-[1-(4-Amino-3-méthyl-phényl)-pyrrolidin-3-yloxy]-ethyl)-triméthyl-ammonium; chlorure |
| 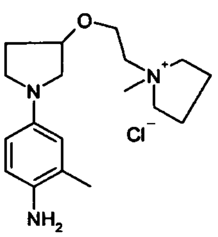 | 1-{2-[1-(4-Amino-3-méthyl-phényl)-pyrrolidin-3-yloxy]-ethyl}-1-méthyl-pyrrolidinium ; chlorure | 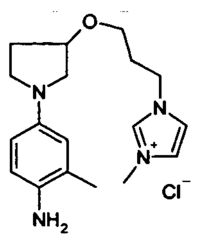 | 3-{3-[1-(4-Amino-3-méthyl-phényl)-pyrrolidin-3-yloxy]-propyl}-1-méthyl-3H-imidazol-1-um ; chlorure |
| 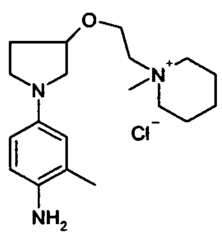 | 1-{2-[1-(4-Amino-3-méthyl-phényl)-pyrrolidin-3-yloxy]-ethyl}-1-méthyl-piperidin | 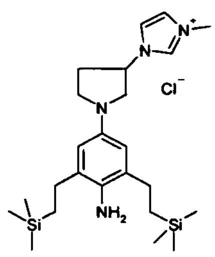 | 3-[1-(5-triméthylsilanylethyl-4-Amino-3-triméthylsilanylethyl-phényl)-pyrrolidin-3-yl]-1-méthyl-3H-imidazol-1-ium; chlorure |
| 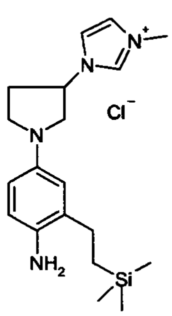 | 3-[1-(4-Amino-3-triméthylsilanylethyl-phényl)-pyrrolidin-3-yl]-1-méthyl-3H-imidazol-1-ium, chlorure | 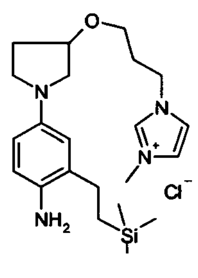 | 3-{3-[1-(4-Amino-3-triméthylsilanylethyl -phényl)-pyrrolidin-3-yloxy]-propyl}-1-méthyl-3H-imidazol-1-um ; chlorure |

(suite)

| | | | |
|---|---|---|---|
| 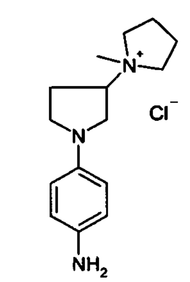 | 1'-(4-Amino-phényl)-1-méthyl-[1,3']bipyrrolidinyl-1-ium; chlorure | 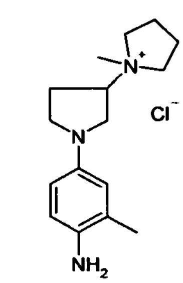 | 1'-(4-Amino-3-méthyl-phényl)-1-méthyl-[1,3']bipyrrolidinyl-1-ium; chlorure |
| 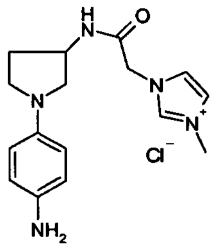 | 3-{[1-(4-Amino-phényl)-pyrrolidin-3-ylcarbamoyl]-méthyl}-1-méthyl-3H-imidazol-1-ium ; chlorure | 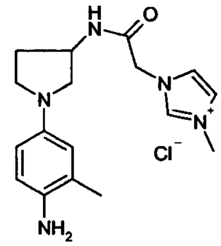 | 3-{[1-(4-Amino-3-méthyl-phényl)-pyrrolidin-3-ylcarbamoyl]-méthyl}-1-méthyl-3H-imidazol-1-ium ; chlorure |
| 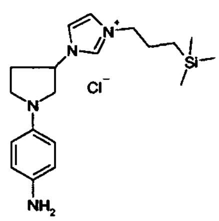 | 3-[1-(4-Amino-phényl)-pyrrolidin-3-yl]-1-(3-triméthylsilanyl-propyl)-3H-imidazol-1-ium ; chlorure | 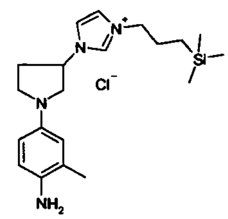 | 3-[1-(4-Amino-3-méthyl-phényl)-pyrrolidin-3-yl]-1-(3-triméthylsilanyl-propyl)-3H-imidazol-1-ium ; chlorure |
| 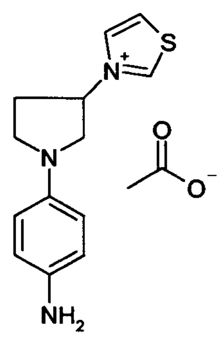 | Acétate de 3-[1-(4-Amino-phenyl)-pyrrolidin-3-yl]-thiazol-3-ium | 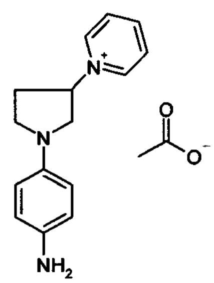 | Acétate de 1-[1-(4-aminophenyl)pyrrolidin-3-yl] pyridinium |
| 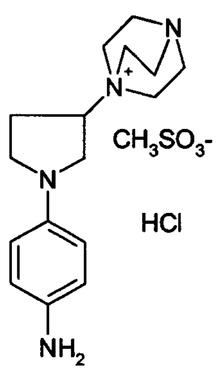 | 1-[1-(4-Amino-phenyl)-pyrrolidin-3-yl]-4-aza-1-azonia-bicyclo[2.2.2] octane ;méthanesulfonate ; chlorhydrate | | |

Parmi ces bases d'oxydation, les composés suivants sont particulièrement préférés :

- N'-[1-(4-Amino-phényl)-pyrrolidin-3-yl]-N,N-diméthyl-guanidinium ; chlorure

- N-[1-(4-Amino-phényl)-pyrrolidin-3-yl]-guanidinium ; chlorure
- 3-[1-(4-Amino-phényl)-pyrrolidin-3-yl]-1-méthyl-3H-imidazol-1-ium; chlorure
- [1-(4-Amino-phényl)-pyrrolidin-3-yl]-diméthyl-(3-triméthylsilanyl-propyl)-ammonium ; chlorure
- N'-[1-(4-Amino-3-méthyl-phényl)-pyrrolidin-3-yl]-N,N-diméthyl-guanidinium ; chlorure
- N-[1-(4-Amino-3-méthyl-phényl)-pyrrolidin-3-yl]-guanidinium ; chlorure
- 3-[1-(4-Amino-3-méthyl-phényl)-pyrrolidin-3-yl]-1-méthyl-3H-imidazol-1-ium; chlorure
- [1-(4-amino 3-méthyl phényl) pyrrolidin 3 -yl] diméthyl (3-triméthylsilanyl propyl ammonium ; chlorure ;
- 3-[1-(4-Amino-phényl)-pyrrolidin-3-yl]-1-(3-triméthylsilanyl-propyl)-3H-imidazol-1-ium ; chlorure
- 3-[1-(4-Amino-3-méthyl-phényl)-pyrrolidin-3-yl]-1-(3-triméthylsilanyl-propyl)-3H-imidazol-1-ium ; chlorure
- 1'-(4-Amino-phényl)-1-méthyl-[1 ,3']bipyrrolidinyl-1-ium; chlorure
- 1'-(4-Amino-3-méthyl-phényl)-1-méthyl-[1,3']bipyrrolidinyl-1-ium; chlorure
- 3-{[1-(4-Amino-phényl)-pyrrolidin-3-ylcarbamoyl]-méthyl}-1-méthyl-3H-imidazol-1-ium ; chlorure
- 3-{[1-(4-Amino-3-méthyl-phényl)-pyrrolidin-3-ylcarbamoyl]-méthyl}-1-méthyl-3H- imidazol-1-ium ; chlorure
- 1'-(4-amino-phényl)-1-méthyl-[1,3']bipyrrolidinyl-1-ium, chlorure.

On peut aussi citer à titre de bases d'oxydation du type para-phénylènediamine substituées par un ou plusieurs radicaux Z, des bases bis-paraphénylènediamine à groupement pyrrolidinyle substitué par un ou plusieurs radicaux ammoniums quaternaires telles que décrites dans la demande de brevet EP 1 396 486.

A titre d'exemple, on peut citer

| Structure | Nomenclature |
|---|---|
| 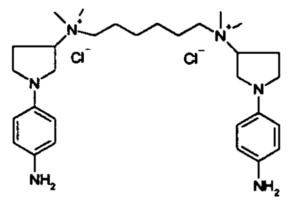 | N,N'-bis[1-(4-aminophenyl)pyrrolidin-3-yl]-N,N,N',N'-tetramethylhexane-1,6-diaminium dichlorure |
| 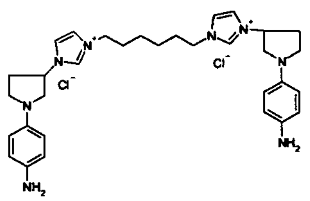 | 3-[1-(4-aminophenyl)pyrrolidin-3-yl]-1-(6-{1-[1-(4-aminophenyl)pyrrolidin-3-y)]-1H-imidazol-3-ium-3-yl}hexyl)-1H-imidazol-3-ium dichlorure |
| 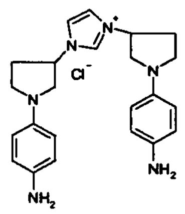 | 1,3-bis[1-(4-aminophenyl)pyrrolidin-3-yl]-1H-imidazol-3-ium chlorure |
| 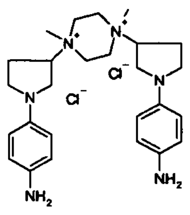 | 1,4-bis[1-(4-aminophenyl)pyrrolidin-3-yl]-1,4-dimethylpiperazinediium dichlorure |

(suite)

| Structure | Nomenclature |
|---|---|
| 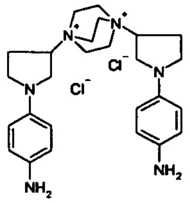 | 1,4-bis[1-(4-aminophenyl)pyrrolidin-3-yl]-1,4-diazoniabicyclo[2.2.2]octane dichlorure |
| 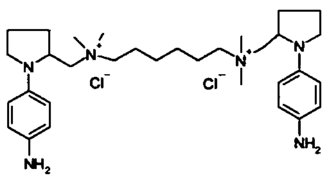 | N,N'-bis{[1-(4-aminophenyl)pyrrolidin-2-yl]methyl}-N,N,N',N'-tetramethylhexane-1,6-diaminium dichlorure |
| 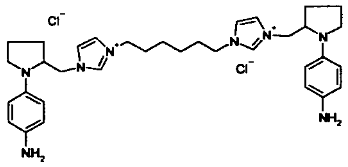 | 3-{[1-(4-aminophenyl)pyrrolidin-2-yl]methyl}-1-[6-(1-([1-(4-aminophenyl) pyrrolidin-2-yl]methyl}-1H-imidazol-3-ium-3-yl)hexyl]-1H-imidazol-3-ium dichlorure |
| 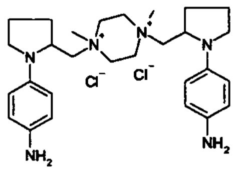 | 1,4-bis([1-(4-aminophenyl)pyrrolidin-2-yl]methyl}-1,4-dimethylpiperazinediium dichlorure |
| 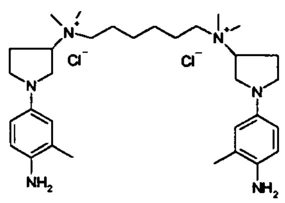 | N,N'-bis[1-(4-amino-3-methylphenyl)pyrrolidin-3-yl]-N,N,N',N'-tetramethylhexane-1,6-diaminium dichlorure |
| 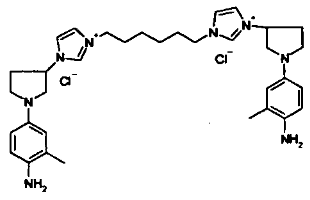 | 3-[1-(4-amino-3-methylphenyl)pyrrolidin-3-yl]-1-(6-{1-[1-(4-amino-3-methylphenyl)pyrrolidin-3-yl]-1H-imidazol-3-ium-3-yl}hexyl)-1H-imidazol-3-ium dichlorure |
| 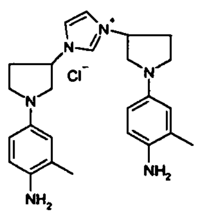 | 1,3-bis[1-(4-amino-3-methylphenyl)pyrrolidin-3-yl]-1H-imidazol-3-ium chlorure |

(suite)

| Structure | Nomenclature |
|---|---|
| 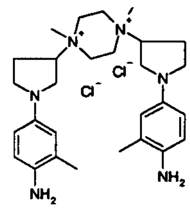 | 1,4-bis[1-(4-amino-3-methylphenyl)pyrrolidin-3-yl]-1,4-dimethylpiperazinediium dichlorure |
| 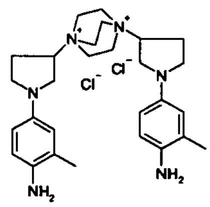 | 1,4-bis[1-(4-amino-3-methylphenyl)pyrrolidin-3-yl]-1,4-diazoniabicyclo[2.2.2] octane dichlorure |
| 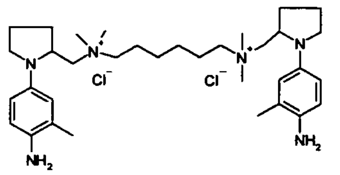 | N,N'-bis{[1-(4-amino-3-methylphenyl)pyrrolidin-2-yl]methyl}-N,N,N',N'-tetramethylhexane-1,6-diaminium dichlorure |
| 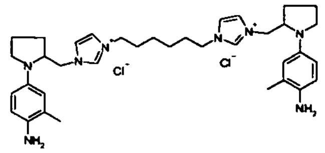 | 3-{[1-(4-amino-3-methylphenyl)pyrrolidin-2-yl]methyl}-1-[6-(1-([1-(4-amino-3-methy)phenyl)pyrrolidin-2-y)]methyl}-1H-imidazol-3-ium-3-yl)hexyl]-1H-imidazol-3-ium dichlorure |
| 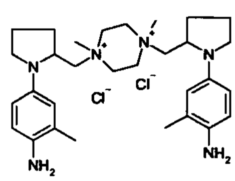 | 1,4-bis{[1-(4-amino-3-methylphenyl)pyrrolidin-2-yl]methyl}-1,4-dimethylpiperazinediium dichlorure |
| 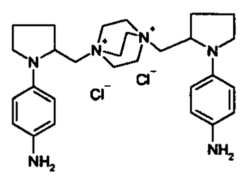 | 1,4-bis{[1-(4-aminophenyl)pyrrolidin-2-yl]methyl}-1,4-diazoniabicyclo[2.2.2] octane dichlorure |
| 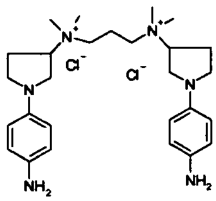 | N,N'-bis[1-(4-aminophenyl)pyrrolidin-3-yl]-N,N,N',N'-tetramethylpropane-1,3-diaminium dichlorure |
| 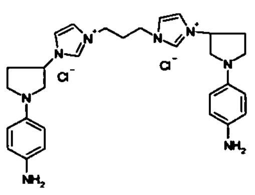 | 3-[1-(4-aminophenyl)pyrrolidin-3-yl]-1-(3-{1-[1-(4-aminophenyl)pyrrolidin-3-yl]-1H-imidazol-3-ium-3-yl}propyl)-1H-imidazol-3-ium dichlorure |

(suite)

| Structure | Nomenclature |
|---|---|
| 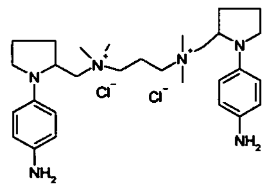 | N,N'-bis{[1-(4-aminophenyl)pyrrolidin-2-yl]methyl}-N,N,N',N'-tetramethyl propane-1,3-diaminium dichlorure |
| 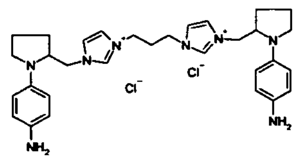 | 3-{[1-(4-aminophenyl)pyrrolidin-2-yl]methyl}-1-[3-(1-{[1-(4-aminophenyl) pyrrolidin-2-yl]methyl}-1H-imidazol-3-ium-3-yl)propyl]-1H-imidazol-3-ium dichlorure |
| 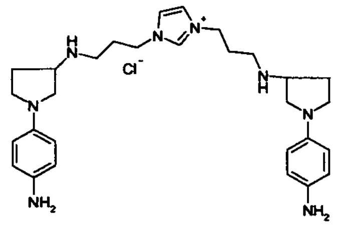 | 1,3-bis(3-{[1-(4-aminophenyl)pyrrolidin-3-yl]amino}propyl)-1H-imidazol-3-ium chlorure |
| 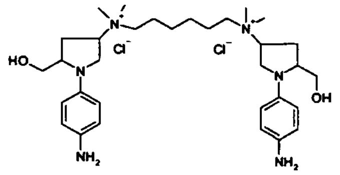 | N,N'-bis[1-(4-aminophenyl)-5-(hydroxymethyl)pyrrolidin-3-yl]-N,N,N',N'-tetramethylhexane-1,6-diaminium dichlorure |
| 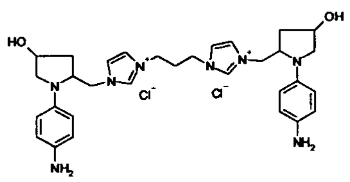 | 3-{[1-(4-aminophenyl)-4-hydroxypyrrolidin-2-yl]methyl}-1-[3-(1-{[1-(4-aminophenyl)-4-hydroxypyrrolidin-2-yl]methyl}-1H-imidazol-3-ium-3-yl) propyl]-1H-imidazol-3-ium dichlorure |
| 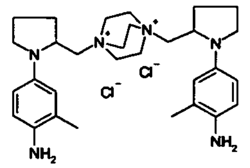 | 1,4-bis{[1-(4-amino-3-methylphenyl)pyrrolidin-2-yl]methyl}-1,4-diazoniabicyclo[2.2.2]octane dichlorure |
| 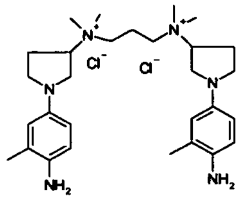 | N,N'-bis[1-(4-amino-3-methylphenyl)pyrrolidin-3-yl]-N,N,N',N'-tetramethylpropane-1,3-diaminium dichlorure |

(suite)

| Structure | Nomenclature |
|---|---|
| 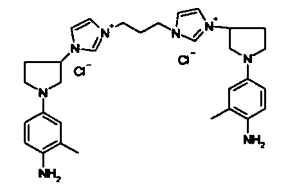 | 3-[1-(4-amino-3-methylphenyl)pyrrolidin-3-yl]-1-(3-{1-[1-(4-amino-3-methylphenyl)pyrrolidin-3-yl]-1H-imidazol-3-ium-3-yl}propyl)-1H-imidazol-3-ium dichlorure |
| 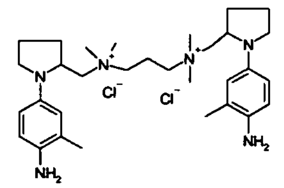 | N,N'-bis{[1-(4-amino-3-methylphenyl)pyrrolidin-2-yl]methyl}-N, N,N',N'-tetramethylpropane-1,3-diaminium dichlorure |
| 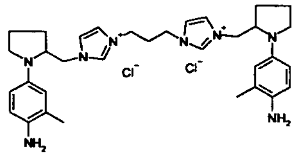 | 3-{[1-(4-amino-3-methylphenyl)pyrrolidin-2-y)]methyl}-1-[3-(1-{[1-(4-amino-3-methylphenyl)pyrrolidin-2-yl]methyl}-1H-imidazol-3-ium-3-yl)propyl]-1H-imidazol-3-ium dichlorure |
| 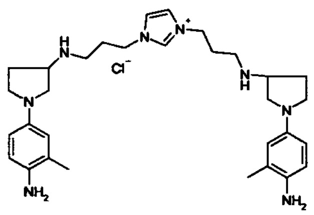 | 1,3-bis(3-{[1-(4-amino-3-methylphenyl)pyrrolidin-3-yl]amino}propyl)-1H-imidazol-3-ium chlorure |
| 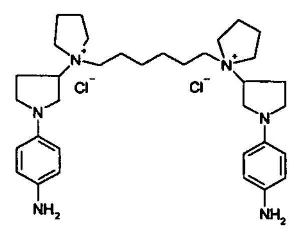 | N,N'-bis[1-(4-aminophenyl)pyrrolidin-3-yl]-N,N',dipyrrolidinehexane-1,6-diaminium dichlorure |
| 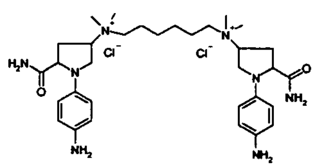 | N,N'-bis[1-(4-aminophenyl)pyrrolidin-5-amido-3-yl]-N,N,N',N'-tetramethylhexane-1,6-diaminium dichlorure |
| 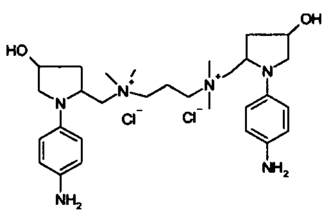 | N,N'-bis{[1-(4-aminophenyl)-4-hydroxypyrrolidin-2-yl]methy}-N,N,N',N'-tetramethylpropane-1,3-diaminium dichlorure |

Des bases d'oxydation du type ortho-phénylènediamine substituées par un radical Z utiles dans la présente invention sont par exemple décrites dans la demande de brevet EP 983 996 ou EP 984006.
A titre d'exemple, on peut notamment citer :

- le monochlorure de {2-[2-amino-phénylamino]-éthyl}-triméthyl-ammonium, monohydrate ;
- le monochlorure de [2-(2-amino-5-chloro-phénylamino)-éthyl]-triméthylammonium ;
- le monochlorure de [2-(2-amino-6-chloro-phénylamino)-éthyl]-triméthylammonium ;
- le monochlorure de [2-(2-amino-4-chloro-phénylamino)-éthyl]-triméthylammonium ;
- le monochlorure de {2-[2-amino-4-chloro-5-(2-hydroxyéthoxy)-phénylamino]-éthyl}-triméthyl-ammonium ;
- le monochlorure de [2-(2-amino-5-méthoxy-phénylamino)-éthyl]-triméthylammonium ;
- le monobromure de [2-(2-amino-phénylamino)-éthyl]-(2-hydroxyethyl)-diméthyl-ammonium ;
- le monochlorure de 4-[2-(2-amino-phénylamino)-éthyl]-4-méthyl-morpholin-4-ium ;
- le monochlorure de 1-[2-(2-amino-phénylamino)-éthyl]-1-éthyl-pipéridinium ;
- le monochlorure de 1-[2-(2-amino-phénylamino)-éthyl]-1,4-diméthyl-pipérazin-1-ium ;
- le dichlorure de 4-[2-(1-méthyl-pipéridinium)-éthoxy]-N2-[2-(1-méthyl-pipéridinium)-éthyl]-benzène-1,2-diamine ;
- le monochlorure de 1-[2-(2-amino-5-méthylsulfanyl-phénylamino)-éthyl]-1-méthyl-pipéridinium ;
- le monochlorure de 1-[2-(2-amino-phénylamino)-éthyl]-1-méthyl-pyrrolidinium ;
- le monochlorure de [3-(2-amino-phénylamino)-propyl]-diéthyl-méthyl-ammonium ;
- le dichlorure de N,N'-bis-[2-(1-méthyl-pipéridinium)-éthyl]-benzène-1,2-diamine ;
- le monochlorure de [2-(2-amino-4-méthyl-phénylamino)-éthyl]-triméthyl- ammonium ; et leurs sels d'addition avec un acide.

Des bases d'oxydation hétérocycliques du type pyrazolo[1,5-a]pyrimidine substituées par un radical Z et utiles dans la présente invention sont par exemple décrites dans la demande de brevet EP 1 147 109.
A titre d'exemple, on peut notamment citer :

- le chlorure de 3-[3-(3-amino-5-méthyl-pyrazolo[1,5-a]pyrimidin-7-ylamino)-propyl]-1-(2-hydroxyéthyl)-3H-imidazol-1-ium,
- le chlorure de 3-[(3-amino-pyrazolo[1,5-a]pyrimidin-7-ylcarbamoyl)-méthyl]-1-méthyl-3H-imidazol-1-ium,
- le méthyl sulfate de 3-(3-amino-7-hydroxy-5-méthyl-pyrazolo[1,5-a]pyrimidin-6-ylméthyl)-1-méthyl-pyridinium,
- le chlorure de 3-(3-amino-7-hydroxy-5-méthyl-pyrazolo[1,5-a]pyrimidin-6-ylméthyl)-1-(2-hydroxy-éthyl)-pyridinium,
- le méthyl sulfate de 2-[(3-amino-pyrazolo[1,5-a]pyrimidin-7-ylamino)-méthyl]-1,3-diméthyl-3H-imidazol-1-ium,
- le méthyl sulfate de 3-[(3-amino-pyrazolo[1,5-a]pyrimidin-7-ylamino)-méthyl]-1-méthyl-pyridinium,
- le méthyl sulfate de 3-[(3-amino-pyrazolo[1,5-a]pyrimidin-7-ylamino)-méthyl]-1-méthyl-pyridinium,
- le méthyl sulfate de 2-(3,7-diamino-5-méthyl-pyrazolo[1,5-a]pyrimidin-6-ylméthyl)-1,3-diméthyl-3H-imidazol-1-ium,
- le méthyl sulfate de 2-(3-amino-7-hydroxy-5-méthyl-pyrazolo[1,5-a]pyrimidin-6-ylméthyl)-1,3-diméthyl-3H-imidazol-1-ium,
- le méthyl sulfate de 2-(3,7-diamino-pyrazolo[1,5-a]pyrimidin-2-yl)-1-méthyl-pyridinium,
- le chlorure de [3-(3-amino-5-méthyl-pyrazolo[1,5-a]pyrimidin-7-ylamino)-propyl]-triméthyl-ammonium,
- le méthyl sulfate de [3-(3-amino-5-méthyl-pyrazolo[1,5-a]pyrimidin-7-ylamino)-propyl]-triméthyl-ammonium,
- le chlorure de 1-[3-(3-amino-5-méthyl-pyrazolo[1,5-a]pyrimidin-7-ylamino)-propyl]-1-méthyl-pipéridinium,
- le méthyl sulfate de 1-[3-(3-amino-5-méthyl-pyrazolo[1,5-a]pyrimidin-7-ylamino)-propyl]-1-méthyl-pipéridinium,
- le chlorure de 4-[3-(3-amino-5-méthyl-pyrazolo[1,5-a]pyrimidin-7-ylamino)-propyl]-4-méthyl-morpholin-4-ium,
- le méthyl sulfate de 4-[3-(3-amino-5-méthyl-pyrazolo[1,5-a]pyrimidin-7-ylamino)-propyl]-4-méthyl-morpholin-4-ium,

et leurs sels d'addition avec un acide.
Parmi ces composés de formule (I), on préfère plus particulièrement :

- le chlorure de 3-[3-(3-amino-5-méthyl-pyrazolo[1,5-a]pyrimidin-7-ylamino)-propyl]-1-(2-hydroxyéthyl)-3H-imidazol-1-ium,
- le méthyl sulfate de 3-(3-amino-7-hydroxy-5-méthyl-pyrazolo[1,5-a]pyrimidin-6-ylméthyl)-1-méthyl-pyridinium,
- le chlorure de 3-(3-amino-7-hydroxy-5-méthyl-pyrazolo[1,5-a]pyrimidin-6-ylméthyl)-1-(2-hydroxy-éthyl)-pyridinium,
- le chlorure de 3-(3-amino-7-hydroxy-5-méthyl-pyrazolo[1,5-a]pyrimidin-6-ylméthyl)-1-méthyl-pyridinium,
- le chlorure de 4-[3-(3-amino-5-méthyl-pyrazolo[1,5-a]pyrimidin-7-ylamino)-propyl]-4-méthyl-morpholin-4-ium,
- le méthyl sulfate de 4-[3-(3-amino-5-méthyl-pyrazolo[1,5-a]pyrimidin-7-ylamino)-propyl]-4-méthyl-morpholin-4-ium,

et leurs sels d'addition avec un acide.
A titre d'exemple de bases orthophénylènediamine double substitué par un ou plusieurs radicaux Z, on peut citer :

- le dibromure de 1,3-bis-{3-{3-[(2-amino-aniline)-N-propyl]}-3H-imidazol-1-ium}-propane,
- le dibromure de N1,N3-bis-[3-N-(2-amino-aniline)-propyl]-1,1,3,3-tétraméthyl-diammonium 1-3-propane,

- le dichlorure de 1,4-bis-{3-{2-[(2-amino-aniline)-N-éthyl]}-3H-imidazol-1-ium}-butane,
- le monochlorure de 1-[2-(2-amino-aniline)-éthyl]-3-[3-(2-amino-aniline)-propyl]-3H-imidazol-1-ium,

et leurs sels d'addition avec un acide.

Des bases d'oxydation hétérocycliques du type pyrazole substituées par un radical Z et utiles dans la présente invention sont par exemple décrites dans la demande de brevet EP 1 147 090.
A titre d'exemple, on peut notamment citer :

- le chlorure de [3-(4-amino-2,5-diméthyl-2H-pyrazol-3-ylamino)-propyl]-triméthyl-ammonium ;
- le chlorure de [3-(4-amino-2,5-diméthyl-2H-pyrazol-3-ylamino)-propyl]-(2-hydroxy-éthyl)-diméthyl-ammonium ;
- le chlorure de 3-[3-(4-amino-2,5-diméthyl-2H-pyrazol-3-ylamino)-propyl]-1-(2-hydroxy-éthyl)-3H-imidazol-1-ium ;
- le chlorure de 3-[(4-amino-2H-pyrazol-3-ylcarbamoyl)-méthyl]-1-méthyl-3H-imidazol-1-ium ;
- le chlorure de 3-[2-(4,5-diamino-3-méthyl-pyrazol-1-yl)-éthyl]-1-méthyl-3H-imidazol-1-ium ;
- le chlorure de 3-[2-(4,5-diamino-pyrazol-1-yl)-éthyl]-1-méthyl-3H-imidazol-1-ium;
- le chlorure de [2-(4,5-diamino-3-méthyl-pyrazol-1-yl)-éthyl]-triméthylammonium ;
- le chlorure de [2-(4,5-diamino-pyrazol-1-yl)-éthyl]-triméthyl-ammonium ;
- le chlorure de [2-(4-amino-5-hydroxy-pyrazol-1-yl)-éthyl]-triméthyl-ammonium ;
- le chlorure de [2-(4-amino-5-hydroxy-3-méthyl-pyrazol-1-yl)-éthyl]-triméthylammonium ;
- le chlorure de 3-[2-(4-amino-5-hydroxy-3-méthyl-pyrazol-1-yl)-éthyl]-1-méthyl-3H-imidazol-1-ium ;
- le chlorure de 3-[2-(4-amino-5-hydroxy-pyrazol-1-yl)-éthyl]-1-méthyl-3H-imidazol-1-ium ;
- le chlorure de 3-[2-(4,5-diamino-1-méthyl-1H-pyrazol-3-yl)-éthyl]-1-méthyl-3H-imidazol-1-ium,

et leurs sels d'addition avec un acide ou avec une base, et leurs possibles formes tautomères.

Parmi ces composés de formule (I), on préfère plus particulièrement :

- le chlorure de [3-(4-amino-2,5-diméthyl-2H-pyrazol-3-ylamino)-propyl]-triméthyl-ammonium ;
- le chlorure de [3-(4-amino-2,5-diméthyl-2H-pyrazol-3-ylamino)-propyl]-(2-hydroxy-éthyl)-diméthyl-ammonium ;
- le chlorure de 3-[3-(4-amino-2,5-diméthyl-2H-pyrazol-3-ylamino)-propyl]-1-(2-hydroxy-éthyl)-3H-imidazol-1-ium ;
- le chlorure de 3-[(4-amino-2H-pyrazol-3-ylcarbamoyl)-méthyl]-1-méthyl-3H-imidazol-1-ium ;
- le chlorure de 3-[2-(4,5-diamino-3-méthyl-pyrazol-1-yl)-éthyl]-1-méthyl-3H-imidazol-1-ium ;
- le chlorure de 3-[2-(4,5-diamino-1-méthyl-1H-pyrazol-3-yl)-éthyl]-1-méthyl-3H-imidazol-1-ium ;

et leurs sels d'addition avec un acide ou avec une base, et leurs possibles formes tautomères.

Des coupleurs d'oxydation substitués par un ou plusieurs radicaux Z et utiles dans la présente invention sont par exemple des coupleurs méta-phénylènediamines, méta-aminophénols, méta-diphénols, naphtaléniques, ou les coupleurs hétérocycliques et leur sels d'addition.

A titre de coupleurs méta-phénylènediamine ou méta-aminophénol substitués par un ou plusieurs radicaux Z, on peut citer ceux décrits dans la demande de brevet EP 1 064 252, EP 1 064 267 et EP 1 064 268.

A titre d'exemple de coupleurs méta-phénylènediamine ou méta-aminophénol monobenzéniques, on peut notamment citer :

- le chlorure de [2-(2,4-diamino-phénoxy)-éthyl]-diéthyl-méthyl-ammonium ;
- le chlorure de 1-[3-(2,4-diamino-phénoxy)-propyl]-1,4-diméthyl-pipérazin-1-ium ;
- le chlorure de 1-[(3-hydroxy-4-méthyl-phénylcarbamoyl)-méthyl]-1,4-diméthyl-pipérazin-1-ium ;
- le chlorure de 1,4-bis-(2-hydroxy-éthyl)-1-[(3-hydroxy-phénylcarbamoyl)-méthyl]-piperazin-1-ium ;
- le chlorure de [3-(2,4-diamino-phénoxy)-propyl]-triéthyl-ammonium ;
- le chlorure de 1-[(2,4-dihydroxy-phénylcarbamoyl)-méthyl]-1-méthyl-pipéridinium ;
- le chlorure de [2-(2,4-dihydroxy-phényl)-2-oxo-éthyl]-triéthyl-ammonium ;
- le chlorure de 1-[(3-hydroxy-4-méthyl-phénylcarbamoyl)-méthyl]-1-méthyl-pyrrolidinium ;
- le chlorure de 1-[(2,6-dihydroxy-phénylcarbamoyl)-méthyl]-1-méthyl-pyrrolidinium ;
- le chlorure de [2-(4-amino-2-hydroxy-phénoxy)-éthyl]-diéthyl-méthyl-ammonium ;
- le bromure de triéthyl-[2-(3-hydroxy-4-méthyl-phénylamino)-éthyl]-ammonium ;
- le bromure de 1-[2-(3-hydroxy-4-méthyl-phénylamino)-éthyl]-1,4-diméthyl-piperazin-1-ium ;
- le chlorure de 4-[(3-hydroxy-phénylcarbamoyl)-méthyl]-4-méthyl-morpholin-4-ium ;
- le chlorure de triéthyl-[2-(3-hydroxy-2,4-diméthyl-phénylcarbamoyloxy)-éthyl]-ammonium ;
- le bromure de [2-(4-chloro-3-hydroxy-phénylamino)-éthyl]-triéthyl-ammonium ;
- le chlorure de 1-[3-(2-amino-4-méthylamino-phénoxy)-propyl]-1-méthyl-pipéridinium ;
- le chlorure de [2-(2,4-diamino-phénoxy)-éthyl]-diéthyl-méthyl-ammonium ;

- le dichlorure de 1-(3-triméthylammonium-2-hydroxy-propyl)-1-[(3-hydroxy-4-méthyl-phénylcarbamoyl)-méthyl]-pyrrolidinium ;
- le bromure de 1-[2-(3-amino-4-méthoxy-phénylamino)-éthyl]-1,4-diméthyl-pipérazin-1-ium ;
- le chlorure de [2-(2,4-diamino-phényl)-éthyl]-triéthyl-ammonium ;
- le chlorure de 1-[(3-hydroxy-2,4-diméthyl-phénylcarbamoyl)-méthyl]-1 ,4-diméthyl-pipérazin-1-ium ;
- le dichlorure de N,N-bis-[2-(1-méthyl-pyrrolidinium)-éthyl]-benzène-1,3-diamine ;
- le chlorure de triéthyl-[(3-hydroxy-4-méthyl-phénylcarbamoyl)-méthyl]-ammonium ;
- le dichlorure de N,N'-bis-(2-[1,4-bis-(2-hydroxy-éthyl)-pipérazin-1-ium]-éthyl}-benzène-1,3-diamine-2-méthyl ;
- le chlorure de 1-[3-(2,4-diamino-phénoxy)-propyl]-1-méthyl-pyrrolidinium ;
- le chlorure de 1-[(3-hydroxy-phénylcarbamoyl)-méthyl]-1,4-diméthyl-pipérazin-1-ium ;
- le trichlorure de 1-[3-(2,4-diamino-phénoxy)-propyl]-4-(3-triméthylamonium-2-hydroxy-propyl)-1 ,4-diméthyl-pipérazin-1 ,4-di-ium ;
- le iodure de [2-[4-(diméthylamino)-salicylamido]-éthyl]-diéthyl-méthyl ammonium ;
- le bromure d'éthyl-(2-hydroxyéthyl)-diméthyl-ammonium 4-(méthylamino)-salicylate ;
- le iodure de 3-[(4-amino-2-hydroxybenzoyl)-oxy]-N-éthyl-N,N-diméthyl-1-propanaminium ;
- le iodure de 3-[(4-amino-2-hydroxybenzoyl)-oxy]-N,N,N-triméthyl-1-propanaminium ;
- le bromure de triéthyl-(2-hydroxyéthyl)-ammonium 4-aminosalicylate ;
- le iodure de 2-[(4-amino-2-hydroxybenzoyl)-oxy]-N,N-diéthyl-N-méthyl-éthanaminium ;
- le iodure de 2-[(4-amino-2-hydroxybenzoyl)-oxy]-N-éthyl-N,N-diméthyl-éthanaminium ;
- le bromure d'éthyl-(2-hydroxyéthyl)-diméthyl-ammonium 4-aminosalicylate ;
- le iodure de 2-[(4-amino-2-hydroxybenzoyl)-oxy]-N,N,N-triméthyl-éthanaminium ;

et leurs sels d'addition avec un acide.

Parmi ces composés ci dessus, on préfère :

- le chlorure de [2-(2,4-diamino-phénoxy)-éthyl]-diéthyl-méthyl-ammonium ;
- le chlorure de 1-[3-(2,4-diamino-phénoxy)-propyl]-1,4-diméthyl-pipérazin-1-ium ;
- le chlorure de 1-[(3-hydroxy-4-méthyl-phénylcarbamoyl)-méthyl]-1,4-diméthyl-piperazin-1-ium ;
- le chlorure de 1,4-bis-(2-hydroxy-éthyl)-1-[(3-hydroxy-phénylcarbamoyl)-méthyl]-piperazin-1-ium ;
- le chlorure de [3-(2,4-diamino-phénoxy)-propyl]-triéthyl-ammonium ;
- le chlorure de 1-[(2,4-dihydroxy-phénylcarbamoyl)-méthyl]-1-méthyl-pipéridinium ;
- le chlorure de [2-(2,4-dihydroxy-phényl)-2-oxo-éthyl]-triéthyl-ammonium ;
- le chlorure de 1-[(3-hydroxy-4-méthyl-phénylcarbamoyl)-méthyl]-1-méthyl-pyrrolidinium ;
- le chlorure de 1-[(2,6-dihydroxy-phénylcarbamoyl)-méthyl]-1-méthyl-pyrrolidinium ;
- le chlorure de [2-(4-amino-2-hydroxy-phénoxy)-éthyl]-diéthyl-méthyl-ammonium ;
- le bromure de triéthyl-[2-(3-hydroxy-4-méthyl-phénylamino)-éthyl]-ammonium ;
- le bromure de 1-[2-(3-hydroxy-4-méthyl-phénylamino)-éthyl]-1,4-diméthyl-pipérazin-1-ium ;
- le chlorure de 4-[(3-hydroxy-phénylcarbamoyl)-méthyl]-4-méthyl-morpholin-4-ium;
- le chlorure de triéthyl-[2-(3-hydroxy-2,4-diméthyl-phénylcarbamoyloxy)-éthyl]-ammonium ;
- le bromure de [2-(4-chloro-3-hydroxy-phénylamino)-éthyl]-triéthyl-ammonium ;
- le chlorure de 1-[3-(2-amino-4-méthylamino-phénoxy)-propyl]-1-méthyl-pipéridinium ;
- le chlorure de [2-(2,4-diamino-phénoxy)-éthyl]-diéthyl-méthyl-ammonium;
- le dichlorure de 1-(3-triméthylammonium-2-hydroxy-propyl)-1-[(3-hydroxy-4-méthyl-phénylcarbamoyl)-méthyl]-pyrrolidinium ;
- le bromure de 1-[2-(3-amino-4-méthoxy-phénylamino)-éthyl]-1,4-diméthyl-pipérazin-1-ium ;
- le chlorure de [2-(2,4-diamino-phényl)-éthyl]-triéthyl-ammonium ;
- le chlorure de 1-[(3-hydroxy-2,4-diméthyl-phénylcarbamoyl)-méthyl]-1,4-diméthyl-pipérazin-1-ium ,
- le dichlorure de N,N-bis-[2-(1-méthyl-pyrrolidinium)-éthyl]-benzène-1,3-diamine ;
- le chlorure de triéthyl-[(3-hydroxy-4-méthyl-phénylcarbamoyl)-méthyl]-ammonium ;
- le dichlorure de N,N'-bis-(2-[1,4-bis-(2-hydroxy-éthyl)-pipérazin-1-ium]-éthyl}-benzène-1,3-diamine-2-méthyl ;
- le chlorure de 1-[3-(2,4-diamino-phénoxy)-propyl]-1-méthyl-pyrrolidinium;
- le chlorure de 1-[(3-hydroxy-phénylcarbamoyl)-méthyl]-1,4-diméthyl-pipérazin-1-ium ;
- le trichlorure de 1-[3-(2,4-diamino-phénoxy)-propyl]-4-(3-triméthylamonium-2-hydroxy-propyl)-1,4-diméthyl-pipérazin-1,4-di-ium ;

et leurs sels d'addition avec un acide.

A titre de coupleurs monobenzéniques substitués par un radical Z cyclique, on peut citer :

- le chlorure de 1-[3-(2,4-diamino-phénoxy)-propyl]-3-méthyl-3H-imidazol-1-ium ;
- le chlorure de 1-[(3-hydroxy-4-méthyl-phénylcarbamoyl)-méthyl]-3-méthyl-3H-imidazol-1-ium ;
- le chlorure de 3-éthyl-1-[(3-hydroxy-phénylcarbamoyl)-méthyl]-3H-imidazol-1-ium ;
- le chlorure de 1-[(3-hydroxy-2,4-diméthyl-phénylcarbamoyl)-méthyl]-3-méthyl-3H-imidazol-1-ium ;
- le chlorure de 1-[(4-chloro-3-hydroxy-phénylcarbamoyl)-méthyl]-3-méthyl-3H-imidazol-1-ium ;
- le chlorure de 1-[(3-hydroxy-4-méthoxy-phénylcarbamoyl)-méthyl]-3-méthyl-3H-imidazol-1-ium ;
- le chlorure de 1-[(3-hydroxy-4-méthyl-phénylcarbamoyl)-méthyl]-2-méthyl-2H-pyrazol-1-ium ;
- le bromure de 1-[2-(3-hydroxy-4-méthyl-phénylamino)-éthyl]-3-méthyl-3H-imidazol-1-ium ;
- le chlorure de 1-[2-(3-hydroxy-4-méthyl-phénylcarbamoyloxy)-éthyl]-2,3-diméthyl-3H-imidazol-1-ium ;
- le dichlorure de 1-{[3-amino-4-(3-(3-méthyl-3H-imidazol-1-ium)-propoxy)-phénylcarbamoyl]-méthyl)-3-methyl-3H-imidazol-1-ium,
- le dichlorure de 3-(3-triméthylammonium-2-hydroxy-propyl)-1-[(3-hydroxy-4-méthyl-phénylcarbamoyl)-méthyl]-3H-imidazol-1-ium,
- le chlorure de 1-{[2-(2,4-diamino-phénoxy)-éthylcarbamoyl]-méthyl}-3-méthyl-3H-imidazol-1-ium ;
- le chlorure de 1-[(2,4-dihydroxy-phénylcarbamoyl)-méthyl]-3-méthyl-3H-imidazol-1-ium ;
- le dichlorure de N,N-bis-[2-(3-méthyl-3H-imidazol-1-ium)-éthyl]-benzene-1,3-diamine ;
- le dichlorure de 1-{3-[4-amino-2-(2-triéthylammonium-acétylamino)-phénoxy]-propyl}-3-méthyl-3H-imidazol-1-ium ;
- le dichlorure de 1-(3-{4-amino-2-[2-(3-méthyl-3H-imidazol-1-ium)-acétylamino]-phénoxy}-propyl)-1,4-diméthyl-pipérazin-1-ium ;
- le chlorure de 1-[2-(2,4-dihydroxy-phényl)-2-oxo-éthyl]-3-méthyl-3H-imidazol-1-ium ;
- le chlorure de 1-[2-(2,4-diamino-phényl)-éthyl]-3-méthyl-3H-imidazol-1-ium ;

et leurs sels d'addition avec un acide.

A titre de coupleurs dibenzéniques, on peut citer,

- le dichlorure de 1,4-bis-1-{3-[3-(2,4-diamino-phénoxy)-propyl]-3H-imidazol-1-ium}-butane, monohydrate ;
- le chlorure de 1,3-bis-[3-(2,4-diamino-phénoxy)-propyl]-3H-imidazol-1-ium ;
- le chlorure de 3-[3-(2,4-diamino-phénoxy)-propyl]-1-[(3-hydroxy-4-méthyl-phénylcarbamoyl)-méthyl]-3H-imidazol-1-ium ;
- le dichlorure de 1,4-bis-{3-[(3-hydroxy-4-méthyl-phénylcarbamoyl)-méthyl]-3H-imidazol-1-ium}-butane ;
- le dichlorure de 1,4-bis-[3-(2,4-diamino-phénoxy)-propyl]-1,4-diméthyl-piperazin-1,4-di-ium ;
- le dichlorure de 1,4-bis-{3-[2-(2,4diamino-phényl)-éthyl]-3H-imidazol-1-ium}-butane ;
- le dichlorure de 1-[3-(2,4-diamino-phénoxy)-propyl]-4-[(3-hydroxy-4-méthyl-phénylcarbamoyl)-méthyl]-1,4-diméthyl-pipérazin-1,4-di-ium;
- le dibromure de 1,4-bis-{3-[2-(3-hydroxy-4-méthyl-phénylamino)-éthyl]-3H-imidazol-1-ium}-butane;
- le dichlorure de 1,4-bis-{3-[(2,4dihydroxy-phénylcarbamoyl)-méthyl]-3H-imidazol-1-ium}-butane ;
- le chlorure de 3-[3-(2,4-diamino-phénoxy)-propyl]-1-[(2,4dihydroxy-phénylcarbamoyl)-méthyl]-3H-imidazol-1-ium ;
- le bromure, chlorure de 4-[2-(2,4-dihydroxy-phényl)-2-oxo-éthyl]-1-[2-(3-hydroxy-4-méthyl-phénylamino)-éthyl]-1,4-diméthyl-pipérazin-1,4-di-ium ;
- le dibromure de 1,3-bis-{[2-(2,4-diamino-phénoxy)-éthyl]-diéthyl-ammonium}-propane ;

et leurs sels d'addition avec un acide.

A titre de coupleurs hétérocycliques substitués par un ou plusieurs radicaux Z, on peut citer les coupleurs hydroxyindoles tels que décrits dans la demande de brevet EP 989 128.

On peut notamment citer les composés

- le méthosulfate de 3-(4-hydroxy-1-méthyl-1H-indol-5-ylméthyl)-1-méthyl-pyridinium ;
- le méthosulfate de 4-(4-hydroxy-1-méthyl-1H-indol-5-ylméthyl)-1-méthyl-pyridinium ;
- le diméthosulfate de 3-[3-(4-hydroxy-5-(1-méthylpyridinium)-4-ylméthyl-indol-1-yl)-propyl]-1-méthyl-imidazol-1-ium ;
- le méthosulfate de 4-(4-hydroxy-1-(2-hydroxyéthyl)-1H-indol-5-ylméthyl)-1-méthyl-pyridinium ;
- le diméthosulfate de 3-[3-(4-hydroxy-5-(1-méthylpyridinium)-5-ylméthyl-indol-1-yl)-propyl]-1-méthyl-imidazol-1-ium ;
- le diméthosulfate de 3-[4-hydroxy-5-(1-méthylpyridinium)-3-ylméthyl-indol-1-ylméthyl]-1-méthyl pyridinium ;
- le méthosulfate de 3-[3-(5-benzyl-4-hydroxy-indol-1-yl)-propyl]-1-méthyl-3H-imidazol-1-ium ;
- le méthosulfate de [2-(4-hydroxy-1 H-indol-3-yl)-éthyl]-triméthyl-ammonium ;
- le méthosulfate de [2-(4-hydroxy-1-méthyl-1H-indol-3-yl)-éthyl]-triméthylammonium ;
- le méthosulfate de (4-hydroxy-1-méthyl-1H-indol-3-ylméthyl)-triméthylammonium ;

- le méthosulfate de (4-hydroxy-1H-indol-3-ylméthyl)-triméthyl-ammonium;
- le méthosulfate de {3-[(4-hydroxy-1 H-indol-2-carbonyl)-amino]-propyl}-triméthylammonium ;
- le méthosulfate de {3-[(4-hydroxy-1-méthyl-1 H-indol-2-carbonyl)-amino]-propyl}-triméthyl-ammonium ;
- le méthosulfate de {3-[(4-hydroxy-5-méthyl-1H-indol-2-carbonyl)-amino]-propyl}-triméthyl-ammonium ;
- le méthosulfate de {3-[(4-hydroxy-1,5-diméthyl-1H-indol-2-carbonyl)-amino]-propyl}-triméthyl-ammonium
- le méthosulfate de 3-{3-[(4-hydroxy-1H-indol-2-carbonyl)-amino]-propyl}-1-méthyl-3H-imidazol-1-ium ;
- le méthosulfate de 3-{3-[(4-hydroxy-1-méthyl-1H-indol-2-carbonyl)-amino]-propyl}-1-méthyl-3H-imidazol-1-ium ;
- le méthosulfate de 3-{3-[(4-hydroxy-5-méthyl-1H-indol-2-carbonyl)-amino]-propyl}-1-méthyl-3H-imidazol-1-ium ;
- le méthosulfate de 3-{3-[(4-hydroxy-1,5-diméthyl-1H-indol-2-carbonyl)-amino]-propyl}-1-méthyl-3H-imidazol-1-ium ;
- le monochlorure de {3-[(4-hydroxy-1H-indo)-6-carbonyl)-amino]-propyl}-triméthyl-ammonium ;
- le monochlorure de {3-[(4-hydroxy-1-méthyl-1H-indol-6-carbonyl)-amino]-propyl}-triméthyl-ammonium ;

et leurs sels d'addition avec un acide.

Selon un mode de réalisation particulier, les colorants d'oxydation substitués par un radical Z utiles pour l'invention sont des colorants d'oxydation doubles et substitués par deux radicaux Z. Il est à noter que la nature du ou des contre-ions et des éventuels résidus de solvatation n'est pas critique dans l'invention.

La quantité de colorants d'oxydation cationiques est en général comprise entre 0,0001 % et 20%, de préférence entre 0.01 et 15% et encore plus préférentiellement entre 0,05 et 10% par rapport au poids total de la composition.

Selon un mode de réalisation préféré, le colorant d'oxydation est une base d'oxydation, de préférence para-phénylènediamine.

La composition de coloration utile dans la présente invention peut comprendre une ou plusieurs autres bases d'oxydation non cationiques bien connues de la technique telle que décrites dans les demandes de brevet citées précédemment.

La composition de coloration d'oxydation utile dans la présente invention peut contenir par ailleurs un ou plusieurs coupleurs non cationiques. Ceci est une variante préférée lorsque le colorant d'oxydation substitué par au moins un radical comportant un atome d'azote quaternisé est une base d'oxydation. Ces coupleurs sont bien connus de la technique et sont en particulier décrit dans les demandes de brevet citées précédemment. Parmi ces coupleurs, on peut notamment citer les méta-phénylènediamines, les méta-aminophénols, les méta-diphénols, les coupleurs naphtaléniques, les coupleurs hétérocycliques et leur sels d'addition.

A titre d'exemple, on peut citer le 2-méthyl 5-aminophénol, le 5-N-(β-hydroxyéthyl)amino 2-méthyl phénol, le 6-chloro-2-méthyl-5-aminophénol, le 3-amino phénol, le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxybenzène, le 1,3-diamino benzène, le 1,3-bis-(2,4-diaminophénoxy) propane, la 3-uréido aniline, le 3-uréido 1-diméthylamino benzène, le sésamol, le 1-β-hydroxyéthylamino-3,4-méthylènedioxybenzène, l'α-naphtol, le 2 méthyl-1-naphtol, le 6-hydroxy indole, le 4-hydroxy indole, le 4-hydroxy N-méthyl indole, la 2-amino-3-hydroxy pyridine, la 6-hydroxy benzomorpholine la 3,5-diamino-2,6-diméthoxypyridine, le 1-N-(β-hydroxyéthyl)amino-3,4-méthylène dioxybenzène, le 2,6-bis-(β-hydroxyéthylamino)toluène et leurs sels d'addition avec un acide.

Dans la composition de coloration d'oxydation, le ou les coupleurs sont chacun généralement présents en quantité comprise entre 0,001 et 10 % en poids environ du poids total de la composition tinctoriale, de préférence entre 0,005 et 6 %.

D'une manière générale, les sels d'addition avec un acide utilisables dans le cadre des compositions de coloration d'oxydation (bases d'oxydation et coupleurs) sont notamment choisis parmi les chlorhydrates, les bromhydrates, les sulfates et les tartrates, les lactates et les acétates.

La composition tinctoriale conforme à l'invention peut en outre contenir un ou plusieurs colorants directs pouvant notamment être choisis parmi les colorants nitrés de la série benzénique, les colorants directs azoïques, les colorants directs méthiniques. Ces colorants directs peuvent être de nature non ionique, anionique ou cationique.

Le milieu approprié pour la teinture appelé aussi support de teinture est un milieu cosmétique généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en $C_1$-$C_4$, tels que l'éthanol et l'isopropanol ; les polyols et éthers de polyols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, et leurs mélanges.

Les solvants sont, de préférence, présents dans des proportions de préférence comprises entre 1 et 40 % en poids environ par rapport au poids total de la composition tinctoriale, et encore plus préférentiellement entre 5 et 30 % en poids environ.

La composition tinctoriale conforme à l'invention peut également renfermer divers adjuvants utilisés classiquement dans les compositions pour la teinture des cheveux, tels que des agents tensio-actifs anioniques, cationiques,

non-ioniques, amphotères, zwittérioniques ou leurs mélanges, des polymères anioniques, cationiques, non-ioniques, amphotères, zwittérioniques ou leurs mélanges, des agents épaississants minéraux ou organiques, et en particulier les épaississants associatifs polymères anioniques, cationiques, non ioniques et amphotères, des agents antioxydants, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiants.

Les adjuvants ci dessus sont en général présents en quantité comprise pour chacun d'eux entre 0,01 et 20 % en poids par rapport au poids de la composition tinctoriale.

Bien entendu, l'homme de l'art veillera à choisir ce ou ces éventuels composés complémentaires de manière telle que les propriétés avantageuses attachées intrinsèquement à la composition de teinture d'oxydation conforme à l'invention ne soient pas, ou substantiellement pas, altérées par la ou les adjonctions envisagées.

Le pH de la composition tinctoriale conforme à l'invention est généralement compris entre 3 et 12 environ, et de préférence entre 5 et 11 environ. Il peut être ajusté à la valeur désirée au moyen d'agents acidifiants ou alcalinisants habituellement utilisés en teinture des fibres kératiniques ou bien encore à l'aide de systèmes tampons classiques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (II) suivante :

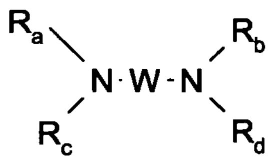
(II)

dans laquelle W est un reste propylène éventuellement substitué par un groupement hydroxyle ou un radical alkyle en $C_1$-$C_4$ ; $R_a$, $R_b$, $R_c$ et $R_d$, identiques ou différents, représentent un atome d'hydrogène, un radical alkyle en $C_1$-$C_4$ ou hydroxyalkyle en $C_1$-$C_4$.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

Le procédé de la présente invention est un procédé dans lequel on applique sur les fibres la composition selon la présente invention telle que définie précédemment, et on révèle la couleur à l'aide d'un agent oxydant. La couleur peut être révélée à pH acide, neutre ou alcalin et l'agent oxydant peut être ajouté à la composition de l'invention juste au moment de l'emploi ou il peut être mis en oeuvre à partir d'une composition oxydante le contenant, appliquée simultanément ou séquentiellement à la composition de l'invention. De préférence, cette coloration est révélée à pH neutre.

Selon un mode de réalisation particulier, la composition selon la présente invention est mélangée, de préférence au moment de l'emploi, à une composition contenant, dans un milieu approprié pour la teinture, au moins un agent oxydant, cet agent oxydant étant présent en une quantité suffisante pour développer une coloration. Le mélange obtenu est ensuite appliqué sur les fibres kératiniques. Après un temps de pose de 3 à 50 minutes environ, de préférence 5 à 30 minutes environ, les fibres kératiniques sont rincées, lavées au shampooing, rincées à nouveau puis séchées.

Les agents oxydants classiquement utilisés pour la teinture d'oxydation des fibres kératiniques sont par exemple le peroxyde d'hydrogène, le peroxyde d'urée, les bromates de métaux alcalins, les persels tels que les perborates et persulfates, les peracides et les enzymes oxydases parmi lesquelles on peut citer les peroxydases, les oxydo-réductases à 2 électrons telles que les uricases et les oxygénases à 4 électrons comme les laccases. Le peroxyde d'hydrogène est particulièrement préféré.

La composition oxydante peut également renfermer divers adjuvants utilisés classiquement dans les compositions pour la teinture des cheveux et tels que définis précédemment.

Le pH de la composition oxydante renfermant l'agent oxydant est tel qu'après mélange avec la composition tinctoriale, le pH de la composition résultante appliquée sur les fibres kératiniques varie de préférence entre 3 et 12 environ, et encore plus préférentiellement entre 5 et 11. Il peut être ajusté à la valeur désirée au moyen d'agents acidifiants ou alcalinisants habituellement utilisés en teinture des fibres kératiniques et tels que définis précédemment.

La composition prête à l'emploi qui est finalement appliquée sur les fibres kératiniques peut se présenter sous des formes diverses, telles que sous forme de liquides, de crèmes, de gels ou sous toute autre forme appropriée pour

réaliser une teinture des fibres kératiniques, et notamment des cheveux humains.

L'invention a aussi pour objet un dispositif à plusieurs compartiments ou "kit" de teinture dans lequel un premier compartiment renferme la composition tinctoriale de la présente invention définie ci-dessus à l'exception de l'agent oxydant et un deuxième compartiment renferme une composition oxydante. Ce dispositif peut être équipé d'un moyen permettant de délivrer sur les cheveux le mélange souhaité, tel que les dispositifs décrits dans le brevet FR-2 586 913 au nom de la demanderesse.

La présente invention a également pour objet l'utilisation pour la coloration d'oxydation des fibres kératiniques, et en particulier des fibres kératiniques humaines telles que les cheveux, d'une composition telle que définie précédemment.

Les dérivés de diamino-N,N-dihydropyrazolone de formule (I) peuvent être obtenus à partir d'intermédiaires et de voies de synthèse décrites dans la littérature et notamment dans les références suivantes : J. Het. Chem., 2001, 38(3), 613-616 , Helvetica Chimica Acta, 1950, 33, 1183-1194, J.Org.Chem., 23, 2029 (1958), J.Am.Chem.Soc., 73, 3240 (1951), J.Am.Chem.Soc., 84, 590 (1962), Justus Liebig Ann.Chem., 686, 134 (1965), Tetrahedron. Lett., 31, 2859-2862 (1973), les brevets US4128425 et US 2841584 et les références citées.

Suivant ces références, les composés de formule (I) ayant les radicaux $R_3$ et $R_4$ égaux à des atomes d'hydrogène peuvent être obtenus à partir de la voie de synthèse représentée sur le schéma A ci-dessous :

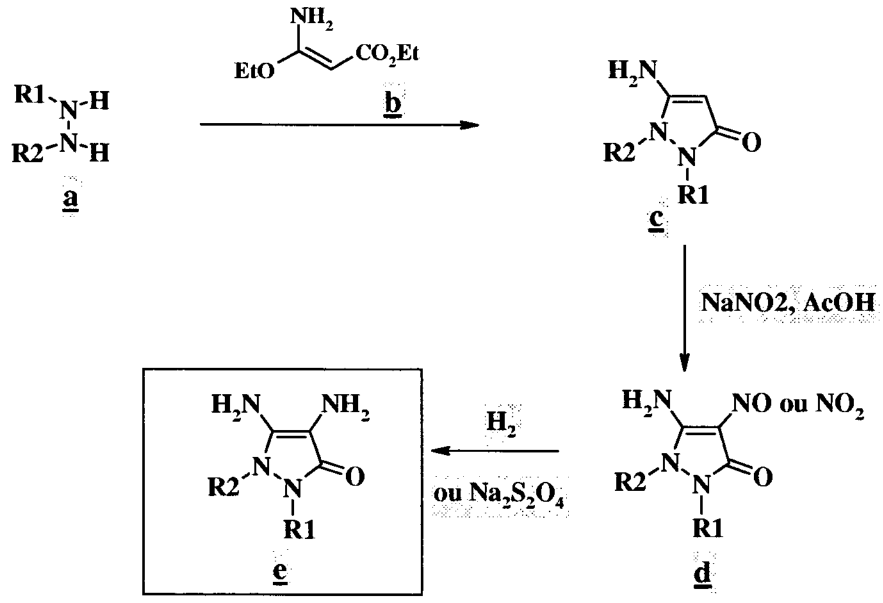


schéma A

Les composés dont les radicaux $R_1$ et $R_2$ représentent simultanément un groupe méthyle et les radicaux $R_3$ et $R_4$ des atomes d'hydrogène peuvent être obtenus en s'inspirant de la méthode décrite dans Justus Lieb.Ann.Chem., 686, 134 (1965) (schéma B) :

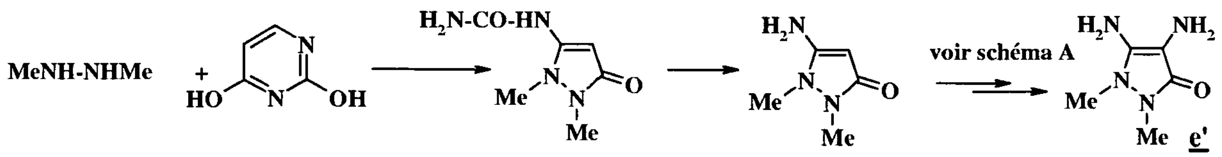


schéma B

Les composés dont le radical $R_1$ représente un groupe méthyle, $R_2$ un radical phényle, et les radicaux $R_3$ et $R_4$ des atomes d'hydrogène peuvent être obtenus en s'inspirant de la méthode décrite dans, J.Org.Chem., 23, 2029 (1958), J.Am.Chem.Soc., 73, 3240 (1951) (schéma C) :

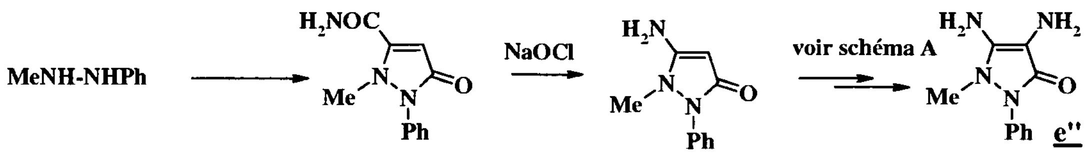


schéma C

Les composés dont les radicaux $R_1$ et $R_2$ forment ensemble un cycle à 5 chaînons et dont les radicaux $R_3$ et $R_4$ représentent des atomes d'hydrogène peuvent être obtenus en s'inspirant de la méthode décrite dans J. Het. Chem., 2001, 38(3), 613-616 (schéma D) :

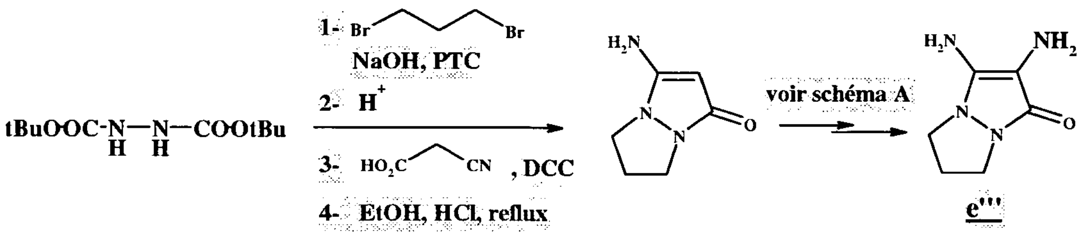


schéma D

Selon un procédé différent, les composés de formule (I) peuvent être obtenus selon la synthèse illustrée dans le schéma E :

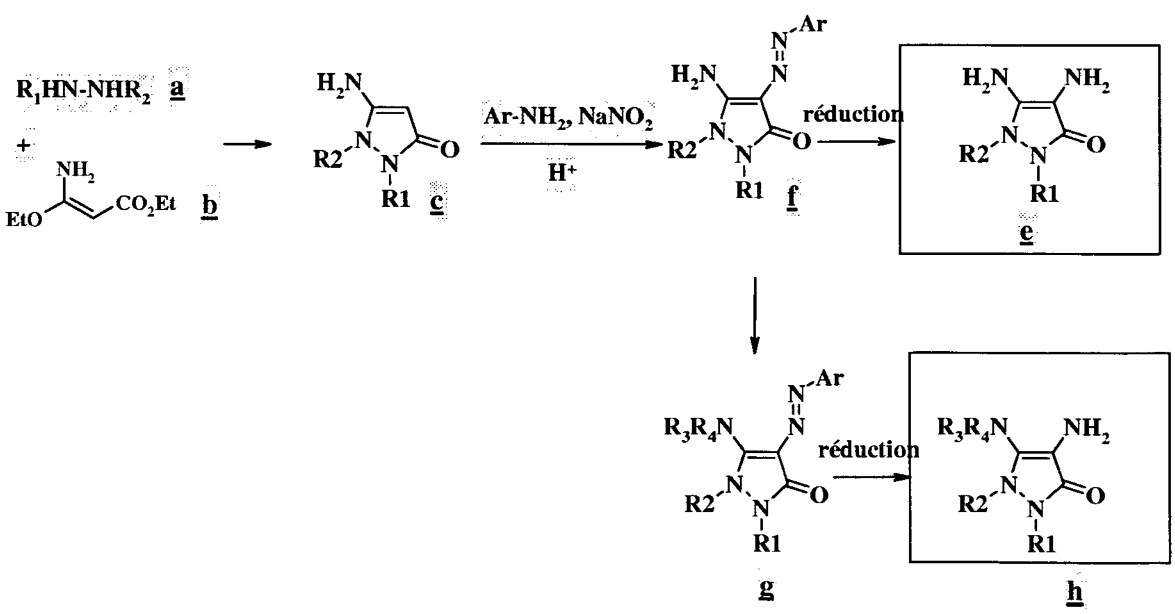


schéma E

Selon ce procédé, on met en oeuvre les étapes suivantes :

a) étape 1 :on fait réagir un composé a

**$R_1HN-NHR_2$** a

avec un composé b :

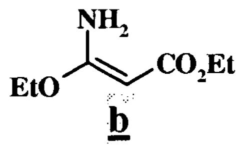

b

pour obtenir un composé 5-amino-1,2-dihydro-pyrazol-3-one c :

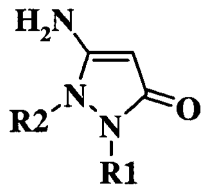

c

b) étape 2 : on fait réagir le dérivé c ainsi obtenu un sel d'aryldiazonium (Ar-$NH_2$, $NaNO_2$, $H^+$) pour obtenir un composé azoïque f :

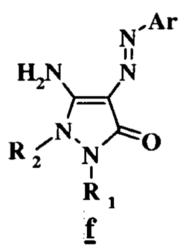

f

c) étape 3 : on effectue éventuellement une étape de fonctionalisation du groupement amine primaire du composé azoïque résultant f pour obtenir un composé g suivant :

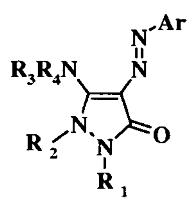

g

d) étape 4 : on effectue une réaction de réduction du composé azoïque f ou g pour obtenir, respectivement, un composé e ou h aminé :

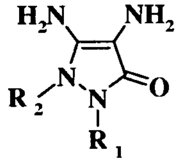

e

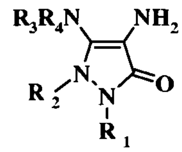

h

L'étape éventuelle de fonctionalisation du groupement amine primaire en position 5 en amine secondaire et tertiaire $NR_3R_4$, pour obtenir les composés g, est réalisée selon les méthodes classiques de synthèse organique (halogénure d'alkyle, O-sulfonate d'alkyle, trialkylammonium d'alkyle, amination réductrice, etc...voir par exemple Advanced Organic Chemistry, 3ème édition, 1985 , J. March , Willey Interscience*)*.

La réduction du groupe azoïque conduit aux composés e et h conformes à l'invention.

L'étape de réduction est réalisée de manière classique, par exemple en effectuant une réaction d'hydrogénation par catalyse hétérogène en présence de Pd/C, Pd(II)/C, Ni/Ra, etc... ou encore en effectuant une réaction de réduction par un métal, par exemple par du zinc, fer, étain, etc. (voir Advanced Organic Chemistry, 3ème édition, J. March, 1985 , Willey Interscience et Réduction in organic Chemistry, M . Hudlicky, 1983 , Ellis Horwood Séries Chemical Science).

Selon un autre procédé, les dérivés 2,3-diamino-6,7-dihydro-1H,5H-pyrazolo[1,2-a]-pyrazol-1-one et 2,3-diamino-5,6,7,8-tétrahydro-1H,6H-pyridazino[1,2-a]-pyrazol-1-one sont obtenus selon la synthèse illustrée par le schéma F :

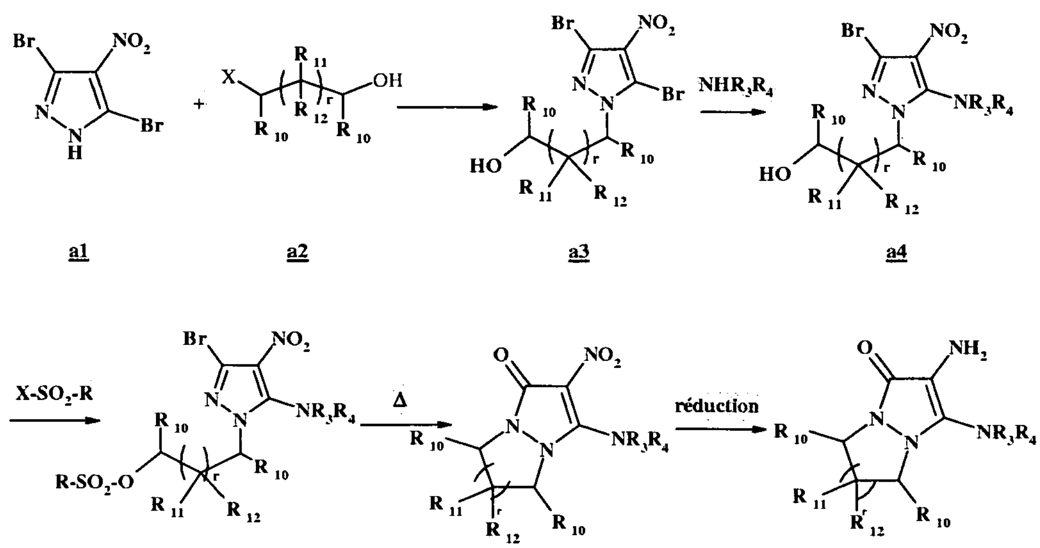


a1 a2 a3 a4

a5 a6 a7

**schéma F**

Selon ce procédé, on met en oeuvre les étapes suivantes :

a) étape 1 : on fait réagir un composé a1 suivant :

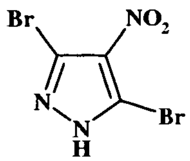

a1

avec un composé a2 :

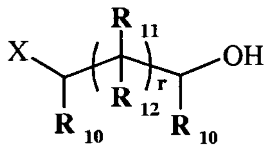

a2

pour obtenir un composé a3 :

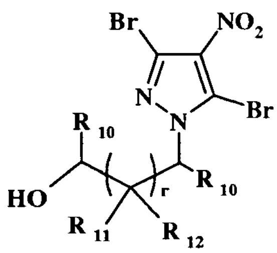

a3

dans lesquelles :

le radical $R_{10}$ représente un atome d'hydrogène, un carboxy ; un carboxamido ; un radical alkyle en $C_1$-$C_4$ éventuellement substitués par un ou plusieurs radicaux hydroxy, amino, (di)-alkylamino, alcoxy, carboxy, sulfonyle ;

les radicaux $R_{11}$ et $R_{12}$ représentent indépendamment les uns des autres des atomes d'hydrogène, d'halogène ; des radicaux amino ; (di)alkyl($C_1$-$C_4$)amino ; hydroxy ; carboxy ; carboxamido ; ($C_1$-$C_2$)alcoxy ; radical alkyle en $C_1$-$C_4$ éventuellement substitués par un ou plusieurs radicaux hydroxy, amino, (di)-alkylamino, alcoxy, carboxy, sulfonyle ;

X représente un atome d'halogène ou un alkyle sulfonate.
r est un entier compris entre 1 et 3 ;
b) étape 2 : on fait réagir le composé a3 avec une amine de formule $NHR_3R_4$ pour obtenir un composé a4 :

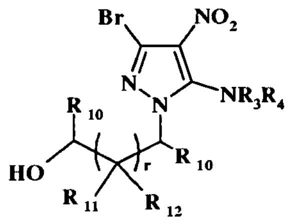

a4

c) étape 3 : on fait réagir le composé a4 avec au moins un halogénure d'alkylsulfonyle, d'arylsulfonyle ou de perfluoroalkylsulfonyle $R-O_2S-X_1$ (R représente un alkyle, un aryle ou un perfluoroalkyle, $X_1$ représente un halogène), dans un solvant de point d'ébullition compris entre 60°C et 190°C pour obtenir un composé a5 :

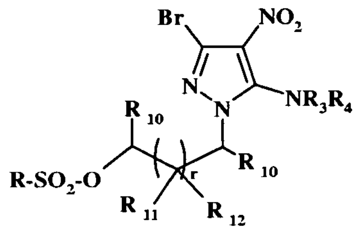

a5

d) étape 4 : le composé a5 résultant est ensuite chauffé dans un solvant de point d'ébullition compris entre 60°C et 190°C pour obtenir un composé a6 :

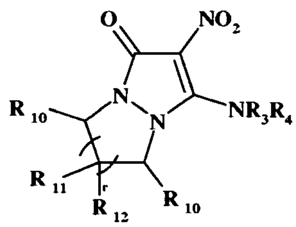

a6

e) étape 5 : Le composé a6 obtenu est réduit pour obtenir le composé a7 de formule ci-dessous (III):

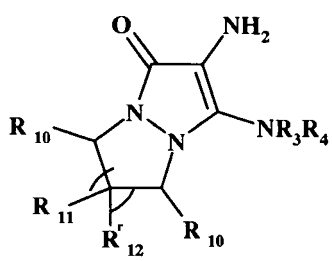

a7

formule (III)

Plus particulièrement, suivant ce procédé, le 3,5-dibromo-4-nitropyrazole a1, obtenu par exemple selon la méthode décrite dans le DE 4234885, réagit avec le réactif a2, de préférence dans un solvant de point d'ébullition compris entre 60°C et 190°C. A titre d'exemple, on peut citer le pentanol, le diméthylformamide, la N-méthylpyrrolidine. Plus particulièrement, la réaction est effectuée en présence d'une base organique ou minérale, telle que par exemple le carbonate de sodium, l'hydroxyde de sodium, l'acétate de sodium, ou la triéthylamine. La température du milieu réactionnel est avantageusement maintenue entre 60°C et 160°C, de préférence entre 80°C et 120°C.

Le 1-hydroxyalkyl-3,5-dibromo-4-nitropyrazole a3 est de préférence isolé par précipitation ou cristallisation après ajout de glace au milieu réactionnel.

Dans l'étape 2, le dérivé a3 est mis en réaction avec une amine $NHR_3R_4$, de préférence dans un solvant de point d'ébullition compris entre 60°C et 190°C, tel que par exemple le butanol, le pentanol, le diméthylformamide. La température est plus particulièrement comprise entre 60°C et 160°C, de préférence entre 80°C et 120°C. Après consommation des réactifs, le composé 5-amino-4-nitro-3-bromo-1-hydroxyalkylpyrazole a4 est isolé par précipitation ou cristallisation à l'aide d'eau.

Conformément à l'étape 3, le dérivé a5 est obtenu par réaction de l'alcool a4 et d'un halogénure d'alkylsulfonyle, d'arylsulfonyle ou de perfluoroalkylsulfonyle. La réaction a lieu de préférence dans un solvant aprotique tel que par exemple le tétrahydrofurane, le dioxane. La réaction a lieu avantageusement à une température comprise entre -20°C et 60°C, de préférence entre 0°C et 25°C. De plus, cette étape a lieu en présence d'une base organique ou minérale telle que par exemple le carbonate de potassium, la triéthylamine, la N-méthylmorpholine. Après disparition des réactifs, le composé **a5** est isolé par précipitation ou cristallisation dans l'eau.

Le sulfonate **a5** obtenu à l'issue de l'étape 3 est mis, dans l'étape 4, en solution ou en dispersion dans un solvant de point d'ébullition compris entre 60°C et 190°C, de préférence entre 90°C et 140°C. La température du milieu réactionnel est ensuite amenée entre 90°C et 140°C, de préférence entre 105°C et 125°C jusqu'à consommation totale du sulfonate a5. Après retour à la température ambiante, le composé perhydro-pyrazolo[1,2-a]pyrazol-1-one (r=1), perhydro-pyridazino[1,2-a]pyrazol-1-one (r=2) ou perhydro-diazépino[1,2-a]pyrazolone (r=3) a6 cristallise et est isolé par les méthodes classiques de synthèse organique.

Le composé final a7 conforme à l'invention est obtenu, lors d'une étape 5 par réduction du dérivé nitré a6, les méthodes de réduction utilisées étant par exemple une hydrogénation par catalyse hétérogène en présence de Pd/C, Pd(II)/C, Ni/Ra, etc... ou encore telles une réaction de réduction par un métal, par exemple par du zinc, fer, étain, etc, (voir Advanced Organic Chemistry, 3ème édition, J. March, 1985, Willey Interscience et Réduction in organic Chemistry, M. Hudlicky, 1983 ,Ellis Horwood Series Chemical Science).

Les exemples qui suivent servent à illustrer l'invention sans toutefois présenter un caractère limitatif.

## EXEMPLES

**Exemple 1 : synthèse du dichlorhydrate de 2,3-diamino-6,7-dihvdro-1H,5H-avrazolo[1,2-alayrazo]-1-one 5**

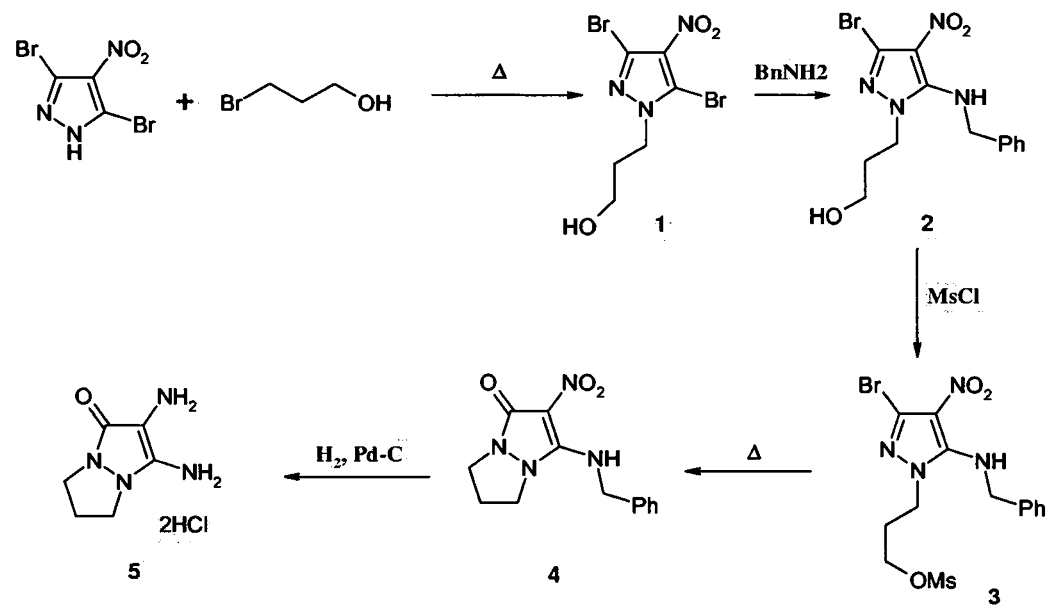


-Etape 1 : synthèse du 3-(3,5-dibromo- 4-nitro- 1H- pyrazol- 1-yl) propan- 1-ol 1

Dans un tricol de 500 ml, on introduit 0.369 mole d'acétate de sodium à une solution de 0.184 mole de dibromonitropyrazole dans 250 ml de N-méthyl pyrrolidone et le milieu réactionnel est porté à 80°C.

A cette température, on ajoute au goutte à goutte 0.369 mole de 3-bromo propanol. Cette température est maintenue pendant 5 heures.
Après refroidissement à température ambiante, le milieu est versé sur de la glace sous agitation.
Le 3-(3,5-dibromo-4-nitro-1H-pyrazol-1-yl)propan-1-ol 1 précipite. Il est essoré, séché et obtenu avec un rendement de 75%.

La masse du composé attendu $C_6H_7Br_2N_3O_3$ est détectée en spectrométrie de masse.

Les analyses RMN ($^1H$ 400 MHz et $^{13}C$ 100,61 MHz DMSO $d_6$) sont conformes à la structure attendue.

-Etape 2 : synthèse du 3-[5-(benzylamino)- 3-bromo- 4-nitro- 1H- pyrazol- 1-yl] propan- 1-ol 2

Dans un tricol de 500 ml contenant 150 ml d'éthanol, on disperse 0.135 mole de 3-(3,5-dibromo-4-nitro-1H-pyrazol-1-yl)propan-1-ol 1, chauffe à 60°C, puis additionne 0.825 mole de benzylamine en 30 minutes.

Après 6 heures à 60°C, le milieu réactionnel est refroidi à température ambiante.
Le 3-[5-(benzylamino)-3-bromo-4-nitro-1H-pyrazol-1-yl]propan-1-ol 2 est précipité en versant le milieu réactionnel sur 1 litre de glace sous agitation. Après essorage et séchage sous vide en présence de $P_2O_5$, le composé 2 est isolé avec un rendement de 90%.
Les analyses RMN ($^1H$ 400 MHz et $^{13}C$ 100,61 MHz DMSO $d_6$) sont conformes à la structure attendue.

Analyse élémentaire :

| | | | | | |
|---|---|---|---|---|---|
| théorique : | C43.96 | H4.26 | N15.77 | O13.51 | Br22.50 |
| mesuré : | C44.09 | H4.22 | N15.44 | O14.37 | Br21.50 |

-Etape 3 : synthèse du 3-[5-(benzylamino)- 3-bromo- 4-nitro- 1H- pyrazol- 1-yl] propyl méthanesulfonate 3

Dans un tricol de 500 ml contenant 200 ml de THF, on introduit, sous agitation, 0.126 mole de 3-[5-(benzylamino)-3-bromo-4-nitro-1H-pyrazol-1-yl]propan-1-ol 2 et 15.82 ml de triéthylamine. Le mélange obtenu est ensuite refroidi à 5°C et 0.126 mole de chlorure de mésyle sont coulés en 45 minutes.
Le milieu réactionnel est maintenu à cette température pendant 2 heures, puis le 3-[5-(benzylamino)-3-bromo-4-nitro-1H-pyrazol-1-yl]propylmethanesulfonate 3 est précipité en versantle milieu réactionnel sur 800 ml de la glace.

Après filtration, le solide est lavé abondamment à l'eau et à l'éther diisopropylique. Le séchage est réalisé sous vide en présence de $P_2O_5$. Le rendement de cette étape est de 94%

La masse du composé attendu $C_{14}H_{17}BrN_4O_5S$ est détectée en spectrométrie de masse.

Les analyses RMN ($^1H$ 400 MHz et $^{13}C$ 100,61 MHz DMSO $d_6$) sont conformes à la structure attendue.

Analyse élémentaire :

| | | | | | | |
|---|---|---|---|---|---|---|
| Théorie : | C38.81 | H3.96 | N12.93 | O18.46 | S7.40 | Br18.44 |
| Mesuré : | C39.03 | H3.91 | N12.83 | O18.52 | S7.29 | Br18.26 |

-Etape 4: synthèse de la 3-(benzylamino)- 2-nitro- 6,7-dihydro- 1H, 5H- pyrazolo [1,2-a] pyrazol- 1-one 4

Dans un tricol de 500 ml contenant 300 ml de pentanol, on disperse sous agitation 0.1 mole de 3-[5-(benzylamino)-3-bromo-4-nitro-1H-pyrazol-1-yl]propyl méthanesulfonate 3 et porte le milieu réactionnel à 130°C pendant 2 heures.

Après refroidissement à température ambiante, le solide formé est essoré sur fritté, lavé à l'éther diisopropylique et séché sous vide en présence de $P_2O_5$, Le 3-(benzylamino)-2-nitro-6,7-dihydro-1H,5H-pyrazolo[1,2-a]pyrazol-1- one 4 est obtenu avec un rendement de 86%.
Les analyses RMN ($^1H$ 400 MHz et $^{13}C$ 100,61 MHz DMSO $d_6$) sont conformes à la structure attendue.
La masse du composé attendu $C_6H_{11}N_4O$ est détectée en spectrométrie de masse.

Analyse élémentaire :

| | | | | |
|---|---|---|---|---|
| Théorie : | C56.72 | H5.49 | N20.36 | O17.44 |
| Mesuré : | C56.68 | H5.13 | N20.38 | O17.69 |

-Etape 5 : synthèse du dichlorhydrate de 2,3-diamino- 6,7-dihydro- 1H, 5H- pyrazolo [1,2-a] pyrazol- 1-one 5

Dans un autoclave de 1 litre contenant 800 ml d 'éthanol, on introduit 20 g de 3-(benzylamino)-2-nitro-6,7-dihydro-1H,5H-pyrazolo[1,2-a]pyrazol-1-one 4 et 4g de palladium sur charbon à 5%. La réduction est ensuite réalisée sous une pression d'hydrogène de 8 Bars et à une température comprise entre 50°C et 100°C ( agitation comprise entre 1000 et 2500 tr/min)

Au bout de 4 heures de réaction, il n'a plus consommation d'hydrogène et le milieu est refroidi à 20°C.

Le catalyseur est éliminé sous azote par filtration, puis de l'éthanol chlorhydrique est ajouté au filtrat. Le produit

cristallisé est essoré, lavé à l'éther diisopropylique, puis séché sous vide en présence de $P_2O_5$. Le dichlorhydrate de 2,3-diamino-6,7-dihydro-1H,5H-pyrazolo[1,2-a]pyrazol-1-one 5 est obtenu avec un rendement de 89%.
La masse du composé attendu est détectée en spectrométrie de masse.
Les analyses RMN ($^1$H 400 MHz et $^{13}$C 100,61 MHz DMSO $d_6$) sont conformes à la structure attendue.

| Analyse élémentaire : | | | | | |
|---|---|---|---|---|---|
| Théorique : | C31.73 | H5.33 | N24.67 | O7.07 | Cl31.22 |
| Mesuré : | C31.45 | H5.20 | N24.62 | O7.24 | Cl30.86 |

**Exemple 2 : synthèse du dichlorhvdrate de 2-amino-3-(éthylamino)-6,7-dihydro-1H,5H-pyrazolo[1,2-a]pyrazol-1-one 9**

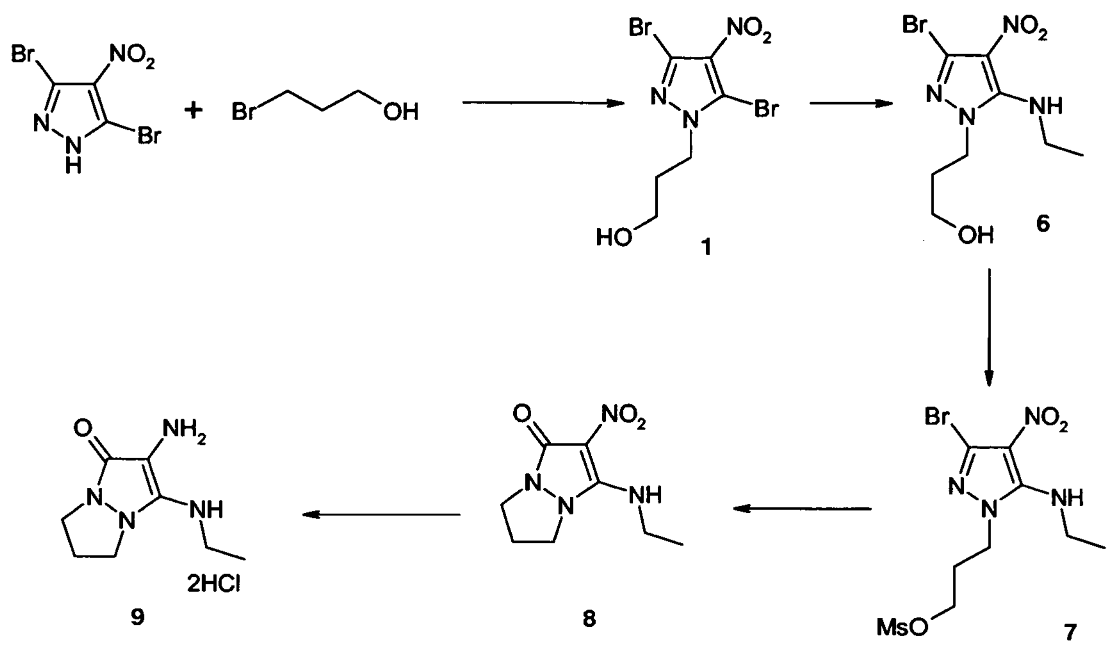

-Etape 2 : synthèse du 3-[3-bromo- 5-(éthylamino)- 4-nitro- 1H- pyrazol- 1-yl] propan- 1-ol 6

Dans un tricol, sous agitation, on introduit 15 mmoles de 3-(3,5-dibromo-4-nitro-1H-pyrazol-1-yl)propan-1-ol dans 30 ml d'éthanol. Le milieu homogène est chauffé à 75˚C puis 93 mmoles d'éthylamine sont coulées au goutte à goutte et l'agitation est maintenue quatre heures.
Après refroidissement à température ambiante, le milieu est versé sur de la glace et le 3-[3-bromo-5-(éthylamino)-4-nitro-1H-pyrazol-1-yl]propan-1-ol 6 précipite.
Le solide jaune est essoré, puis lavé abondamment à l'eau et à l'éther düsopropylique. Le séchage est réalisé sous vide en présence de $P_2O_5$. La masse récupérée est de 3.6 g.
Les analyses RMN ($^1$H 400 MHz et $^{13}$C 100,61 MHz DMSO $d_6$) sont conformes à la structure attendue.
La masse du composé attendu $C_8H_{13}BrN_4O_3$ est détectée en spectrométrie de masse.

-Etape 3 : synthèse du 3-[5-(éthylamino)- 3-bromo- 4-nitro- 1 H- pyrazol- 1-yl] propyl methanesulfonate 7

Dans un tricol de 100 ml contenant 30 ml de THF, sous agitation, on introduit 11.2 mmoles de 3-[3-bromo-5-(éthylamino)-4-nitro-1H-pyrazol-1-yl]propan-1-ol 6 et 1.6 ml de triéthylamine. Le mélange homogène orange obtenu est refroidi à 0˚C et 1.44 ml de chlorure de mésyle sont coulés en 20 minutes.
Le milieu réactionnel est maintenu à cette température pendant 2 heures puis le 3-[5-(éthylamino)-3-bromo-4-nitro-1H-pyrazol-1-yl]propylméthanesulfonate 7 est précipité en versant le milieu réactionnel sur 500 ml de glace.

Le solide jaune est essoré, puis lavé abondamment à l'eau et à l'éther diisopropylique ; le séchage est réalisé sous vide en présence de $P_2O_5$. La masse récupérée est de 3.1g.
Les analyses RMN ($^1$H 400 MHz et $^{13}$C 100,61 MHz DMSO $d_6$) sont conformes à la structure attendue.
La masse du composé attendu $C_9H_{15}BrN_4O_5S$ est détectée en spectrométrie de masse.

-Etape 4: synthèse de la 3-(éthylamino)- 2-nitro- 6,7-dihydro- 1H, 5H- pyrazolo [1,2-alpyrazol- 1-one 8

Dans un tricol de 50 ml contenant 20 ml de pentanol, on disperse sous agitation 8 mmoles de 3-[5-(éthylamino)-3-bromo-4-nitro-1H-pyrazol-1-yl]propyl méthanesulfonate 7, et porte le milieu réactionnel à 130°C pendant 2 heures. Après refroidissement à température ambiante, le solide formé est essoré, puis lavé à l'éther düsopropylique.
Après séchage sous vide en présence de $P_2O_5$, on obtient 1.46 g de 3-(éthylamino)-2-nitro-6,7-dihydro-1H, 5H-pyrazolo[1,2-a]pyrazol-1one 8.
Les analyses RMN ($^1$H 400 MHz et $^{13}$C 100,61 MHz DMSO $d_6$) sont conformes à la structure attendue.
La masse du composé attendu est détectée en spectrométrie de masse.

-Etape 5 : synthèse du dichlorhydrate de 2-amino- 3-(éthylamino)- 6,7-dihydro- 1 H, 5H- pyrazolo [1,2-a] pyrazol- 1-one 9

Dans un autoclave de 300 ml contenant 200 ml d'éthanol, on introduit 1.45 g de 3-(éthylamino)-2-nitro-6,7-dihydro-1H,5H-pyrazolo[1,2-a]pyrazol-1-one 8 et 300 mg de palladium sur charbon à 5%. La réduction est réalisée sous une pression d'hydrogène de 8 Bars à une température de 60°C (agitation de 1700 tr/min).
Au bout de 2 heures de réaction, il n'y a plus consommation d'hydrogène et le milieu est refroidi à 20°C.
Le catalyseur est éliminé par filtration sous azote et le filtrat est dilué avec 100 ml d'éther isopropylique chlorhydrique.
La solution jaune pale est évaporée à sec puis le solide est repris avec un mélange éthanol / éther isopropylique. Le dichlorhydrate de 2-amino-3-(éthylamino)-6,7-dihydro-1H,5H-pyrazolo[1,2-a]pyrazol-1-one 9 précipite ; il est essoré et après séchage sous vide en présence de $P_2O_5$, on récupère 1.18 g de dichlorhydrate de 2-amino-3-(éthylamino)-6,7-dihydro-1H,5H-pyrazolo[1,2-a]pyrazol-1-one 9.
Les analyses RMN ($^1$H 400 MHz et $^{13}$C 100,61 MHz DMSO $d_6$) sont conformes à la structure attendue.
La masse du composé attendu $C_8H_{14}N_4O$ est détectée en spectrométrie de masse.

**Exemple 3 : dichlorhydrate de 2-amino-3-(isopropylamino)-6,7-dihydro-1H,5H-pyrazolo[1,2-a]pyrazol-1-one 13**

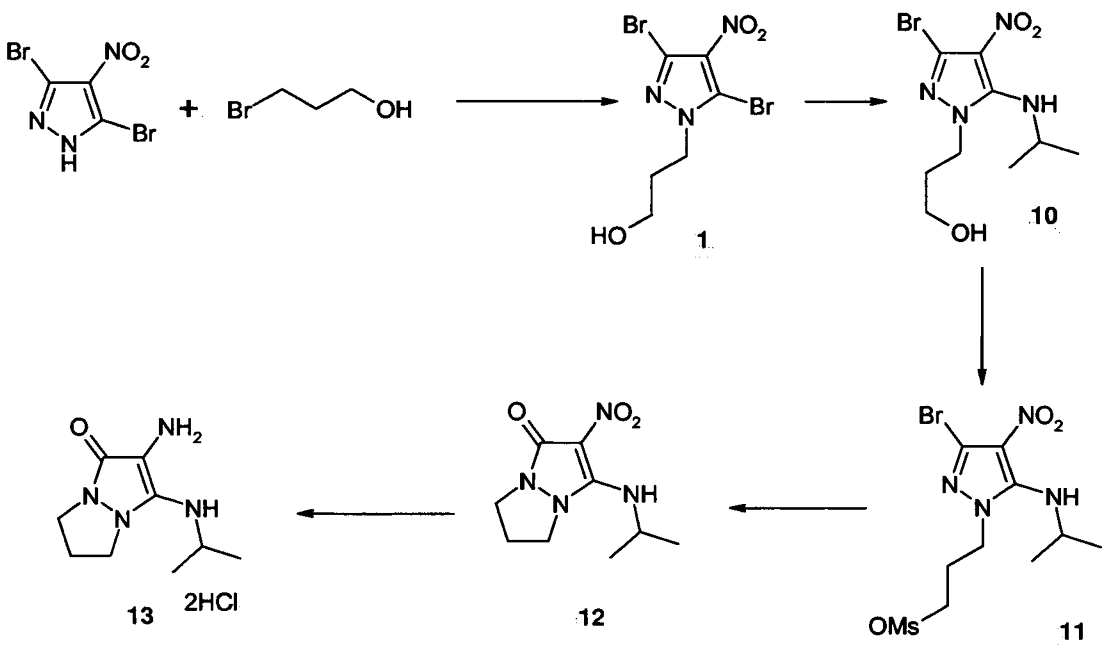
1 10 11 12 13 2HCl

-Etape 2 : 3-[3-bromo- 5-(isopropylamino)- 4-nitro- 1H- pyrazol- 1-yl] propan- 1-ol 10

Dans un tricol, sous agitation, on introduit 15 mmoles de 3-(3,5-dibromo-4-nitro-1H-pyrazol-1-yl)propan-1-ol dans 30 ml d'éthanol. Le milieu homogène est chauffé à 75˚C, puis 93 mmoles d'isopropylamine sont coulées au goutte à goutte tout en maintenant l'agitation quatre heures.

Après refroidissement à température ambiante, le milieu est versé sur de la glace, puis neutralisé à l'acide chlorhydrique. On extrait le 3-[3-bromo-5-(isopropylamino)-4-nitro-1H-pyrazol-1-yl]propan-1-ol 10 au dichlorométhane.

Après séchage de la phase organique sur sulfate de sodium et élimination du solvant par évaporation sous vide, on obtient 4.37 g de 3-[3-bromo-5-(isopropylamino)-4-nitro-1H-pyrazol-1-yl]propan-1-ol 10.

Les analyses RMN ($^1H$ 400 MHz et $^{13}C$ 100,61 MHz DMSO $d_6$) sont conformes à la structure attendue.

La masse du composé attendu $C_9H_{15}BrN_4O_3$ est détectée en spectrométrie de masse.

-Etape 3 : synthèse du 3-[5-(isopropylamino)- 3-bromo- 4-nitro- 1H- pyrazol- 1-yl] propyl méthanesulfonate 11

Dans un tricol de 50 ml contenant 20 ml de THF, on introduit, sous agitation, 13.7 mmoles de 3-[3-bromo-5-(isopropylamino)-4-nitro-1H-pyrazol-1-yl]propan-1-ol 10 et 1.94 ml de triéthylamine. Le mélange homogène orange ainsi obtenu est refroidi à 0˚C et 1.76 ml de chlorure de mésyle sont coulés en 20 minutes.

Le milieu réactionnel est maintenu à cette température pendant 2 heures, puis le 3-[5-(éthylamino)-3-bromo-4-nitro-1H-pyrazol-1-yl]propylméthanesulfonate 11 est précipité en versant le milieu réactionnel sur 500 ml de glace.

Le solide jaune est essoré, puis lavé abondamment à l'eau et à l'éther de pétrole, le séchage est réalisé sous vide en présence de $P_2O_5$. La masse récupérée est de 4.2 g.

Les analyses RMN ($^1H$ 400 MHz et $^{13}C$ 100,61 MHz DMSO $d_6$) sont conformes à la structure attendue.

La masse du composé attendu est détectée en spectrométrie de masse..

-Etape 4 : synthèse de la 3-(isopropylamino)- 2-nitro- 6,7-dihydro- 1H, 5H- pyrazolo [1,2-a] pyrazol- 1-one 12

Dans un tricol de 50 ml, on disperse, sous agitation, 10 mmoles de 3-[5-(isopropylamino)-3-bromo-4-nitro-1H-pyrazol-1-yl]propylméthanesulfonate 11 dans 20 ml de pentanol et chauffe à 130˚C pendant 2 heures.

Après refroidissement à température ambiante, le solide obtenu est essoré sur fritté, et lavé à l'éther diisopropylique.

Après séchage sous vide en présence de $P_2O_5$, 1,71 g de 3-(isopropylamino)-2-nitro-6,7-dihydro-1H,5H-pyrazolo[1,2-a]pyrazol-1one 12 sont obtenus

Les analyses RMN ($^1H$ 400 MHz et $^{13}C$ 100,61 MHz DMSO $d_6$) sont conformes à la structure attendue.

La masse du composé attendu $C_9H_{14}N_4O_3$ est détectée en spectrométrie de masse.

-Etape 5: synthèse du dichlorhydrate de 2-amino- 3-(isopropylamino)- 6,7-dihydro- 1H, 5H- pyrazolo [1,2-a] pyrazol-1-one 13

Dans un autoclave de 300 ml contenant 200 ml d'éthanol, on introduit 1.70 g de 3-(isopropylaminoamino)-2-nitro-6,7-dihydro-1 H,5H-pyrazolo[1,2-a]pyrazol-1-one 12 et 300 mg de palladium sur charbon à 5%. La réduction est réalisée sous une température de 60˚C et sous une pression d'hydrogène de 6 Bars (agitation de 2000 tr/min).

Au bout de 2 heures de réaction, il n'a plus consommation d'hydrogène et le milieu est refroidi à 20˚C. Le catalyseur est éliminé par filtration sous azote après refroidissement à température ambiante et de l'éther isopropylique chlorhydrique est additionné.

La solution jaune pale est évaporée à sec, puis le solide est repris avec 50 ml d'éther diisopropylique saturé en acide chlorhydrique, le précipité est récupéré par essorage. Après séchage sous vide en présence de $P_2O_5$, 1,5 g de dichlorhydrate de 2-amino-3-(isopropylamino)-6,7-dihydro-1H,5H-pyrazolo[1,2-a]pyrazol-1-one 13 sont isolés.

Les analyses RMN ($^1H$ 400 MHz et $^{13}C$ 100,61 MHz DMSO $d_6$) sont conformes à la structure attendue.

La masse du composé attendu $C_9H_{16}N_4O$ est détectée en spectrométrie de masse.

**Exemple 4 : dichlorhydrate de 2-amino-3-(pyrrolidin-1-yl)-6,7-dihydro-1H,5H-pyrazolo[1.2-a]pyrazol-1-one 17**

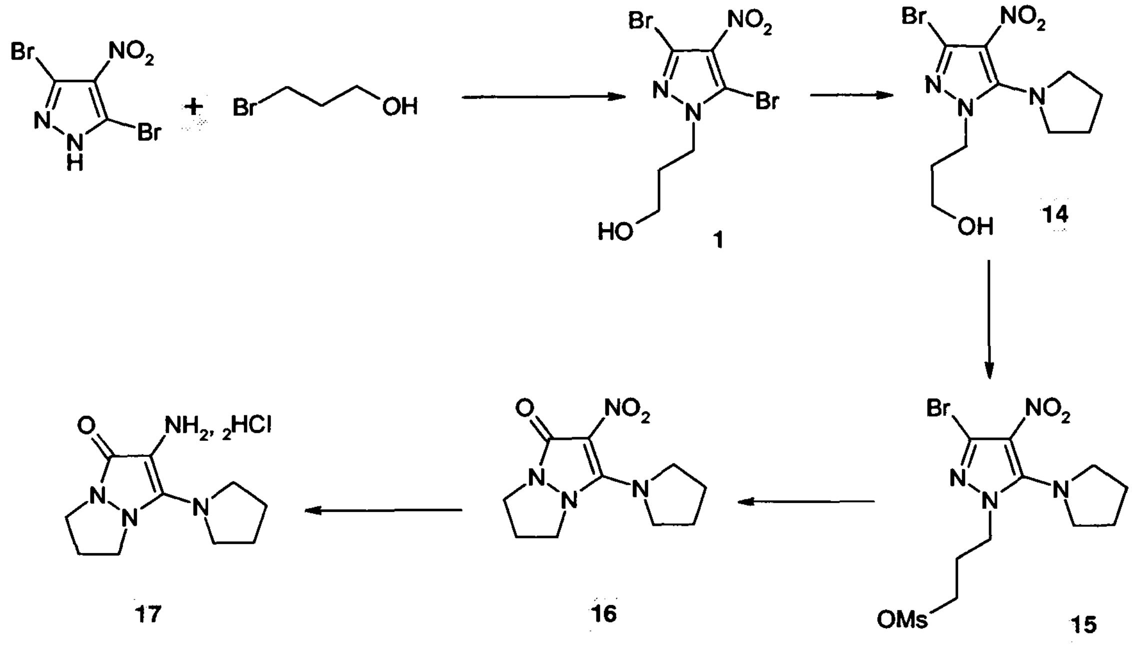

1

14

17

16

15

-Etape 2 : 3-(3-bromo- 4-nitro- 5-(pyrrolidin- 1-yl)- 1H- pyrazol- 1-yl) propan- 1-ol 14

Dans un tricol, sous agitation, on introduit 15 mmoles de 3-(3,5-dibromo-4-nitro-1H-pyrazol-1-yl)propan-1-ol dans 20 ml d'isopropanol. Le milieu homogène est chauffé à 75˚C puis 90 mmoles de pyrrolidine sont coulées au goutte à goutte et l'agitation est maintenue deux heures.

Après refroidissement à température ambiante, le milieu est versé sur de la glace et neutralisé à l'acide chlorhydrique. On extrait le 3-(3-bromo-4-nitro-5-(pyrrolidin-1-yl)-1H-pyrazol-1-yl)propan-1-ol 14 par du dichlorométhane.

Après séchage de la phase organique sur sulfate de sodium et distillation du solvant par évaporation sous vide, on obtient 4.8 g de 3-(3-bromo-4-nitro-5-(pyrrolidin-1-yl)-1H-pyrazol-1-yl)propan-1-ol 14.

Les analyses RMN ($^1$H 400 MHz et $^{13}$C 100,61 MHz DMSO $d_6$) sont conformes à la structure attendue.

La masse du composé attendu $C_{10}H_{17}BrN_4O$ est détectée en spectrométrie de masse.

-Etape 3 : synthèse du 3-(3-bromo- 4-nitro- 5-(pyrrolidin- 1-yl)- 1H- pyrazol- 1-yl) propylméthanesulfonate 15

Dans un tricol de 100 ml contenant 50 ml de THF, on introduit, sous agitation, 30 mmoles 3-(3-bromo-4-nitro-5-(pyrrolidin-1-yl)-1H-pyrazol-1-yl)propan-1-ol 14 et 4.25 ml de triéthylamine. Le mélange homogène orange obtenu est refroidi à 0˚C et 2.32 ml de chlorure de mésyle sont coulés en 20 minutes.

Le milieu réactionnel est maintenu à cette température pendant 2 heures puis le 3-(3-bromo-4-nitro-5-(pyrrolidin-1-yl)-1H-pyrazol-1-yl)propylméthanesulfonate 15 est précipité en versant le milieu réactionnel sur de la glace. Le solide est essoré, puis séché sous vide en présence de $P_2O_5$. La masse récupérée est de 9.3 g.

Les analyses RMN ($^1$H 400 MHz et $^{13}$C 100,61 MHz DMSO $d_6$) sont conformes à la structure attendue.

La masse du composé attendu $C_{11}H_{19}BrN_4O_3S$ est détectée en spectrométrie de masse.

-Etape 4 : synthèse 2-nitro- 3-(pyrrolidin- 1-yl)- 6,7-dihydro- 1H, 5H- pyrazolo [1,2-alpyrazol- 1-one 16

Dans un tricol de 250 ml, on introduit, sous agitation, 22.5 mmoles de 3-(3-bromo-4-nitro-5-(pyrrolidin-1-yl)-1H-pyrazol-1-yl)propylméthanesulfonate 15 dans 100 ml de pentanol. Le milieu ainsi obtenu est porté à 130˚C pendant 2 heures.

Après refroidissement à température ambiante, le 2-nitro-3-(pyrrolidin-1-yl)-6,7-dihydro-1H,5H-pyrazolo[1,2-a]pyrazol-1-one 16 est extrait au dichlorométhane.

Après séchage de la phase organique sur sulfate de sodium et distillation du solvant sous vide, on obtient 1.2 g de 2-nitro-3-pyrrolidin-1-yl-6,7-dihydro-1H,5H-pyrazolo[1,2-a]pyrazol-1-one 16

Les analyses RMN ($^1$H 400 MHz et $^{13}$C 100,61 MHz DMSO $d_6$) sont conformes à la structure attendue..

La masse du composé attendu $C_{10}H_{14}N_4O_3$ est détectée en spectrométrie de masse.

-Etape 5 : synthèse du dichlorhydrate de 2-amino- 3-(pyrrolidin- 1-yl)- 6,7-dihydro- 1H, 5H- pyrazolo [1,2-a] pyrazol-1-one 17

Dans un autoclave de 300 ml contenant 200 ml d 'éthanol, on introduit 1.1 g de 2-nitro-3-(pyrrolidin-1-yl)-6,7-dihydro-1H,5H-pyrazolo[1,2-a]pyrazol-1-one 16 et 300 mg de palladium sur charbon à 5%. La réduction est réalisée sous une agitation de 2000 tr/min, sous une température de 60°C et sous une pression d'hydrogène de 6 Bars.
Au bout de 2 heures de réaction, il n'a plus consommation d'hydrogène et le milieu est refroidit à 20°C.
Le catalyseur est éliminé par filtration sous azote après refroidissement à température ambiante et de l'éther isopropylique chlorhydrique est additionné.
La solution jaune pale est évaporée à sec, puis le solide est repris avec 50 ml d'éther diisopropylique saturé en acide chlorhydrique, le précipité est récupéré par essorage . Après séchage sous vide en présence de $P_2O_5$, on obtient 1.5 g de dichlorhydrate de 2-amino-3-(pyrrolidin-1-yl)-6,7-dihydro-1 H,5H-pyrazolo[1 ,2-a]pyrazol-1-one 5 17.
Les analyses RMN ($^1H$ 400 MHz et $^{13}C$ 100,61 MHz DMSO $d_6$) sont conformes à la structure attendue.
La masse du composé attendu $C_{10}H_{16}N_4O$ est détectée en spectrométrie de masse.

**Exemple 5 . Synthèse du diméthanesulfonate de 2,3-diamino-6,7-dihydro-1H,5H-pyrazolo[1,2-a]pyrazol-1-one**

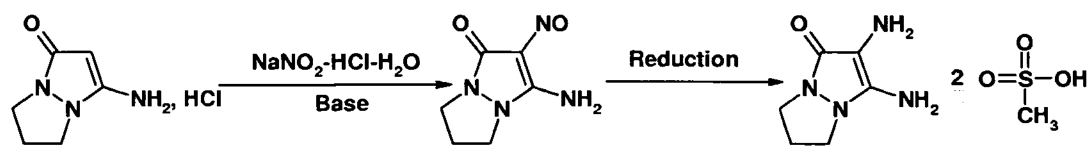


1 2 3

Synthèse du 3-amino-2-nitroso-6,7-dihydro-1H,5H-pyrazolo[1,2-a]pyrazol-1-one:2

Dans un tricol de 500 ml, on dissout, sous agitation, à température ambiante, 43g (0.245 mole) de chlorhydrate de 3-amino-6,7-dihydro-1H,5H-pyrazolo[1,2-a]pyrazol-1-one dans un mélange de 180 ml d'eau et 35 ml d'acide chlorhydrique à 35%.
On refroidit à 0°C et ajoute goutte à goutte, en 30 minutes, une solution de 17.3 g de nitrite de sodium (0.25 mole) dans 20 ml d'eau. La température du milieu réactionnel est maintenue entre 0 et +5°C pendant toute la durée de l'addition et pendant une heure après la fin de l'addition.
Le milieu réactionnel est amené à pH 8 par addition de soude, sous agitation, tout en maintenant la température entre 0 et 5°C. La 3-amino-2-nitroso-6,7-dihydro-1H,5H-pyrazolo[1,2-a]pyrazol-1-one 2 précipite sous la forme d'un solide orangé rouge qui est filtre sur verre fritté n°4, empâté dans le minimum de 2-propanol, lavé à l'éther diisopropylique et séché sous vide en présence de pentaoxyde de phosphore. On obtient ainsi 35 g de produit rouge orangé (rendement : 85%).
Les spectres de RMN (1 H 400 MHz et 13C 100,61 MHz DMSO d6) et de masse sont conformes à la structure attendue 2.

Synthèse du diméthanesulfonate de 2,3-diamino-6,7-dihydro-1H,5H-pyrazolo[1,2-a]pyrazol-1-one : 3

Dans un autoclave de 1 litre, on introduit 33.6 g (0.2mole) de 3-amino-2-nitroso-6,7-dihydro-1H,5H-pyrazolo [1,2-a]pyrazol-1-one 2, 500 ml d'éthanol et 6 g de palladium sur charbon à 5% contenant 50% d'eau.
Le milieu est purgé 3 fois à l'azote puis 3 fois à l'hydrogène et la température du mélange est portée à 40°C.
La réduction est réalisée en deux heures sous une pression de 8 bars. Cette réduction est exothermique et la température atteint d'elle-même 70°C.
On laisse la température redescendre à 50°C puis le catalyseur est filtré sur un filtre presse sous un courant d'azote.
Le filtrat est coulé dans un mélange de 50 ml d'éthanol et 40 ml d'acide méthane sulfonique, en refroidissant à 0°C. Le diméthanesulfonate de 2,3-diamino-6,7-dihydro-1H,5H-pyrazolo[1,2-a]pyrazol-1-one 3 cristallise sous la forme d'un solide jaune pâle qui est essoré sur verre fritté n°4, lavé à l'éther diisopropylique puis à l'éther de pétrole et enfin séché sous vide en présence de pentaoxyde de phosphore. On obtient ainsi 43g de solide jaune pâle (rendement : 65%).

Les spectres de RMN ($^1$H 400 MHz et $^{13}$C 100,61 MHz DMSO $d_6$) et de masse sont conformes à la structure attendue 3.

| Analyse élémentaire : | | | | | |
|---|---|---|---|---|---|
| Théorie : | C27.74 | H5.23 | N16.17 | O32.33 | S18.51 |
| Mesuré : | C27.16 | H5.22 | N15.63 | O32.81 | S18.64 |

**Exemple 6 : synthèse du chlorhydrate de 2,3-diamino-5,6,7,8-tetrahydro-1H-pyrazolo[1,2-a]pyridazin-1-one .**

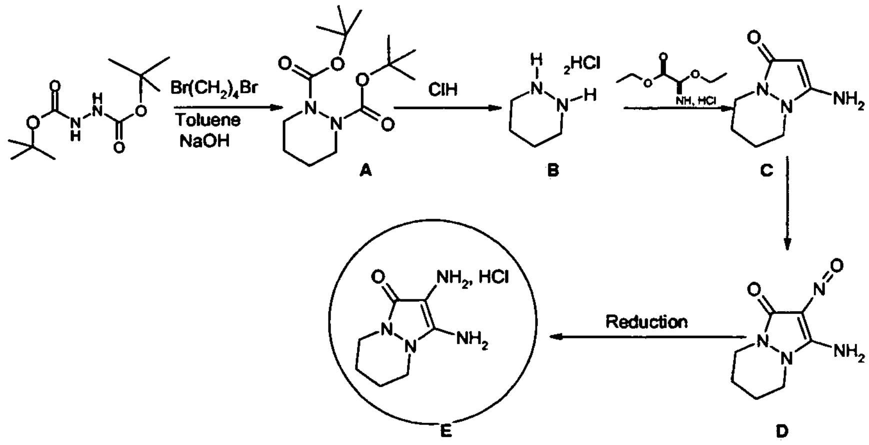


Synthèse du di-tert-butyl tetrahydropyridazine-1,2-dicarboxylate : **A**

Dans un tricol de 250 ml équipé d'un réfrigérant, d'un thermomètre et d'une ampoule de coulée, on introduit sous agitation mécanique 50 ml de toluène, 5 g (21.5 mmoles) de N,N'-di-tert-butoxycarbonylhydrazide, 680 mg de bromure de tétraéthylammonium et 25 ml de soude à 50%.
Le milieu hétérogène est chauffé à 100°C puis on ajoute goutte à goutte en 15 minutes le 1,4-dibromobutane.
Le milieu réactionnel est chauffé à 100°C pendant 3 jours. Après refroidissement, on ajoute 100 ml d'acétate d'éthyle et on transfère dans une ampoule à décanter. La phase organique est lavée avec 4 fois 70 ml de solution aqueuse saturée en carbonate de sodium, puis avec 4 x 70 ml d'eau et enfin avec 4 x 70ml d'eau salée. La phase organique est séchée sur sulfate de sodium et le solvant est évaporé sous vide. On obtient ainsi une huile incolore qui cristallise en un solide blanc.
On récupère une masse de 6.1 g (rendement : 99%).
Les spectres RMN ($^1$H 400 MHz et $^{13}$C 100,61 MHz DMSO $d_6$) et de masse sont conformes à la structure attendue **A.**

Synthèse du dichlorhydrate d'hexahydropyridazine : **B**

Dans un tricol de 100 ml équipé d'un réfrigérant et d'un thermomètre, on introduit sous agitation mécanique 5.9 g du composé **A** dans 50 ml de mélange 3/1 de dioxanne et d'acide chlorhydrique à 35%.
La solution incolore obtenue est agitée à température ambiante pendant 3 heures puis le milieu réactionnel est dilué par de l'éther diisopropylique. Les solvants sont évaporés sous vide. Le résidu pâteux obtenu est repris par un mélange éther/éthanol. Après filtration du solide et séchage sous vide, on obtient 1.39 g de solide blanc.

Les spectres de RMN ($^1$H 400 MHz et $^{13}$C 100,61 MHz DMSO $d_6$) et de masse sont conformes à la structure attendue **B**.

Synthèse du 3-amino-5,6,7,8-tetrahydro-1H-pyrazolo[1,2-a]pyridazin-1-one : **C**

Dans un tricol de 25 ml équipé d'un réfrigérant et d'un thermomètre, on introduit sous agitation mécanique 7.5 ml d'éthanol, 1,5 ml de triéthylamine et 0.73 ml d'acide 3-amino-3-ethoxyacrylique. On ajoute ensuite 500 mg de dichlorhydrate d'hexahydropyridazine (composé **B**) et on agite pendant 3 heures à température ambiante.
On filtre l'insoluble et distille le solvant sous vide. Le solide est repris par le minimum d'eau, filtré et séché sous vide.

On obtient ainsi 0.9 g de poudre légèrement jaune.

Les spectres de RMN ($^1$H 400 MHz et $^{13}$C 100,61 MHz DMSO $d_6$) et de masse sont conformes à la structure attendue **C**.

Synthèse du 3-amino-2-nitroso-5,6,7,8-tetrahydro-1H-pyrazolo[1,2-a]pyridazin-1-one : D

Dans un tricol de 50 ml équipé d'un réfrigérant et d'un thermomètre, on introduit sous agitation mécanique 20 ml d'acide chlorhydrique à 35% et 1g de 3-amino-5,6,7,8-tetrahydro-1H-pyrazolo[1,2-a]pyridazin-1-one(composé **C**).
On refroidit à 0°C et on coule une solution de 675 mg nitrite de sodium dans 5 ml d'eau en maintenant cette température.
La couleur du mélange réactionnel vire du jaune à l'orange et un précipité commence à se former.
En 30 minutes, la réaction est terminée et le solide orange est filtré sur verre fritté n°4, lavé à l'eau puis séché sous vide. Le rendement est de 78.3%.
Les spectres de RMN ($^1$H 400 MHz et $^{13}$C 100,61 MHz DMSO $d_6$) masse sont conformes à la structure attendue **D**.

Synthèse du chlorhydrate de 2,3-diamino-5,6,7,8-tetrahydro-1H-pyrazolo[1,2-alpvridazin-1-one : E

Dans un autoclave de 300 ml contenant 250 ml d 'éthanol, on introduit 1.3 g de 3-amino-2-nitroso-5,6,7,8-tetrahydro-1H-pyrazolo[1,2-a]pyridazin-1-one (composé **D**) et 250 mg de palladium sur charbon à 5%. La réduction est réalisée sous une agitation de 2000 tr/min, à une température de 60°C et sous une pression d'hydrogène de 6 bars.
Au bout de 2 heures de réaction, il n'y a plus de consommation d'hydrogène et le milieu est refroidi à 20°C.
Le catalyseur est éliminé par filtration sous azote après refroidissement à température ambiante et la solution est versée sur 75 ml de dioxanne chlorhydrique.
La solution ainsi obtenue est évaporée jusqu'à obtention d'une poudre légèrement jaune que l'on reprend dans de l'éther diisopropylique.
Le solide est récupéré par filtration. Après séchage sous vide en présence de pentaoxyde de phosphore, on obtient 1.1 g de dichlorhydrate de 2,3-diamino-5,6,7,8-tetrahydro-1H-pyrazolo[1,2-a]pyridazin-1-one.
Les spectres de RMN ($^1$H 400 MHz et $^{13}$C 100,61 MHz DMSO $d_6$) et de masse sont conformes à la structure attendue **E**.

**Exemple 7 : Synthèse du chlorhydrate de 4-Amino-1,2-diethyl-5-pyrrolidin-1-yl-1,2-dihydro-pyrazol-3-one**

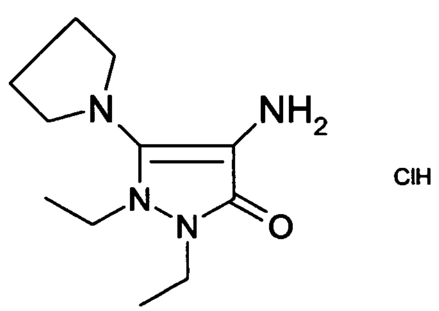

**Etape 1 : Synthèse de la 1, 2 - diéthylpyrazolidine-3,5-dione**

Dans un tricol de 3000 ml muni d'un thermomètre et sous agitation magnétique, on introduit successivement sous atmosphère d'azote 100 g du dichlorhydrate de diéthylhydrazine (0,63 moles) dans 1000 ml de dichlorométhane, 85,3 g d'acide malonique (0,82 moles ; 1,3éq.), 196 g de hydroxybenzotriazole (1,45 moles ; 2,3éq.), 278 g de 1-(3-diméthylaminopropyl)-3-ethyl-carbodiimide, hydrochlorure (EDCI ; 1,45 moles ; 2,3 éq.).

Le milieu réactionnel est ensuite refroidi entre 0°C et 5°C. On y additionne alors lentement 407 g de N, N-diisopropylethylamine (3,14 moles ; 520 ml : 5éq.). A la fin de l'addition, le milieu réactionnel devenu homogène est laissé sous agitation à température ambiante. Après une nuit à température ambiante, la réaction est terminée.

Le milieu réactionnel est lavé par trois fois 600 ml d'eau permutée. La phase organique est séchée sur sulfate de sodium, filtrée et concentrée sous vide pour fournir 46g de produit brut. La pyrazolidine-dione étant soluble en milieu aqueux, la phase aqueuse est donc concentrée à sec puis reprise par 800 ml d'une solution d'acide chlorhydrique 1 N. Le précipité formé est filtré et la phase aqueuse est extraite par trois fois 1300 ml de dichlorométhane. Les phases organiques rassemblées sont séchées sur sulfate de sodium, filtrées et concentrées sous vide pour fournir 67,5 g de

poduit brut.

On obtient ainsi la 1,2-diéthylpyrazolidine-3,5-dione sous forme de solide jaune avec un rendement de 40% (39,5 g).

**Etape 2 : Synthèse de la 1,2-diéthyl-3-chloro-5-pyrazolone**

Dans un tricol de 500 ml équipé d'un réfrigérant et d'une agitation magnétique, sont introduits, sous atmosphère d'azote, 30 g de 1,2 g de 1,2-diéthylpyrazolidine-3,5-dione (0,19 moles) en solution dans 200 ml de toluène et 35,8 ml d'oxyde de trichlorophosphine (258,9g ; 0,38 moles ; 2éq.).

Le milieu réactionnel est porté au reflux du toluène et la réaction est suivie par CCM (dichlorométhane/méthanol 95/5). Le milieu réactionnel initialement sous forme de pâte, s'homogénéise dès le reflux puis devient biphasique.

Après une heure de reflux, la réaction est hydrolysée à 0°C par addition très lente de 100 ml d'eau permutée. Après décantation, la phase toluène est séparée de la phase aqueuse. La phase aqueuse est lavée par 50 ml de toluène puis amenée à pH 12 par 184 ml d'une solution de soude à 35 %. On observe la formation d'un précipité. La phase aqueuse est portée à 100°C pendant 10 minutes et le précipité se solubilise. Le milieu réactionnel présente alors deux phases. La phase supérieure de coloration brune est séparée après décantation à chaud. Cette phase supérieure est solubilisée par 200 ml de dichlorométhane, lavée par une fois 50 ml d'eau permutée, séchée sur sulfate de sodium et concentrée sous vide pour fournir 20,5 g d'une huile brune.

Un précipité se forme dans la phase aqueuse inférieure lors du retour à la température ambiante. Après filtration sur fritté, le précipité est rincé à l'eau et le filtrat est extrait par trois fois 300 ml de dichlorométhane. La phase dichlorométhane est séchée sur sulfate de sodium et concentrée sous vide pour fournir 5,5 g de cristaux bruns.

L'huile et les cristaux bruns sont rassemblés, greffés sur silice et chromatographiés sur gel de silice (40-60μm ; 2000 g) par un gradient d'élution :

1) Dichlorométhane 100 (131)
2) Dichlorométhane / MeOH 99,5/0,5 (0,8 litres)
3) Dichlorométhane / MeOH 99/1 (8 litres) produit attendu + 15 % impureté m=6,6 g
4) Dichlorométhane / MeOH 98,5/1,5 (35 litres) produit attendu (14,7 g)

La 1,2-diéthyl-3-chloro-5-pyrazolone est ainsi obtenue sous la forme de cristaux jaune avec un rendement de 44%.

**Etape 3 : Synthèse du 1,2-Diethyl-5-pyrrolidin-1-yl-1,2-dihydro-pyrazol-3-one**

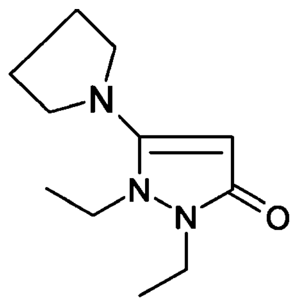

Dans un réacteur 2,5 ml du micro-onde Biotage initiator, on introduit 1g de 5-Chloro-1,2-diéthyl-1,2-dihydro-pyrazol-3-one ($5{,}7.10^{-4}$ moles), auquel on ajoute 2 ml de pyrrolidine (4,2 eq).

Conditions opératoires : micro-onde à puissance maximum $\theta$= 120° C pendant 17 min.

Après 17 minutes, la réaction est terminée (suivi CCM éluant : 90/10 $CH_2Cl_2$ / MeOH).

5 ml d'eau déminéralisée sont alors ajoutés au milieu réactionnel, puis l'ensemble est transféré en ampoule à décanter. La phase aqueuse est extraite avec quatre fois 10 ml de dichlorométhane. Les phases organiques sont ensuite réunies et séchées sur du sulfate de sodium anhydre, puis filtrées et évaporées à sec. 1.2 grammes d'une huile orangée brune sont obtenus avec un rendement de 100%.

**RMN** ($^1$H 400 MHz DMSO $d_6$)

0.81 (1t, 3H), 0.89 (1t, 3H), 1.88 (1m, 1H), 3.22 (1m, 4H), 3.4 (1m, 4H), 4.4 (1s, 1H) Masse : Analyse faite en OpenLynx (FIA/MS).

La masse principalement détectée est en accord avec la structure attendue : M=20

**Etape 4: Synthèse du 1,2-diéthyl-4-nitroso-5-pyrrolidin-1-yl-1,2-dihydro-3H-pyrazol-3-one**

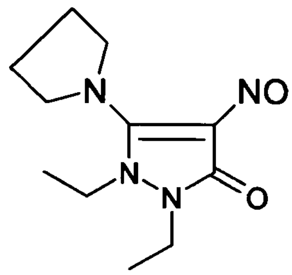

Dans un tricol de 25 ml tout équipé, on introduit 1.2 g de 1,2-Diéthyl-5-pyrrolidin-1-yl-1,2-dihydro-pyrazol-3-one que l'on solubilise dans un mélange composé de 0.84 ml d'acide chlorhydrique 37% et de 4 ml d'eau déminéralisée. Le milieu réactionnel est refroidit entre 0°C et 5°C, à l'aide d'un bain d'eau glacée.
Une solution composée de 400 mg de nitrite de sodium ($5{,}7.10^{-4}$ moles) solubilisés dans 0.6 ml d'eau déminéralisée, est alors ajoutée au goutte à goutte.
Le milieu réactionnel vire instantanément au rouge vif dès l'ajout de la première goutte du mélange précédent.

Après une heure, la réaction est terminée.
Le pH est ajusté à environ 7-8 à l'aide d'une solution de soude à 30%, puis le milieu réactionnel est transféré en ampoule à décanter. La phase aqueuse est extraite avec 4 fois 10 ml de dichlorométhane. Les phases organiques sont réunies et séchées sur sulfate de sodium anhydre puis évaporées à sec. 1.2 grammes d'une poudre bleu turquoise sont obtenus avec un rendement de 89.6 %.

Les spectres de RMN ($^1H$ 400 MHz DMSO $d_6$) et de masse sont conformes à la structure attendue.
**RMN** ($^1H$ 400 MHz DMSO $d_6$)
0.94 (1t, 3H), 1 (1t, 3H), 2.05 (1m, 4H), 3.51 (1q, 4H), 3.76 (1q, 4H), 3.94 (1m, 4H) Analyse faite en OpenLynx (FIA/MS).
La masse principalement détectée est en accord avec la structure attendue. M=238.

**Etape 5 : Synthèse du chlorhydrate de 4-Amino-1,2-diéthyl-5-pyrrolidin-1-ym-1,2-dihydro-pyrazol-3-one:**

Dans un Tricol de 500ml tout équipé, on introduit 4 grammes de zinc en poudre (0.06 moles) dans 300 ml d'éthanol absolu auxquels on ajoute 1 ml d'acide acétique.
Le milieu réactionnel est chauffé à 40°C, puis 1,15 g ($4{,}8\ .10^{-3}$ moles) de 1,2-diéthyl-4-nitroso-5-pyrrolidin-1-yl-1,2-dihydro-3H-pyrazol-3-one sont introduits par petites spatules. 4 ml d'acide acétique sont enfin introduits ml par ml et le milieu est porté au reflux. Le milieu est totalement soluble et incolore. Après 30 minutes, la réaction est terminée sur CCM selon l'éluant Acétate d'éthyle/MeOH 90/10.

Le milieu réactionnel est refroidi puis filtré sur fritté contenant un lit de Céllite 545. Les eaux mères sont filtrées dans un ballon contenant 2,5 ml d'isopropanol chlorhydrique 5N refroidi. Le tout est ensuite évaporé à sec. Le produit obtenu est une poudre rose conforme par RMN et Masse.
**RMN** ($^1H$ 400 MHz DMSO $d_6$)
0.79 (1t, 3H), 0.96 (1t, 3H), 1.87 (1m, 4H), 3.49 (1q, 2H), 3.59 (1m, 6H)
Analyse FIA/MS réalisée via OpenLynx.
Les ions quasi moléculaires [M+H]+, [M+Na]+, [2M+H]+, [2M+Na]+ de la base attendue C11H20N4O sont principalement détectés.

En reproduisant les étapes précédentes avec les réactifs appropriés, on peut obtenir le 4-amino-5-[3-(diméthylamino)pyrrolidin-1-yl]-1,2-diéthyl-1,2-dihydro-3H-pyrazol-3-one hydrochlorure

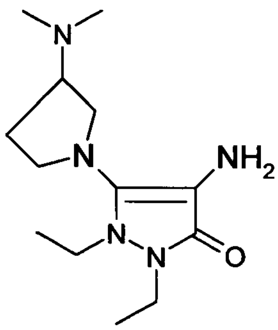

2HCl

## EXEMPLES DE TEINTURE

On prépare les compositions de teinture suivantes :

| Constituant | g% |
|---|---|
| Alcool oléïque polyglycérolé à 2 moles de glycérol | 4 |
| Alcool oléïque polyglycérolé à 4 moles de glycérol | 5.69 MA |
| Acide oléïque | 3 |
| Amine oléïque à 2 moles d'oxyde d'éthylène vendue sous la dénomination commerciale ETHOMEEN 012 par la société Akzo | 7 |
| Laurylamino succinamate de diéthylaminopropyle , sel de sodium à 55% de M.A. | 3.0 MA |
| Alcool oléïque | 5 |
| Diethanolamide d'acide oléïque | 12 |
| Alcool éthylique | 7 |
| Propylène glycol | 3.5 |
| Dipropylèneglycol | 0.5 |
| Monométhylether de propylèneglycol | 9 |
| Antioxydant /séquestrant | qs |
| Acétate d'ammonium | 0.8 |
| Métabisulfite de sodium en solution aqueuse à 35% | 0.455 |
| colorants d'oxydation | Voir tableau 1 |
| Ammoniaque à 20 % | 10 |
| Eau déminéralisée q.s.p. | 100 g |

**Tableau 1** - Concentrations des colorants d'oxydation présents

| compositions | B1 (mol%) | 6-hydroxy indole (mol%) | B2 (mol%) |
|---|---|---|---|
| I | $3.10^{-2}$ | $3.1.10^{-2}$ | $0.1.10^{-2}$ |
| II | $3.1.10^{-2}$ | $3.1.10^{-2}$ | 0 |
| III | 0 | $3.1.10^{-2}$ | $3.1.10^{-2}$ |

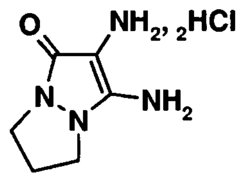

B1 B2...

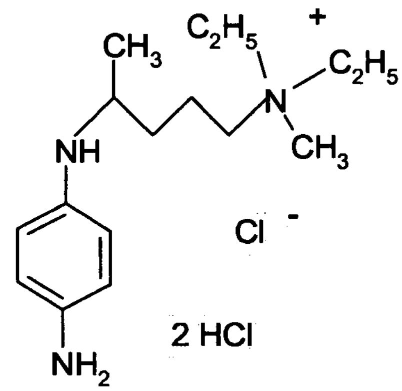

Au moment de l'emploi, les compositions I, II et III sont mélangées à un oxydant titrant 20V en eau oxygénée (poids pour poids), puis appliquées sur des mèches de cheveux naturels à 90% blancs permanentés (BP) et des mèches de cheveux naturels à 90% blancs décolorés. Le temps de pause est de 30 min
Après 30 minutes de pose à température ambiante, les mèches sont rincées, lavées avec un shampooing standard, rincées à nouveau puis séchées.

La couleur des mèches a été évaluée dans le système L* a* b*, au moyen d'un spectrophotomètre CM 2002 MINOLTA ®, (Illuminant D65). Dans ce système L* a* b*, L* représente l'intensité de la couleur, a* indique l'axe de couleur vert/rouge et b* l'axe de couleur bleu/jaune. Plus la valeur de L est faible, plus la couleur est foncée ou très intense. Plus la valeur de a* est élevée plus la nuance est rouge et plus la valeur de b* est élevée plus la nuance est bleue.

On mesure l'uniformité de la couleur entre les mèches permanentés et décolorés par la variation de la coloration mesurée par (ΔE) selon l'équation suivante :

$$\Delta E = \sqrt{(L^* - L_o^*)^2 + (a^* - a_o^*)^2 + (b^* - b_o^*)^2}$$

Dans cette équation, L*, a* et b* représentent les valeurs mesurées sur cheveux décolorés et L0*, a0* et b0* représentent les valeurs mesurées sur cheveux permanentés.

Plus la valeur de ΔE est importante, plus la différence de couleur est importante, ce qui montre une moindre uniformité de la couleur.

Les résultats sont reportés dans les tableaux ci dessous

**Tableau 2 -**

| | ΔE (BN/SA) |
|---|---|
| composition I | 0.94 |
| composition II | 4.89 |
| Composition III | 4.95 |

Le tableau 2 montre que la composition I selon l'invention qui associe une base d'oxydation pyrazolidinone de formule (I) et une base d'oxydation cationique conduit à une coloration moins sélective entre cheveu permanenté et cheveu décoloré que la composition II qui contient uniquement la base d'oxydation de formule (I) et que la composition III qui contient uniquement la base cationique.

## Revendications

**1.** Composition de teinture des fibres kératiniques comprenant, dans un milieu approprié,

• au moins une base d'oxydation choisie parmi un dérivé de la diamino-N,N-dihydro-pyrazolone de formule (I) ou l'un de ses sels d'addition : dans laquelle :

$R_1$, $R_2$, $R_3$ et $R_4$, identiques ou différents, représentent :

- un radical alkyle en $C_1$-$C_{10}$ linéaire ou ramifié éventuellement substitué par un ou plusieurs radicaux choisi dans le groupe constitué par un radical $OR_5$, un radical $NR_6R_7$, un radical carboxy, un radical sulfonique, un radical carboxamido $CONR_6R_7$, un radical sulfonamido $SO_2NR_6R_7$, un hétéroaryle, un aryle éventuellement substitué par un o plusieurs groupes ($C_1$-$C_4$)alkyle, hydroxy, alcoxy en $C_1$-$C_2$, amino, (di)alkyl($C_1$-$C_2$)amino;
- un radical aryle éventuellement substitué par un ou plusieurs ($C_1$-$C_4$)alkyle, hydroxy, alcoxy en $C_1$-$C_2$, amino, (di)alkyl($C_1$-$C_2$)amino ;
- un radical hétéroaryle à 5 ou 6 chaînons, éventuellement substitué par un ou plusieurs radicaux choisis parmi ($C_1$-$C_4$)alkyle, ($C_1$-$C_2$)alcoxy;

$R_3$ et $R_4$ peuvent représenter également un atome d'hydrogène ; $R_5$, $R_6$ et $R_7$, identiques ou différents, représentent

- un atome d'hydrogène ;
- un radical alkyle linéaire ou ramifié en $C_1$-$C_4$ éventuellement substitué par un ou plusieurs radicaux choisis parmi hydroxy, alcoxy en $C_1$-$C_2$, carboxamido $CONR_8R_9$, sulfonyle $SO_2R_8$, aryle éventuellement substitué par un ($C_1$-$C_4$)alkyle, hydroxy, alcoxy en $C_1$-$C_2$, amino, (di)alkyl($C_1$-$C_2$)amino ; un aryle éventuellement substitué par un ($C_1$-$C_4$)alkyle, hydroxy, alcoxy en $C_1$-$C_2$, amino, (di)alkyl($C_1$-$C_2$) amino ;

$R_6$ et $R_7$, identiques ou différents, peuvent représenter également un radical carboxamido $CONR_8R_9$; un radical sulfonyle $SO_2R_8$ ;
$R_8$ et $R_9$, identiques ou différents, représentent un atome d'hydrogène ; un radical alkyle en $C_1$-$C_4$ linéaire ou ramifié éventuellement substitués par un ou plusieurs hydroxy, alcoxy en $C_1$-$C_2$ ;
$R_1$ et $R_2$ d'une part, et $R_3$ et $R_4$ d'autre part peuvent former avec le ou les atomes d'azote auxquels ils sont rattachés, un hétérocycle saturé ou insaturé, comportant 5 à 7 chaînons, éventuellement substitué par un ou plusieurs radicaux choisis dans le groupe constitué par les atomes d'halogène, les radicaux amino, (di) alkyl($C_1$-$C_4$)amino, hydroxy, carboxy, carboxamido, ($C_1$-$C_2$)alcoxy, les radicaux alkyles en $C_1$-$C_4$ éventuellement substitués par un ou plusieurs radicaux hydroxy, amino, (di)-alkylamino, alcoxy, carboxy, sulfonyle ;
$R_3$ et $R_4$ peuvent également former ensemble avec l'atome d'azote auquel ils sont rattachés, un hétérocycle à 5 ou 7 chaînons dont les atomes de carbone peuvent être remplacés par un atome d'oxygène ou d'azote éventuellement substitué, et

• au moins un colorant d'oxydation substitué par au moins un radical comportant un atome d'azote quaternisé.

**2.** Composition selon la revendication 1 dans laquelle $R_1$ et $R_2$ sont choisis parmi un radical alkyle en $C_1$-$C_6$ éventuellement substitué par un hydroxy, ($C_1$-$C_2$)alcoxy, amino, (di)alkyl($C_1$-$C_2$)amino; un radical phényle, méthoxyphényle, éthoxyphényle, benzyle.

**3.** Composition selon la revendication 2 dans laquelle $R_1$ et $R_2$ sont choisis parmi un radical méthyle, éthyle, 2-hydroxyéthyle, 3-hydroxypropyle, 2-hydroxypropyle, phényle.

**4.** Composition selon la revendication 1, dans laquelle $R_1$ et $R_2$ forment ensemble avec les atomes d'azotes auxquels ils sont rattachés un cycle à 5 ou 6 chaînons saturé ou insaturé éventuellement substitués.

**5.** Composition selon l'une quelconque des revendications 1 et 4, dans laquelle $R_1$ et $R_2$ forment ensemble avec les atomes d'azotes auxquels ils sont rattachés un cycle pyrazolidine, pyridazolidine, éventuellement substitué par un ou plusieurs radicaux alkyle en $C_1$-$C_4$, un hydroxy, un ($C_1$-$C_2$)alcoxy, un carboxy, un carboxamido, un amino, un (di)alkyl($C_1$-$C_2$)amino.

**6.** Composition selon l'une quelconque des revendications 1 et 4 à 5, dans laquelle $R_1$ et $R_2$ forment ensemble avec les atomes d'azotes auxquels ils sont rattachés un cycle pyrazolidine, pyridazolidine.

7. Composition selon l'une quelconque des revendications précédentes dans laquelle $R_3$ et $R_4$ sont choisis parmi un atome d'hydrogène ; un radical alkyle en $C_1$-$C_6$ linéaire ou ramifié éventuellement substitué par un ou plusieurs hydroxy, ($C_1$-$C_2$)alcoxy, amino, un (di)alkyl($C_1$-$C_2$)amino; un radical phényle éventuellement substitué par un ou plusieurs radicaux hydroxy, amino, ($C_1$-$C_2$)alcoxy.

8. Composition selon l'une quelconque des revendications 1 à 7, dans laquelle $R_3$ et $R_4$ sont choisis parmi un atome d'hydrogène, un radical méthyle, éthyle, isopropyle, 2-hydroxyéthyle, 3-hydroxypropyle, 2-hydroxypropyle, 2-carboxyéthyle.

9. Composition selon la revendication 8 dans laquelle $R_3$ et $R_4$ représentent un atome d'hydrogène.

10. Composition selon l'une quelconque des revendications 1 à 6, dans laquelle $R_3$ et $R_4$ forment ensemble avec l'atome d'azote auquel ils sont rattachés un cycle à 5 ou 7 chaînons choisi parmi les hétérocycles pyrrolidine, pipéridine, homopipéridine, pipérazine, homopipérazine ; lesdits cycles pouvant être substitués par un ou plusieurs radicaux hydroxy, amino, (di)alkyl($C_1$-$C_2$)amino, carboxy, carboxamido, alkyle en $C_1$-$C_4$ éventuellement substitué par un ou plusieurs hydroxy, amino, (di)alkylamino en $C_1$-$C_2$.

11. Composition selon l'une quelconque des revendications 1 à 6 et 10, dans laquelle $R_3$ et $R_4$ forment ensemble avec l'atome d'azote auquel ils sont rattachés un cycle à 5 ou 7 chaînons choisi parmi la pyrrolidine, le 2,5-diméthylpyrrolidine, l'acide pyrrolidine-2-carboxylique, l'acide 3-hydroxypyrrolidine-2-carboxylique, l'acide 4-hydroxypyrrolidine-2-carboxylique, la 2,4-dicarboxypyrrolidine, la 3-hydroxy-2-hydroxyméthylpyrrolidine, la 2-carboxamidopyrrolidine, la 3-hydroxy-2-carboxamidopyrrolidine, la 2-(diéthylcarboxamido)pyrrolidine, la 2-hydroxyméthyl pyrrolidine, la 3,4-dihydroxy-2-hydroxyméthylpyrrolidine, la 3-hydroxypyrrolidine, la 3,4-dihydroxypyrrolidine, la 3-aminopyrrolidine, la 3-méthylaminopyrrolidine, la 3-diméthylamino-pyrrolidine, la 4-amino-3-hydroxy pyrrolidine, la 3-hydroxy-4-(2-hydroxyéthyl)amino-pyrrolidine, la pipéridine, la 2,6-diméthylpipéridine, la 2-carboxypipéridine, la 2-carboxamidopipéridine, la 2-hydroxyméthylpipéridine, la 3-hydroxy-2-hydroxyméthylpipéridine, 3-hydroxypipéridine, la 4-hydroxypipéridine, la 3-hydroxyméthylpipéridine, la homopipéridine, la 2-carboxyhomopipéridine, la 2-carboxamidohomopipéridine, l'homopipérazine, le N-méthyl-homopipérazine, le N-(2-hydroxyéthyl)-homopipérazine.

12. Composition selon l'une quelconque des revendications 1 à 6 et 10 à 11, dans laquelle $R_3$ et $R_4$ forment ensemble avec l'atome d'azote auquel ils sont rattachés un cycle à 5 ou 7 chaînons choisi parmi la pyrrolidine, la 3-hydroxypyrrolidine, la 3-aminopyrrolidine, la 3-diméthylamino-pyrrolidine, l'acide pyrrolidine-2-carboxylique, l'acide 3-hydroxypyrrolidine-2-carboxylique, la pipéridine, l'hydroxypipéridine, l'homopipéridine, le diazépane, la N-méthylhomopipérazine, la N β-hydroxyéthylhomopipérazine.

13. Composition selon l'une quelconque des revendications 1 à 6 et 10 à 12, dans laquelle $R_3$ et $R_4$ forment ensemble avec l'atome d'azote auquel ils sont rattachés un cycle à 5 chaînons tels que la pyrrolidine, la 3-hydroxypyrrolidine, la 3-aminopyrrolidine, la 3-diméthylamino-pyrrolidine.

14. Composition selon la revendication 1 à 13, dans laquelle le composé de formule (I) ou un de ses sels d'addition est choisi parmi 2,3-Diamino-6,7-dihydro-1H,5H-pyrazolo[1,2-a]pyrazol-1-one 2-Amino-3-éthylamino-6,7-dihydro-1H, 5H-pyrazolo[1,2-a]pyrazol-1-one 2-Amino-3-isopropylamino-6,7-dihydro-1H,5H-pyrazolo[1,2-a]pyrazol-1-one 2-Amino-3-(pyrrolidin-1-yl)-6,7-dihydro-1H,5H-pyrazolo[1,2-a]pyrazol-1-one 4,5-diamino-1,2-diméthyl-1,2-dihydro-pyrazol-3-one. 4,5-diamino-1,2-diéthyl-1,2-dihydro-pyrazol-3-one. 4,5-diamino-1,2-di-(2-hydroxyéthyl)-1,2-dihydro-pyrazol-3-one. 2-amino-3-(2-hydroxyéthyl)amino-6,7-dihydro-1H,5H-pyrazolo[1,2-a]pyrazol-1-one 2-amino-3-diméthylamino-6,7-dihydro-1H,5H-pyrazolo[1,2-a]pyrazol-1-one 2,3-diamino-5,6,7,8-tétrahydro-1H,6H-pyridazino[1,2-a]pyrazol-1-one 4-Amino-1,2-diethyl-5-(pyrrolidin-1-yl)-1,2-dihydro-pyrazol-3-one 4-Amino-5-(3-dimethy) amino-pyrrolidin-1-yl)-1,2-diethyl-1,2-dihydro-pyrazol-3-one 2,3-diamino-6-hydroxy-6,7-dihydro-1H,5H-pyrazolo [1,2-a]pyrazol-1-one.

15. Composition selon l'une quelconque des revendications précédentes dans laquelle le ou les colorants d'oxydation substitués par au moins un radical comportant un atome d'azote quaternisé sont substitués par des radicaux trialkylammonium ou des atomes d'azote quaternisés inclus dans un cycle.

16. Composition selon la revendications 15 dans laquelle les radicaux comportant un atome d'azote quaternisé sont choisis parmi les radicaux triméthylammonium, triéthyl ammonium, diéthyl-méthyl ammonium, tri butylammmonium, pyrrolium, imidazolium, pyrazolium, oxazolium, thiazolium, triazolium, pyridinium, pyrimidinium, pyrazinium, oxazinium et triazinium.

**17.** Composition selon l'une quelconque des revendications 1 à 16 dans laquelle les colorants d'oxydation substitués par au moins un radical comportant un atome d'azote quaternisé sont des bases d'oxydation ou des coupleurs.

**18.** Composition selon l'une quelconque des revendications précédentes dans laquelle les bases d'oxydation substitués par au moins un radical comportant un atome d'azote quaternisé sont choisis parmi les para-phénylènediamines, les bis-phénylalkylènediamines, les para-aminophénols, les bis-para-aminophénols, les ortho-aminophénols, les bases hétérocycliques telles que les dérivés pyridiniques, les dérivés pyrimidiniques et les dérivés pyrazoliques, les dérivés pyrazolo[1,5-a] pyrimidine et leurs sels d'addition.

**19.** Composition selon l'une quelconque des revendications 1 à 18 dans laquelle les coupleurs substitués par au moins un radical comportant un atome d'azote quaternisé sont choisis parmi les coupleurs méta-phénylènediamines, méta-aminophénols, méta-diphénols, naphtaléniques, ou les coupleurs hétérocycliques et leur sels d'addition.

**20.** Composition selon l'une quelconque des revendications 1 à 18 comprenant au moins une base d'oxydation additionnelle non substituée par au moins un radical comportant un atome d'azote quaternisé.

**21.** Composition selon l'une quelconque des revendications 1 à 18 comprenant au moins un coupleur additionnel non substitué par au moins un radical comportant un atome d'azote quaternisé.

**22.** Composition selon l'une quelconque des revendications 1 à 19 dans laquelle le colorant d'oxydation substitué par au moins un radical comportant un atome d'azote quaternisé est choisi parmi les paraphénylènediamines

**23.** Composition selon l'une quelconque des revendications 1 à 19 dans laquelle le colorant d'oxydation substitué par au moins un radical comportant un atome d'azote quaternisé est choisi parmi les paraphénylènediamines à groupement pyrrolidinyle substitués par un ou plusieurs radicaux comportant un atome d'azote quaternisé.

**24.** Composition selon l'une quelconque des revendications précédentes comprenant de plus un agent oxydant.

**25.** Procédé de teinture des fibres kératiniques, **caractérisé en ce qu'**une composition telle que définie dans l'une quelconque des revendications 1 à 23 est appliquée sur les fibres kératiniques en présence d'un agent oxydant pendant un temps suffisant pour développer la coloration désirée.

**26.** Procédé selon la revendication 25 dans lequel l'agent oxydant est choisi parmi le peroxyde d'hydrogène, le peroxyde d'urée, les bromates de métaux alcalins, les persels, les peracides et les enzymes oxydases.

**27.** Dispositif à plusieurs compartiments dans lequel un premier compartiment contient une composition tinctoriale telle que définie dans l'une quelconque des revendications 1 à 23 et un deuxième compartiment contient un agent oxydant.

**28.** Utilisation pour la teinture d'oxydation des fibres kératiniques d'une composition telle que définie dans l'une quelconque des revendications 1 à 24.

## Claims

**1.** Composition for dyeing keratinous fibres comprising, in an appropriate medium,

- at least one oxidation base chosen from a diamino-N,N-dihydropyrazolone derivative of formula (I) or one of its addition salts:

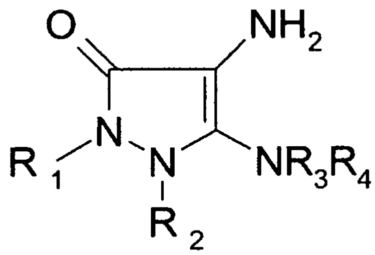

(I)

in which:

$R_1$, $R_2$, $R_3$ and $R_4$, which are identical or different, represent:

- a linear or branched $C_1$-$C_{10}$ alkyl radical optionally substituted with one or more radicals chosen from the group consisting of a radical $OR_5$, a radical $NR_6R_7$, a carboxyl radical, a sulphonic radical, a carboxamido radical $CONR_6R_7$, a sulphonamido radical $SO_2NR_6R_7$, a heteroaryl, an aryl optionally substituted with one or more ($C_1$-$C_4$)alkyl, hydroxyl, $C_1$-$C_2$ alkoxy, amino, (di) alkyl ($C_1$-$C_2$)amino groups;
- an aryl radical optionally substituted with one or more ($C_1$-$C_4$)alkyl, hydroxyl, $C_1$-$C_2$ alkoxy, amino, (di) alkyl ($C_1$-$C_2$) amino;
- a 5- or 6-membered heteroaryl radical optionally substituted with one or more radicals chosen from ($C_1$-$C_4$) alkyl, ($C_1$-$C_2$)alkoxy;

$R_3$ and $R_4$ may also represent a hydrogen atom;
$R_5$, $R_6$ and $R_7$, which are identical or different, represent

- a hydrogen atom;
- a linear or branched $C_1$-$C_4$ alkyl radical optionally substituted with one or more radicals chosen from hydroxyl, $C_1$-$C_2$ alkoxy, carboxamido $CONR_8R_9$, sulphonyl $SO_2R_8$, aryl optionally substituted with ($C_1$-$C_4$)alkyl, hydroxyl, $C_1$-$C_2$ alkoxy, amino, (di) alkyl ($C_1$-$C_2$)amino; aryl optionally substituted with ($C_1$-$C_4$)alkyl, hydroxyl, $C_1$-$C_2$ alkoxy, amino, (di)alkyl ($C_1$-$C_2$) amino;

$R_6$ and $R_7$, which are identical or different, may also represent a carboxamido radical $CONR_8R_9$; a sulphonyl radical $SO_2R_8$;
$R_8$ and $R_9$, which are identical or different, represent a hydrogen atom; a linear or branched $C_1$-$C_4$ alkyl radical optionally substituted with one or more hydroxyl, $C_1$-$C_2$ alkoxy;
$R_1$ and $R_2$, on the one hand, and $R_3$ and $R_4$, on the other hand, may form with the nitrogen atom(s) to which they are attached a saturated or unsaturated 5- to 7-membered heterocycle optionally substituted with one or more radicals chosen from the group consisting of halogen atoms, amino, (di)alkyl($C_1$-$C_4$)amino, hydroxyl, carboxyl, carboxamido or ($C_1$-$C_2$)alkoxy radicals, $C_1$-$C_4$ alkyl radicals optionally substituted with one or more hydroxyl, amino, (di)alkylamino, alkoxy, carboxyl or sulphonyl radicals;
$R_3$ and $R_4$ may also form together with the nitrogen atom to which they are attached a 5- or 7-membered heterocycle whose carbon atoms may be replaced with an oxygen or optionally substituted nitrogen atom, and

• at least one oxidation dye substituted with at least one radical comprising one quaternized nitrogen atom.

**2.** Composition according to Claim 1, in which $R_1$ and $R_2$ are chosen from a $C_1$-$C_6$ alkyl radical optionally substituted with a hydroxyl, ($C_1$-$C_2$)alkoxy, amino, (di)alkyl($C_1$-$C_2$)amino; a phenyl, methoxyphenyl, ethoxyphenyl, benzyl radical.

**3.** Composition according to Claim 2, in which $R_1$ and $R_2$ are chosen from a methyl, ethyl, 2-hydroxyethyl, 3-hydroxypropyl, 2-hydroxypropyl and phenyl radical.

4. Composition according to Claim 1, in which $R_1$ and $R_2$ form together with the nitrogen atoms to which they are attached a saturated or unsaturated optionally substituted 5- or 6-membered ring.

5. Composition according to any one of Claims 1 to 4, in which $R_1$ and $R_2$ form together with the nitrogen atoms to which they are attached a pyrazolidine or pyridazolidine ring optionally substituted with one or more $C_1$-$C_4$ alkyl radicals, a hydroxyl, a ($C_1$-$C_2$)alkoxy, a carboxyl, a carboxamido, an amino, a (di) alkyl ($C_1$-$C_2$) amino.

6. Composition according to any one of Claims 1 and 4 to 5, in which $R_1$ and $R_2$ form together with the nitrogen atoms to which they are attached a pyrazolidine or pyridazolidine ring.

7. Composition according to any one of the preceding claims, in which $R_3$ and $R_4$ are chosen from a hydrogen atom; a linear or branched $C_1$-$C_6$ alkyl radical optionally substituted with one or more hydroxyl, ($C_1$-$C_2$)alkoxy, amino, a (di) alkyl ($C_1$-$C_2$)amino; a phenyl radical optionally substituted with one or more hydroxyl, amino or ($C_1$-$C_2$)alkoxy radicals.

8. Composition according to any one of Claims 1 to 7, in which $R_3$ and $R_4$ are chosen from a hydrogen atom, a methyl, ethyl, isopropyl, 2-hydroxyethyl, 3-hydroxypropyl, 2-hydroxypropyl and 2-carboxyethyl radical.

9. Composition according to Claim 8, in which $R_3$ and $R_4$ represent a hydrogen atom.

10. Composition according to any one of Claims 1 to 6, in which $R_3$ and $R_4$ form together with the nitrogen atom to which they are attached a 5- or 7-membered ring chosen from the pyrrolidine, piperidine, homopiperidine, piperazine and homopiperazine heterocycles; it being possible for the said rings to be substituted with one or more hydroxyl, amino, (di) alkyl ($C_1$-$C_2$) amino, carboxyl or carboxamido radicals, or $C_1$-$C_4$ alkyl radicals optionally substituted with one or more hydroxyl, amino, $C_1$-$C_2$ (di)alkylamino.

11. Composition according to any one of Claims 1 to 6 and 10, in which $R_3$ and $R_4$ form together with the nitrogen atom to which they are attached a 5- or 7-membered ring chosen from pyrrolidine, 2,5-dimethylpyrrolidine, pyrrolidine-2-carboxylic acid, 3-hydroxypyrrolidine-2-carboxylic acid, 4-hydroxypyrrolidine-2-carboxylic acid, 2,4-dicarboxypyrrolidine, 3-hydroxy-2-hydroxymethylpyrrolidine, 2-carboxamidopyrrolidine, 3-hydroxy-2-carboxamidopyrrolidine, 2-(diethylcarboxamido)pyrrolidine, 2-hydroxymethylpyrrolidine, 3,4-dihydroxy-2-hydroxymethylpyrrolidine, 3-hydroxypyrrolidine, 3,4-dihydroxypyrrolidine, 3-aminopyrrolidine, 3-methylaminopyrrolidine, 3-dimethylaminopyrrolidine, 4-amino-3-hydroxypyrrolidine, 3-hydroxy-4-(2-hydroxyethyl)aminopyrrolidine, piperidine, 2,6-dimethylpiperidine, 2-carboxypiperidine, 2-carboxamidopiperidine, 2-hydroxymethylpiperidine, 3-hydroxy-2-hydroxymethylpiperidine, 3-hydroxypiperidine, 4-hydroxypiperidine, 3-hydroxymethylpiperidine, homopiperidine, 2-carboxyhomopiperidine, 2-carboxamidohomopiperidine, homopiperazine, N-methylhomopiperazine, N-(2-hydroxyethyl)homopiperazine.

12. Composition according to any one of Claims 1 to 6 and 10 to 11, in which $R_3$ and $R_4$ form together with the nitrogen atom to which they are attached a 5- or 7-membered ring chosen from pyrrolidine, 3-hydroxypyrrolidine, 3-aminopyrrolidine, 3-dimethylaminopyrrolidine, pyrrolidine-2-carboxylic acid, 3-hydroxypyrrolidine-2-carboxylic acid, piperidine, hydroxypiperidine, homopiperidine, diazepane, N-methylhomopiperazine, N-β-hydroxyethylhomopiperazine.

13. Composition according to any one of Claims 1 to 6 and 10 to 12, in which $R_3$ and $R_4$ form together with the nitrogen atom to which they are attached a 5-membered ring such as pyrrolidine, 3-hydroxypyrrolidine, 3-aminopyrrolidine, 3-dimethylaminopyrrolidine.

14. Composition according to any one of Claims 1 to 13, in which the compound of formula (I) or one of its addition salts is chosen from
2,3-diamino-6,7-dihydro-1H,5H-pyrazolo[1,2-a]pyrazol-1-one
2-amino-3-ethylamino-6,7-dihydro-1H,5H-pyrazolo[1,2-a]-pyrazol-1-one
2-amino-3-isopropylamino-6,7-dihydro-1H,5H-pyrazolo-[1,2-a]pyrazol-1-one
2-amino-3-(pyrrolidin-1-yl)-6,7-dihydro-1H,5H-pyrazolo-[1,2-a]pyrazol-1-one
4,5-diamino-1,2-dimethyl-1,2-dihydropyrazol-3-one
4,5-diamino-1,2-diethyl-1,2-dihydropyrazol-3-one
4,5-diamino-1,2-di(2-hydroxyethyl)-1,2-dihydropyrazol-3-one
2-amino-3-(2-hydroxyethyl)amino-6,7-dihydro-1H,5H-pyrazolo[1,2-a]pyrazol-1-one
2-amino-3-dimethylamino-6,7-dihydro-1H,5H-pyrazolo[1,2-a]pyrazol-1-one

2,3-diamino-5,6,7,8-tetrahydro-1H,6H-pyridazino-[1,2-a]pyrazol-1-one
4-amino-1,2-diethyl-5-(pyrrolidin-1-yl)-1,2-dihydro-pyrazol-3-one
4-amino-5-(3-dimethylaminopyrrolidin-1-yl)-1,2-diethyl-1,2-dihydropyrazol-3-one
2,3-diamino-6-hydroxy-6,7-dihydro-1H,5H-pyrazolo[1,2-a]pyrazol-1-one.

**15.** Composition according to any one of the preceding claims, in which the oxidation dye(s) substituted with at least one radical comprising a quaternized nitrogen atom is (are) substituted with trialkylammonium radicals or quaternized nitrogen atoms included in a ring.

**16.** Composition according to Claim 15, in which the radicals comprising a quaternized nitrogen atom are chosen from trimethylammonium, triethylammonium, diethylmethylammonium, tributylammonium, pyrrolium, imidazolium, pyrazolium, oxazolium, thiazolium, triazolium, pyridinium, pyrimidinium, pyrazinium, oxazinium and triazinium radicals.

**17.** Composition according to any one of Claims 1 to 16, in which the oxidation dyes substituted with at least one radical comprising a quaternized nitrogen atom are oxidation bases or couplers.

**18.** Composition according to any one of the preceding claims, in which the oxidation bases substituted with at least one radical comprising a quaternized nitrogen atom are chosen from para-phenylenediamines, bisphenyl-akylenediamines, para-aminophenols, bis-para-aminophenols, ortho-aminophenols, heterocyclic bases such as pyridine derivatives, pyrimidine derivatives and pyrazole derivatives, pyrazolo[1,5-a]pyrimidine derivatives and their addition salts.

**19.** Composition according to any one of Claims 1 to 18, in which the couplers substituted with at least one radical comprising a quaternized nitrogen atom are chosen from the couplers meta-phenylenediamines, meta-aminophenols, meta-diphenols, naphthalenes, or heterocyclic couplers and their addition salts.

**20.** Composition according to any one of Claims 1 to 18, comprising at least one additional oxidation base not substituted with at least one radical comprising a quaternized nitrogen atom.

**21.** Composition according to any one of Claims 1 to 18, comprising at least one additional coupler not substituted with at least one radical comprising a quaternized nitrogen atom.

**22.** Composition according to any one of Claims 1 to 19, in which the oxidation dye substituted with at least one radical comprising a quaternized nitrogen atom is chosen from para-phenylenediamines.

**23.** Composition according to any one of Claims 1 to 19, in which the oxidation dye substituted with at least one radical comprising a quaternized nitrogen atom is chosen from para-phenylenediamines having a pyrrolidinyl group substituted with one or more radicals comprising a quaternized nitrogen atom.

**24.** Composition according to any one of the preceding claims, additionally comprising an oxidizing agent.

**25.** Method for dyeing keratinous fibres, **characterized in that** a composition as defined in any one of Claims 1 to 23 is applied to the keratinous fibres in the presence of an oxidizing agent, for a period which is sufficient to develop the desired colour.

**26.** Method according to Claim 25, in which the oxidizing agent is chosen from hydrogen peroxide, urea peroxide, alkali metal bromates, persalts, peracids and oxidase enzymes.

**27.** Multicompartment device in which a first compartment contains a dyeing composition as defined in any one of Claims 1 to 23 and a second compartment contains an oxidizing agent.

**28.** Use, for the oxidation dyeing of keratinous fibres, of a composition as defined in any one of Claims 1 to 24.

## Patentansprüche

**1.** Zusammensetzung zum Färben von Keratinfasern, die in einem geeigneten Medium enthält:

○ mindestens eine Oxidationsbase, die unter einem Diamino-N,N-dihydro-pyrazolonderivat der Formel (I) oder einem seiner Additionssalze ausgewählt ist:

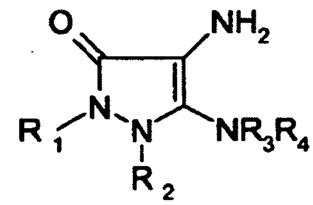

(I)

in der Formel:

$R_1$, $R_2$, $R_3$ et $R_4$, die gleich oder verschieden sind, bedeuten:

- eine geradkettige oder verzweigte $C_{1-10}$-Alkylgruppe, die gegebenenfalls mit einer oder mehreren Gruppen substituiert ist, die unter einer Gruppe $OR_5$, einer Gruppe $NR_6R_7$, einer Carboxygruppe, einer Sulfonsäuregruppe, einer Carboxamidogruppe $CONR_6R_7$, einer Sulfonamidogruppe $SO_2NR_6R_7$, einer Heteroarylgruppe und einer Arylgruppe ausgewählt sind, die gegebenenfalls mit einer oder mehreren Gruppen $(C_{1-4})$-Alkyl, Hydroxy, $(C_{1-2})$Alkoxy, Amino, (Di)alkyl$(C_{1-2})$amino substituiert ist;
- eine Arylgruppe, die gegebenenfalls mit einer oder mehreren Gruppen $(C_{1-4})$Alkyl, Hydroxy, $(C_{1-2})$ Alkoxy, Amino, (Di)alkyl-$(C_{1-2})$amino substituiert ist;
- eine 5- oder 6-gliedrige Heteroarylgruppe, die gegebenenfalls mit einer oder mehreren Gruppen substituiert ist, die unter $(C_{1-4})$Alkyl und $(C_{1-2})$Alkoxy ausgewählt sind;

$R_3$ et $R_4$ können auch ein Wasserstoffatom bedeuten;
$R_5$, $R_6$ et $R_7$, die gleich oder verschieden sind, bedeuten:

- ein Wasserstoffatom;
- eine geradkettige oder verzweigte $C_{1-4}$-Alkylgruppe, die gegebenenfalls mit einer oder mehreren Gruppen substituiert ist, die unter Hydroxy, $(C_{1-2})$Alkoxy, Carboxamido $CONR_8R_9$, Sulfonyl $SO_2R_8$ und einer Arylgruppe ausgewählt sind, die gegebenenfalls mit $(C_{1-4})$Alkyl, Hydroxy, $(C_{1-2})$Alkoxy, Amino, (Di)alkyl$(C_{1-2})$amino substituiert ist; eine Arylgruppe, die gegebenenfalls mit $(C_{1-4})$Alkyl, Hydroxy, $(C_{1-2})$Alkoxy, Amino, (Di)alkyl$(C_{1-2})$amino substituiert ist;

$R_6$ et $R_7$, die gleich oder verschieden sind, können auch eine Carboxamidogruppe $CONR_8R_9$; eine Sulfonylgruppe $SO_2R_8$ bedeuten;
$R_8$ et $R_9$, die gleich oder verschieden sind, bedeuten ein Wasserstoffatom; eine geradkettige oder verzweigte $C_{1-4}$-Alkylgruppe, die gegebenenfalls mit einer oder mehreren Gruppen Hydroxy, $C_{1-2}$-Alkoxy substituiert ist;
$R_1$ und $R_2$ einerseits und $R_3$ et $R_4$ andererseits können mit dem oder den Stickstoffatom(en), an die sie gebunden sind, einen gesättigten oder ungesättigten, 5- bis 7-gliedrigen Heterocyclus bilden, der gegebenenfalls mit einer oder mehreren Gruppen substituiert ist, die ausgewählt sind unter Halogenatomen, den Gruppen Amino, (Di)alkyl$(C_{1-4})$amino, Hydroxy, Carboxy, Carboxamido, $(C_{1-2})$-Alkoxy, den $C_{1-4}$-Alkylgruppen, die gegebenenfalls mit einer oder mehreren Gruppen Hydroxy, Amino, (Di)alkylamino, Alkoxy, Carboxy, Sulfonyl substituiert sind;
$R_3$ et $R_4$ können auch gemeinsam mit dem Stickstoffatom, an das sie gebunden sind, einen 5- oder 7-gliedrigen Heterocyclus bilden, bei dem die Kohlenstoffatome durch ein Sauerstoffatom oder Stickstoffatom ersetzt sein können, das gegebenenfalls substituiert ist ; und

○ mindestens einen Oxidationsfarbstoff, der mit mindestens einer Gruppe substituiert ist, die ein quaternisiertes Stickstoffatom aufweist.

**2.** Zusammensetzung nach Anspruch 1, bei der die Gruppen $R_1$ et $R_2$ unter einer Alkyl$(C_{1-6})$gruppe, die gegebenenfalls mit Hydroxy, $(C_{1-2})$Alkoxy, Amino, (Di)alkyl$(C_{1-2})$amino substituiert ist; einer Phenylgruppe, einer Methoxyphenylgruppe, einer Ethoxyphenylgruppe, einer Benzylgruppe ausgewählt sind.

**3.** Zusammensetzung nach Anspruch 2, bei der die Gruppen $R_1$ et $R_2$ unter Methyl, Ethyl, 2-Hydroxyethyl, 3-Hydroxypropyl, 2-Hydroxypropyl, Phenyl ausgewählt sind.

**4.** Zusammensetzung nach Anspruch 1, bei der die Gruppen $R_1$ et $R_2$ gemeinsam mit den Stickstoffatomen, an die sie gebunden sind, einen 5- oder 6-gliedrigen, gesättigten oder ungesättigten, gegebenenfalls substituierten Ring bilden.

**5.** Zusammensetzung nach einem der Ansprüche 1 bis 4, bei der die Gruppen $R_1$ et $R_2$ gemeinsam mit den Stickstoffatomen, an die sie gebunden sind, einen Pyrazolidinring oder Pyridazolidinring bilden, wobei diese Ringe gegebenenfalls mit einer oder mehreren Gruppen $(C_{1-4})$Alkyl, Hydroxy, $(C_{1-2})$Alkoxy, Carboxy, Carboxamido, Amino, (Di)alkyl$(C_{1-2})$amino substituiert sein können.

**6.** Zusammensetzung nach einem der Ansprüche 1 und 4 bis 5, bei der die Gruppen $R_1$ et $R_2$ gemeinsam mit den Stickstoffatomen, an die sie gebunden sind, einen Pyrazolidinring oder Pyridazolidinring bilden.

**7.** Zusammensetzung nach einem der vorhergehenden Ansprüche, bei der die Gruppen $R_3$ et $R_4$ unter einem Wasserstoffatom; einer geradkettigen oder verzweigten Alkyl$(C_{1-6})$gruppe, die gegebenenfalls mit einer oder mehreren Gruppen Hydroxy, $(C_{1-2})$Alkoxy, Amino, (Di)alkyl$(C_{1-2})$amino substituiert ist; einer Phenylgruppe, die gegebenenfalls mit einer oder mehreren Gruppen Hydroxy, Amino, $(C_{1-2})$Alkoxy substituiert ist, ausgewählt sind.

**8.** Zusammensetzung nach einem der Ansprüche 1 bis 7, bei der die Gruppen $R_3$ et $R_4$ unter einem Wasserstoffatom, Methyl, Ethyl, Isopropyl, 2-Hydroxyethyl, 3-Hydroxypropyl, 2-Hydroxypropyl, 2-Carboxyethyl ausgewählt sind.

**9.** Zusammensetzung nach Anspruch 8, bei der die Gruppen $R_3$ et $R_4$ ein Wasserstoffatom bedeuten.

**10.** Zusammensetzung nach einem der Ansprüche 1 bis 6, bei der die Gruppen $R_3$ et $R_4$ gemeinsam mit dem Stickstoffatom, an das sie gebunden sind, einen 5- oder 7-gliedrigen Ring bilden, der unter den Heterocyclen Pyrrolidin, Piperidin, Homopiperidin, Piperazin, Homopiperazin ausgewählt ist; wobei diese Ringe gegebenenfalls substituiert sein können mit einer oder mehreren Gruppen Hydroxy, Amino, (Di)alkyl$(C_{1-2})$amino, Carboxy, Carboxamido, $(C_{1-4})$Alkyl, wobei diese Gruppe gegebenenfalls mit einer oder mehreren Gruppen Hydroxy, Amino, (Di)alkyl$(C_{1-2})$amino substituiert sein kann.

**11.** Zusammensetzung nach einem der Ansprüche 1 bis 6 und 10, bei der die Gruppen $R_3$ et $R_4$ gemeinsam mit dem Stickstoffatom, an das sie gebunden sind, einen 5- oder 7-gliedrigen Ring bilden, der unter Pyrrolidin, 2,5-Dimethylpyrrolidin, Pyrrolidin-2-carbonsäure, 3-Hydroxypyrrolidin-2-carbonsäure, 4-Hydroxypyrrolidin-2-carbonsäure, 2,4-Dicarboxypyrrolidin, 3-Hydroxy-2-hydroxymethylpyrrolidin, 2-Carboxamidopyrrolidin, 3-Hydroxy-2-carboxamidopyrrolidin, 2-(Diethylcarboxamido)pyrrolidin, 2-Hydroxymethyl-pyrrolidin, 3,4-Dihydroxy-2-hydroxymethylpyrrolidin, 3-Hydroxypyrrolidin, 3,4-Dihydroxypyrrolidin, 3-Aminopyrrolidin, 3-Methylaminopyrrolidin, 3-Dimethylaminopyrrolidin, 4-Amino-3-hydroxypyrrolidin, 3-Hydroxy-4-(2-hydroxyethyl)aminopyrrolidin, Piperidin, 2,6-Dimethylpiperidin, 2-Carboxypiperidin, 2-Carboxamidopiperidin, 2-Hydroxymethylpiperidin, 3-Hydroxy-2-hydroxymethylpiperidin, 3-Hydroxypiperidin, 4-Hydroxypiperidin, 3-Hydroxymethylpiperidin, Homopiperidin, 2-Carboxyhomopiperidin, 2-Carboxamidohomopiperidin, Homopiperazin, N-Methyl-homopiperazin, N-(2-Hydroxyethyl)-homopiperazin ausgewählt ist.

**12.** Zusammensetzung nach einem der Ansprüche 1 bis 6 und 10 bis 11, bei der die Gruppen $R_3$ et $R_4$ gemeinsam mit dem Stickstoffatom, an das sie gebunden sind, einen 5- oder 7-gliedrigen Ring bilden, der unter Pyrrolidin, 3-Hydroxypyrrolidin, 3-Aminopyrrolidin, 3-Dimethylamino-pyrrolidin, Pyrrolidin-2-carbonsäure, 3-Hydroxypyrrolidin-2-carbonsäure, Piperidin, Hydroxypiperidin, Homopiperidin, Diazepan, N-Methylhomopiperazin, N-β-Hydroxyethylhomopiperazin ausgewählt ist.

**13.** Zusammensetzung nach einem der Ansprüche 1 bis 6 und 10 bis 12, bei der die Gruppen $R_3$ et $R_4$ gemeinsam mit dem Stickstoffatom, an das sie gebunden sind, einen 5-gliedrigen Ring bilden, wie Pyrrolidin, 3-Hydroxypyrrolidin, 3-Aminopyrrolidin, 3-Dimethylaminopyrrolidin.

**14.** Zusammensetzung nach Anspruch 1 bis 13, bei der die Verbindung der Formel (I) oder eines ihrer Additionssalze ausgewählt ist unter:

2,3-Diamino-6,7-dihydro-1H,5H-pyrazolo[1,2-a]pyrazol-1-on;

2-Amino-3-methylamino-6,7-dihydro-1H,5H-pyrazolo[1,2-a]-pyrazol-1-on;
2-Amino-3-isopropylamino-6,7-dihydro-1H,5H-pyrazolo[1,2-a]-pyrazol-1-on;
2-Amino-3-(pyrrolidin-1-yl)-6,7-dihydro-1H,5H-pyrazolo[1,2-a]-pyrazol-1-on;
4,5-Diamino-1,2-dimethyl-1,2-dihydro-pyrazol-3-on;
4,5-Diamino-1,2-diethyl-1,2-dihydro-pyrazol-3-on;
4,5-Diamino-1,2-di-(2-hydroxyethyl)-1,2-dihydro-pyrazol-3-on;
2-Amino-3-(2-hydroxyethyl)amino-6,7-dihydro-1H,5H-pyrazolo[1,2-a]pyrazol-1-on;
2-Amino-3-dimethylamino-6,7-dihydro-1H,5H-pyrazolo[1,2-a]-pyrazol-1-on;
2,3-Diamino-5,6,7,8-tetrahydro-1H,6H-pyridazino[1,2-a]pyrazol-1-on;
4-Amino-1,2-diethyl-5-(pyrrolidin-1-yl)-1,2-dihydo-pyrazol-3-on;
4-Amino-5-(3-dimethylamino-pyrrolidin-1-yl)-1,2-diethyl-1,2-dihydro-pyrazol-3-on;
2,3-Diamino-6-hydroxy-6,7-dihydro-1H,5H-pyrazolo[1,2-a]-pyrazol-1-on.

**15.** Zusammensetzung nach einem der vorhergehenden Ansprüche, wobei der oder die Oxidationsfarbstoffe, die mit mindestens einer Gruppe substituiert sind, die ein quaternisiertes Stickstoffatom aufweist, mit Trialkylammoniumgruppen oder in einem Ring enthaltenen quaternisierten Stickstoffatomen substituiert sind.

**16.** Zusammensetzung nach Anspruch 15, wobei die Gruppen, die ein quaternisiertes Stickstoffatom aufweisen, unter den Gruppen Trimethylammonium, Triethylammonium, Diethyl-methylammonium, Tri-butylammmonium, Pyrrolium, Imidazolium, Pyrazolium, Oxazolium, Thiazolium, Triazolium, Pyridinium, Pyrimidinium, Pyrazinium, Oxazinium und Triazinium ausgewählt sind.

**17.** Zusammensetzung nach einem der Ansprüche 1 bis 16, wobei die Oxidationsfarbstoffe, die mit mindestens einer Gruppe substituiert sind, die ein quaternisiertes Stickstoffatom aufweist, Oxidationsbasen oder Kuppler sind.

**18.** Zusammensetzung nach einem der vorhergehenden Ansprüche, wobei die Oxidationsbasen, die mit mindestens einer Gruppe substituiert sind, die ein quaternisiertes Stickstoffatom aufweist, unter den p-Phenylendiaminen, Bisphenylalkylendiaminen, p-Aminophenolen, Bis-p-aminophenolen, o-Aminophenolen, heterocyclischen Basen, wie Pyridinderivaten, Pyrimidinderivaten und Pyrazolderivaten, Pyrazolo[1,5-a]pyrimidinderivaten und deren Additionssalzen ausgewählt sind.

**19.** Zusammensetzung nach einem der Ansprüche 1 bis 18, wobei die Kuppler, die mit mindestens einer Gruppe substituiert sind, die ein quaternisiertes Stickstoffatom aufweist, unter den m-Phenylendiaminen, m-Aminophenolen, m-Dihydroxybenzolen, Naphthalinkupplern, heterocyclischen Kupplern und deren Additionssalzen ausgewählt sind.

**20.** Zusammensetzung nach einem der Ansprüche 1 bis 18, die mindestens eine ergänzenden Oxidationsbase enthält, die nicht mit mindestens einer Gruppe substituiert ist, die ein quaternisiertes Stickstoffatom aufweist.

**21.** Zusammensetzung nach einem der Ansprüche 1 bis 18, die mindestens einen ergänzenden Kuppler enthält, der nicht mit mindestens einer Gruppe substituiert ist, die ein quaternisiertes Stickstoffatom aufweist.

**22.** Zusammensetzung nach einem der Ansprüche 1 bis 19, bei der der Oxidationsfarbstoff, der mit mindestens einer Gruppe substituiert ist, die ein quaternisiertes Stickstoffatom aufweist, unter den p-Phenylendiaminen ausgewählt ist.

**23.** Zusammensetzung nach einem der Ansprüche 1 bis 19, bei der der Oxidationsfarbstoff, der mit mindestens einer Gruppe substituiert ist, die ein quaternisiertes Stickstoffatom aufweist, unter den p-Phenylendiaminen mit Pyrrolidinylgruppe ausgewählt ist, die mit einer oder mehreren Gruppen substituiert sind, die ein quaternisiertes Stickstoffatom aufweisen.

**24.** Zusammensetzung nach einem der vorhergehenden Ansprüche, die ferner ein Oxidationsmittel enthält.

**25.** Verfahren zum Färben von Keratinfasern, **dadurch gekennzeichnet, dass** eine Zusammensetzung nach einem der Ansprüche 1 bis 23 in Gegenwart eines Oxidationsmittels während einer Zeitspanne, die ausreichend ist, um die gewünschte Farbe zu bilden, auf die Keratinfasern aufgebracht wird.

**26.** Verfahren nach Anspruch 25, wobei das Oxidationsmittel unter Wasserstoffperoxid, Harnstoffperoxid, Alkalimetallbromaten, Salzen von Persäuren, Persäuren und Oxidasen ausgewählt ist.

**27.** Vorrichtung mit mehreren Abteilungen, wobei eine erste Abteilung eine Farbmittelzusammensetzung enthält, wie sie in einem der Ansprüche 1 bis 23 definiert ist, und eine zweite Abteilung ein Oxidationsmittel enthält.

**28.** Verwendung der Zusammensetzung nach einem der Ansprüche 1 bis 24 zum oxidativen Färben von Keratinfasern.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- EP 968171 A
- EP 928289 A
- EP 932602 A
- EP 1348695 A
- EP 1396486 A
- EP 983996 A
- EP 984006 A
- EP 1147109 A
- EP 1147090 A
- EP 1064252 A
- EP 1064267 A
- EP 1064268 A
- EP 989128 A
- FR 2586913
- US 4128425 A
- US 2841584 A
- DE 4234885


### Littérature non-brevet citée dans la description


- *J. Het. Chem.,* 2001, vol. 38 (3), 613-616
- *Helvetica Chimica Acta,* 1950, vol. 33, 1183-1194
- *J.Org.Chem.,* 1958, vol. 23, 2029
- *J.Am.Chem.Soc.,* 1951, vol. 73, 3240
- *J.Am.Chem.Soc.,* 1962, vol. 84, 590
- *Justus Liebig Ann.Chem.,* 1965, vol. 686, 134
- *Tetrahedron. Lett.,* 1973, vol. 31, 2859-2862
- **J. MARCH.** Advanced Organic Chemistry. Willey Interscience, 1985
- **M . HUDLICKY.** Réduction in organic Chemistry. Ellis Horwood Séries Chemical Science, 1983
- **M. HUDLICKY.** Réduction in organic Chemistry. Ellis Horwood Series Chemical Science, 1983